# EUROPEAN PATENT APPLICATION

(11) **EP 1 739 172 A1**
(43) Date of publication of application: **03.01.2007**
(21) Application number: 05721335.7
(22) Date of filing: 23.03.2005
(51) Int. Cl.: C12N 15/09, A01H 5/00, C12Q 1/68

(54) **GENETIC MARKER LINKED TO GENE LOCUS INVOLVED IN BARLEY RESISTANCE TO YELLOW MOSAIC DISEASE AND USE THEREOF**

(30) Priority: 25.03.2004 JP 2004090644; 26.11.2004 JP 2004342737
(71) Applicant: Japan Science and Technology Agency, Kawaguchi-shi, Saitama 332-0012 (JP)
(72) Inventor: TAKEDA, Kazuyoshi, Kurashiki-shi, Okayama 710-0031 (JP); SATO, Kazuhiro, Kurashiki-shi, Okayama 710-0031 (JP)
(74) Representative: Müller, Frithjof E.
(86) International application number: PCT/JP2005/005285
(87) International publication number: WO 2005/093058

(57) **Abstract**

Through creation of a detailed linkage map of barley and QTL analysis thereof, there have been found five genetic markers linked to gene locus involved in barley resistance to yellow mosaic disease and situated on barley 1H chromosome, two genetic markers linked to gene locus involved in barley resistance to yellow mosaic disease and situated on barley 2H chromosome barley resistance to yellow mosaic disease, five genetic markers linked to gene locus involved in barley resistance to yellow mosaic disease and situated on barley 3H chromosome, four genetic markers linked to gene locus involved in barley resistance to yellow mosaic disease and situated on barley 4H chromosome, and two genetic markers linked to gene locus involved in barley resistance to yellow mosaic disease and situated on barley 5H chromosome.

SEQUENCE LISTING

<110> Japan Science and Technology Agency

<120> Genetic markers that link to the QTL of resistance to
      barley yellow mosaic virus, and usage thereof

<130> A181-10PCT

<150> JP 2004-090644
<151> 2004-3-25

<150> JP 2004-342737
<151> 2004-11-26

<160> 62

<170> PatentIn Ver. 2.1

<210> 1
<211> 20
<212> DNA
<213> Artificial Sequence

<220>
<223> Description of Artificial Sequence:Artificially
      synthesized primer sequence

<400> 1
cttggccttg atcttctgct                                                20


<210> 2
<211> 20
<212> DNA
<213> Artificial Sequence

<220>
<223> Description of Artificial Sequence:Artificially
      synthesized primer sequence

<400> 2
gaccgtgtca ggaaagcaat                                                20


<210> 3
<211> 20
<212> DNA
<213> Artificial Sequence

<220>
<223> Description of Artificial Sequence:Artificially
      synthesized primer sequence

<400> 3
tctttcctgg gttggtgaac                                                20


<210> 4
<211> 20
<212> DNA
<213> Artificial Sequence

<220>
<223> Description of Artificial Sequence:Artificially
      synthesized primer sequence

<400> 4

gcagcttttg agttcgttcc                                                    20

<210> 5
<211> 20
<212> DNA
<213> Artificial Sequence

<220>
<223> Description of Artificial Sequence:Artificially
      synthesized primer sequence

<400> 5
ctgtttggat gactgcgaga                                                    20

<210> 6
<211> 20
<212> DNA
<213> Artificial Sequence

<220>
<223> Description of Artificial Sequence:Artificially
      synthesized primer sequence

<400> 6
attacgcaac ctgatggagc                                                    20

<210> 7
<211> 20
<212> DNA
<213> Artificial Sequence

<220>
<223> Description of Artificial Sequence:Artificially
      synthesized primer sequence

<400> 7
accccggaag ctaagatgat                                                    20

<210> 8
<211> 20
<212> DNA
<213> Artificial Sequence

<220>
<223> Description of Artificial Sequence:Artificially
      synthesized primer sequence

<400> 8
agtcggaaca tgcggtacac                                                    20

<210> 9
<211> 21
<212> DNA
<213> Hordeum vulgare

<400> 9
atcttctgca ggcacttgtc g                                                  21

<210> 10
<211> 21
<212> DNA
<213> Hordeum vulgare

<400> 10
atcttctgca agcacttgtc g                                                21

<210> 11
<211> 21
<212> DNA
<213> Hordeum vulgare

<400> 11
tgccgtggcc gtgcacgatg a                                                21

<210> 12
<211> 21
<212> DNA
<213> Hordeum vulgare

<400> 12
tgccgtggcc atgcacgatg a                                                21

<210> 13
<211> 21
<212> DNA
<213> Hordeum vulgare

<400> 13
attactcagc tacacaccta t                                                21

<210> 14
<211> 21
<212> DNA
<213> Hordeum vulgare

<400> 14
attactcagc cacacaccta t                                                21

<210> 15
<211> 688
<212> DNA
<213> Hordeum vulgare

<400> 15
gcagtaagtg gaggacaaaa aggaatactg ggattagata gaatgtagcg acattctgcg 60
gggtggggtg gtataagtag atatacagaa cggaggaccg tctcctggtc ctcagctttg 120
ggtcttctct accttggcct tgatcttctg ctcgaggacc gccttctccg tgtcgaggtt 180
gaacatcctg ttgagggcgt gcatgttgtc gagcgtctcg tccagggtgc ggctgtcgct 240
gccatcgcat ctcaggtgct gcaacgggcc gtggaggagc ttgttcacta tgcccgtgct 300
cagctcttcg atagaccttc tcgtcttctt gttgaggttg tcttccccga tcttctgcag 360
gcacttgtcg agctcggatg ccctgatcct gtcggcatac gacctcagct ttttgatggt 420
cgggaccgtc tccagcgagt ccctccacgc ctcgaaccgc ttcagttctt gggtgatgat 480
tgcttgggcc tccattgctt tcctgacacg gtcttccttg ttggcttcca ccacctcttt 540
caagtcgtca acattgtata cccgtgcgtg ctccacttga gataggcagg caccgacgtt 600
ccttgggacg gatatgtcga cgaaaagccg aacaccaccc atggcaagag agataggagg 660
aagcgcctcc gcatgcccct tggtgaat                                         688

<210> 16
<211> 645
<212> DNA
<213> Hordeum vulgare

<400> 16
agatgacagc taggccctga gcaggggaca catgaatatt tcccgctcac taccctatct 60

atactatcca gtatctacac atgaatattt ccagagattt tgctattatg atactgcgat 120
caagaactgt ctgcaaaatc aagaaacact attttgatta cgtccctatg atatactgct 180
agttcctata tatctttcct gggttggtga acttgagctt ctgactgact gcttggtagg 240
ttcatctccg tgttcctctg gacttgaagg tgtgaggaag ggcatgcctc cagagaagaa 300
aagcgtgttg tgtgcctacc aaacttgctt cgttgacagc ccaagggcag gtccccacgt 360
gtcgtcgctt tattttcctt cttttcataa acaccttcaa acttttttca accacgcgct 420
gcaactggaa gtaatgattg tacaggctct tctttaattc aaatcacacg caacgtcaca 480
aatactaacc ttgacatgcc tggaactggc agtgtggcta ccatccacct tcgcctagtt 540
gccatgtata aagatacggt ggagcttgtt ggcattgacc gcataaggaa cgaactcaaa 600
agctgcttaa tgaggacaag acatctaatg aagaacaatt gaaga 645

<210> 17
<211> 696
<212> DNA
<213> Hordeum vulgare

<220>
<221> unsure
<222> (10, 24, 30)

<400> 17
   catgcacatn aattgaatac aggnactagn aacttacaat acataagcgt gacgacattc 60
   cataactcca atgtgatttt agtagtcacg tacaacatga tctaattatt attatggcgc 120
   catccaattc ttagcgaccg acgagatgtt ttcatcgttg tttgtagtaa cacaaaacag 180
   agtagactgt ttggatgact gcgagaagta agccaatagc agctgccatg agcccaacgg 240
   caagccatgg gttcctcaag tacctctgcc ttagccaggc agtccacctc cgggggttgc 300
   tctggaaccg cttctccagc ttcttgcatg tctcccgtag gtagttgcag ccggggtcat 360
   cagggtcaaa cataatcccc ttgcaaaggt cgacgaagca tttggccacc tcttcgttgt 420
   tgccgtggcc gtgcacgatg acgcctctcc tcaccaagag ctccacgtcc ttcgtggtgc 480
   aggccatctg ggacatgaac acgcagtatg ccgtgacatg gctccccacc gtctcccggt 540
   tcctctgctc cagctccatc aggttgcgta atagccgcca cgtctcggcg tcgatgtcca 600
   ggacggggat ctccagcgta ccgccaccgc cgtccagctt cacgtcgagg atgcagcgga 660
   tgacaccctt ctcgctcatg gccccgggcg ttgaac 696

<210> 18
<211> 738
<212> DNA
<213> Hordeum vulgare

<400> 18
aatcttagca ttcctgttct ctatttacga attacctaat ggtaatgcat gagcagtagt 60
cacaggataa cttccgatac ctccaagcag ttgcaactca taagatgaaa ttatttacaa 120
agagtggcca tgactaccta ccctacctgc tatctacagt tttttttgga tatactattc 180
acagttggtg tcgaagagac gtaaccaatc taggaacata catgggcaag gaatgacccc 240
ggaagctaag atgatgaatt ggaagaggag tcatgtacac aacctactta tagactatat 300
ataattttag ggccatttct caaccaacac ctatttgaca agggcaagaa ttttacttcg 360
tattgatgaa acagatatca caattcacca ttacggcatt acttacctag ttgatgccaa 420
agaaacttaa taatatctaa gaaactatcc acctaaggaa tgaccagaga agctgagatg 480
atgaattgga aggagtcaaa tacagaaccc atttttagat tatatgtaca aaattttagg 540
gtcattactc agccacacac ctatttgaaa acaacaggaa gaattttact tagtcatgat 600
gaaacagatt tcttttaaca aataagaatt cgactactcc ttagatatct aaagatggat 660
gacgtagcca gataccatgt agaacactga aaaggcgtgt accgcatgtt ccgactgaag 720
actatcaatc atggtaag 738

<210> 19
<211> 20
<212> DNA
<213> Artificial Sequence

<220>
<223> Description of Artificial Sequence:Artificially
      synthesized primer sequence

<400> 19
acgatcgatc aaaaggacca 20

<210> 20
<211> 20
<212> DNA
<213> Artificial Sequence

<220>
<223> Description of Artificial Sequence:Artificially
      synthesized primer sequence

<400> 20
aatccgacga aatcaacgag                                                    20


<210> 21
<211> 20
<212> DNA
<213> Artificial Sequence

<220>
<223> Description of Artificial Sequence:Artificially
      synthesized primer sequence

<400> 21
ctcgatcatc agcgacttca                                                    20


<210> 22
<211> 20
<212> DNA
<213> Artificial Sequence

<220>
<223> Description of Artificial Sequence:Artificially
      synthesized primer sequence

<400> 22
gaagaggcac cttctgcaac                                                    20


<210> 23
<211> 20
<212> DNA
<213> Artificial Sequence

<220>
<223> Description of Artificial Sequence:Artificially
      synthesized primer sequence

<400> 23
aatgtgcaca ccaaggttga                                                    20


<210> 24
<211> 20
<212> DNA
<213> Artificial Sequence

<220>
<223> Description of Artificial Sequence:Artificially
      synthesized primer sequence

<400> 24
agacaacaac cgcctgtacc                                                    20


<210> 25

<211> 19
<212> DNA
<213> Artificial Sequence

<220>
<223> Description of Artificial Sequence:Artificially
      synthesized primer sequence

<400> 25
gatgagtcct gagtaaacc                                                    19


<210> 26
<211> 19
<212> DNA
<213> Artificial Sequence

<220>
<223> Description of Artificial Sequence:Artificially
      synthesized primer sequence

<400> 26
gactgcgtac caattcacg                                                    19


<210> 27
<211> 20
<212> DNA
<213> Artificial Sequence

<220>
<223> Description of Artificial Sequence:Artificially
      synthesized primer sequence

<400> 27
tccagccgaa caatttcttg                                                   20


<210> 28
<211> 20
<212> DNA
<213> Artificial Sequence

<220>
<223> Description of Artificial Sequence:Artificially
      synthesized primer sequence

<400> 28
agtactccga caccacgtcc                                                   20


<210> 29
<211> 19
<212> DNA
<213> Artificial Sequence

<220>
<223> Description of Artificial Sequence:Artificially
      synthesized primer sequence

<400> 29
gatgagtcct gagtaaatt                                                    19


<210> 30
<211> 19
<212> DNA
<213> Artificial Sequence

<220>
<223> Description of Artificial Sequence:Artificially
      synthesized primer sequence

<400> 30
gactgcgtac caattcacg                                                    19


<210> 31
<211> 19
<212> DNA
<213> Artificial Sequence

<220>
<223> Description of Artificial Sequence:Artificially
      synthesized primer sequence

<400> 31
gatgagtcct gagtaaata                                                    19


<210> 32
<211> 19
<212> DNA
<213> Artificial Sequence

<220>
<223> Description of Artificial Sequence:Artificially
      synthesized primer sequence

<400> 32
gactgcgtac caattcgga                                                    19


<210> 33
<211> 20
<212> DNA
<213> Artificial Sequence

<220>
<223> Description of Artificial Sequence:Artificially
      synthesized primer sequence

<400> 33
atggacccaa caagtggaag                                                   20


<210> 34
<211> 20
<212> DNA
<213> Artificial Sequence

<220>
<223> Description of Artificial Sequence:Artificially
      synthesized primer sequence

<400> 34
aggaagactt tggaggccat                                                   20


<210> 35
<211> 19
<212> DNA
<213> Artificial Sequence

<220>
<223> Description of Artificial Sequence:Artificially

synthesized primer sequence

<400> 35
gatgagtcct gagtaatgg                                                    19


<210> 36
<211> 19
<212> DNA
<213> Artificial Sequence

<220>
<223> Description of Artificial Sequence:Artificially
      synthesized primer sequence

<400> 36
gactgcgtac caattcgga                                                    19


<210> 37
<211> 19
<212> DNA
<213> Artificial Sequence

<220>
<223> Description of Artificial Sequence:Artificially
      synthesized primer sequence

<400> 37
gatgagtcct gagtaacaa                                                    19


<210> 38
<211> 19
<212> DNA
<213> Artificial Sequence

<220>
<223> Description of Artificial Sequence:Artificially
      synthesized primer sequence

<400> 38
gactgcgtac caattcggg                                                    19


<210> 39
<211> 19
<212> DNA
<213> Artificial Sequence

<220>
<223> Description of Artificial Sequence:Artificially
      synthesized primer sequence

<400> 39
gatgagtcct gagtaaacg                                                    19


<210> 40
<211> 19
<212> DNA
<213> Artificial Sequence

<220>
<223> Description of Artificial Sequence:Artificially
      synthesized primer sequence

<400> 40

gactgcgtac caattccat                                                    19

<210> 41
<211> 19
<212> DNA
<213> Artificial Sequence

<220>
<223> Description of Artificial Sequence:Artificially
      synthesized primer sequence

<400> 41
gatgagtcct gagtaaatg                                                    19

<210> 42
<211> 19
<212> DNA
<213> Artificial Sequence

<220>
<223> Description of Artificial Sequence:Artificially
      synthesized primer sequence

<400> 42
gactgcgtac caattcata                                                    19

<210> 43
<211> 19
<212> DNA
<213> Artificial Sequence

<220>
<223> Description of Artificial Sequence:Artificially
      synthesized primer sequence

<400> 43
gatgagtcct gagtaaacg                                                    19

<210> 44
<211> 19
<212> DNA
<213> Artificial Sequence

<220>
<223> Description of Artificial Sequence:Artificially
      synthesized primer sequence

<400> 44
gactgcgtac caattcgat                                                    19

<210> 45
<211> 19
<212> DNA
<213> Artificial Sequence

<220>
<223> Description of Artificial Sequence:Artificially
      synthesized primer sequence

<400> 45
gatgagtcct gagtaaacg                                                    19

<210> 46
<211> 19
<212> DNA
<213> Artificial Sequence

<220>
<223> Description of Artificial Sequence:Artificially
      synthesized primer sequence

<400> 46
gactgcgtac caattcgga                                                    19


<210> 47
<211> 16
<212> DNA
<213> Artificial Sequence

<220>
<223> Description of Artificial Sequence:Artificially
      synthesized primer sequence

<400> 47
gacgatgagt cctgag                                                       16


<210> 48
<211> 14
<212> DNA
<213> Artificial Sequence

<220>
<223> Description of Artificial Sequence:Artificially
      synthesized primer sequence

<400> 48
tactcaggac tcat                                                         14


<210> 49
<211> 17
<212> DNA
<213> Artificial Sequence

<220>
<223> Description of Artificial Sequence:Artificially
      synthesized primer sequence

<400> 49
ctcgtagact gcgtacc                                                      17


<210> 50
<211> 18
<212> DNA
<213> Artificial Sequence

<220>
<223> Description of Artificial Sequence:Artificially
      synthesized primer sequence

<400> 50
aattggtacg cagtctac                                                     18


<210> 51
<211> 16
<212> DNA

<213> Artificial Sequence

<220>
<223> Description of Artificial Sequence:Artificially
      synthesized primer sequence

<400> 51
gatgagtcct gagtaa                                                    16


<210> 52
<211> 16
<212> DNA
<213> Artificial Sequence

<220>
<223> Description of Artificial Sequence:Artificially
      synthesized primer sequence

<400> 52
gactgcgtac caattc                                                    16


<210> 53
<211> 664
<212> DNA
<213> Hordeum vulgare

<220>
<221> unsure
<222> (10)

<400> 53
tatatagatn ccttctcgac agaatgaacc agttaagata gctgaaatca caatttacag 60
ataagcactt ggtattcact aatccatgaa aatagttatt attcaaaaca cagggagatc 120
gcaagggagt ctgatctgaa aaggccctag aagccattca gtaaccacga gagtaacaag 180
ttgcagaaag ctagaaacaa ctggaatact taatcccttg acattcaaaa acatgaagaa 240
tgtcaaaatg atgattcata acacgatcga tcaaaaggac cagtcatctg gaagaactct 300
tggtagtcgt tcagtaacgg cgtggaggga accggtccag gagcctccca gatggacact 360
cgtaagccat gtgaccacga ccaccacagt tgttgcagat catgaaggcg ccacccatgc 420
agtcacggct catatggcca acctggttgc aggcccggca gaccatgtca ctgtagccac 480
cacggaagag agcaccgccg ccaccacgga agggagcatc gccaccacgg aacagagcat 540
cgccgccacg gaacagagcg tcaccgccac ggaacagagc gtcaccacca cggaagggag 600
gaggcccacc cctctcgttg atttcgtcgg atttggggca ttgacgggcc aaatgccctg 660
caac                                                                 664


<210> 54
<211> 545
<212> DNA
<213> Hordeum vulgare

<400> 54
cagcataaat ctcgtaaccc atagcaggca tcaggtagaa gtattactgt atgcaggggg 60
gacatcattg tgataaatca taaggtaggt aattttccat aactcaaaat tttgatctaa 120
gcttctatct ttctctctct ctctctaata ggtgcctcga tcatcagcga cttcaggact 180
tgctcctcgt aacaaaccag ctgagtcttg acgaaacaat ccagaaagtc agcagcaagg 240
aagcaagccc tggggtcttc aaaaggcaat cgaccaatct tcttgtgtgg cctcagtggt 300
ggtctgttct actccgcctt cttggtccgg aagttcgacc tcattgacga aacctttttc 360
ttgcactgca ccactgtttt accagggaca gcagtagcta ctcgctccca tctttggttt 420
gcatccttgg gaaatgcctt caaagcttga acaaggccca gcacctgagc ctctgaccag 480
gcatctggat ctgttgcaga aggtgcctct tcggggacag gatcagcacc agttttctca 540
ttggc                                                                545


<210> 55
<211> 21
<212> DNA

<213> Hordeum vulgare

<400> 55
cttatgtgat cataatactg c                                                21


<210> 56
<211> 21
<212> DNA
<213> Hordeum vulgare

<400> 56
cttatgtgat aataatactg c                                                21


<210> 57
<211> 514
<212> DNA
<213> Hordeum vulgare

<400> 57
ttacaggagg gaacgcattc cttgtatgta tagaagaata tatctaagac atgttacaaa 60
acaacaatgt gcacaccaag gttgacagtt acacggctga cagctgttac aactcccaga 120
agaatgggtt ggcttttaca aagaagaaaa aaaatgatca aaaatttgcg tctccctccg 180
cagtgatcca agttcccaag ggcagcttct taggggagcc cctagatgca aatggatccg 240
ggcatccccg ccaagcgaac ttcttttgtg cgcgtggaag aagcagcctt ccaccggtat 300
catttacttg gcaattctac atccgtaaga actattcatc tatacagccg gaatttttag 360
aaggacgagt gagcagcaat acagacatac ggagatggca accccggcga tgaaattctg 420
gtacaggcgg ttgttgtctc tactgggaat gaaggctgcc ctcagcacgt ttgtgatctc 480
aaatactcgg tatgacagca aaagatagat tgat                                  514


<210> 58
<211> 21
<212> DNA
<213> Hordeum vulgare

<400> 58
agccttccac tggtatcatt t                                                21


<210> 59
<211> 21
<212> DNA
<213> Hordeum vulgare

<400> 59
agccttccac cggtatcatt t                                                21


<210> 60
<211> 540
<212> DNA
<213> Hordeum vulgare

<220>
<221> unsure
<222> (2)

<400> 60
gnttaaattg gcgagattaa catttacatg aaaaatgatg gagatgatat tgctttgagc 60
ttgaccgaat ggacccaaca agtggaaggc attctatatg aattacagtt atagctccat 120
gacgttctac agtactatga cattacatcc atgatagcta attgtacaaa agagtgaatg 180
aaatgaacta cgaaaatcca gcatcatagt tctgcagtcg ggcttctcag aagtgagcca 240
agctgtatac tagagtccat gaaagcagga aaagggtgaa cgaggcaaag agcccctcct 300
gaagtagggc ggcatggcct ccaaagtctt cctcatcaat cttcagcaga tgtgcatagt 360
acaaatgaac tattacggtg gaaatagtaa agaatagggc gatccagaag gcgccgacga 420
gggggacggc gccccagagc aggccgcagg cgagccccac cgcctgccgg atccagtgca 480

ccgcgtccag cagctggtcc ttgtccaagg acgcgtcggg gtcgaggtac cgggcgagcc 540

<210> 61
<211> 21
<212> DNA
<213> Hordeum vulgare

<400> 61
tttatgtgcc tgtagcggtt g 21

<210> 62
<211> 21
<212> DNA
<213> Hordeum vulgare

<400> 62
tttatgtgcc ggtagcggtt g 21

## Description

### TECHNICAL FIELD

The present invention relates to novel genetic markers and use thereof. Particularly, the invention relates to genetic markers linked to gene loci involved in barley resistance to yellow mosaic disease, and use of such genetic markers.

### BACKGROUND ART

Barley yellow mosaic disease is a soilborne viral disease caused by barley yellow mosaic virus (hereinafter, "BaYMV") or barley mild mosaic virus (hereinafter, "BaMMV"), with the involvement of the Phycomycete, *Polymyxa graminis,* acting as a carrier. When developed, it causes necrotic spots or yellowing on the leaves, in addition to other abnormalities such as a slow tillering rate or stunt growth, or even death. The seriousness of the disease is that once it occurs the soils stays contaminated even if the soil is rested for 4 to 5 years. The disease is particularly common in malting barley, and there have been increasing numbers of cases not only in Japan but in China and Germany as well, where cultivation of malting barley has been carried out on a wide scale. As a preventative measure, resistance to yellow mosaic disease has been looked into with interest even in countries which have not seen the disease. Taken together, resistance to yellow mosaic disease has become a very important subject in breeding.

Currently, cultivation of resistant varieties is considered to be the most effective way to eradicate the yellow mosaic disease. Cultivation in a contaminated field can produce varieties with strong resistance. Conventionally, breeding of crops relied on crossing a variety having a target trait with the wildtype or other varieties, followed by actually cultivating individuals in large numbers and screening for individuals with the target trait, and genetically fixing the target trait. This requires a large field and huge labor, not to mention it is time consuming. For example, consider the case where the target trait is the resistance to BaYMV. In this case, since inoculation of BaYMV is difficult, whether or not a resistant gene has been incorporated in the breeding line needs to be assessed in a soil that has been contaminated with BaYMV, and resistant individuals need to be screened for. This is time consuming and labor intensive.

In order to reduce cultivation time, labor, and field area and for more reliable screening of useful genes, recent breeding methods employ screening that uses a genetic marker as an index. Breeding employing a genetic marker enables screening at the seeding stage, based on a genotype of the marker, and therefore it does not require cultivation in a BaYMV-contaminated soil. As a result, the presence or absence of the target trait can be confirmed with ease. That is, the genetic marker can be used to realize efficient breeding. For breeding using genetic markers, development of genetic markers strongly linked to the target trait is necessary.

Meanwhile, resistance to yellow mosaic disease and many other agriculturally important traits vary continuously in the derived lines of hybrids. Such traits can be measured quantitatively, for example, based on time and length, and are therefore known as quantitative traits. Generally, quantitative traits are not controlled by a single major gene, but are often determined by the action of more than one gene. Many of the traits that are modified in crop breeding, for example, such as yield, quality, and taste, are quantitative traits.

Chromosomal locations of genes conferring such quantitative traits are known as QTL (quantitative trait loci). As a method of estimating QTL, a QTL analysis is used that makes use of a genetic marker residing in the vicinity of a QTL. The QTL analysis statistically analyzes which regions of chromosomes relate to the quantitative traits of interest, and DNA markers that reside near these chromosomal regions are detected. Based on the DNA markers, a genetic map (linkage map) is constructed. From the information of the genetic map, regions that influence the target trait are tracked down until a gene responsible for the trait is specified and isolated. Since the advent of DNA markers in the late 1980s, the study of linkage map has advanced greatly. Today, QTL analysis is performed in many organisms based on their genetic maps.

As described above, development of genetic markers linked to a target trait is now possible with the accurate QTL analysis that is based on a detailed linkage map covering the entire chromosomes. The genetic markers can then be used to realize efficient breeding. Concerning resistance to yellow mosaic disease, the inventors of the present invention have previously found QTLs on the long arm of 3H chromosome, near the centromere of 4H chromosome, and on the short arm of 7H chromosome in Chinese six-row barley, Mokusekko 3. (See Chikara Miyazaki, Eiichi Osanai, Kazutoshi Ito, Takeo Konishi, Kazuhiro Sato and Akira Saito. 2001. "Mapping of quantitative trait loci conferring resistance to barley yellow mosaic virus in a Chinese barley landrace Mokusekko 3." Breeding Science 51: 171-177.)

As described above, resistance to yellow mosaic disease has become a very important subject in breeding. However, owning to the fact that more than one gene is usually responsible for resistance to yellow mosaic disease, a crossing or other conventional methods are not sufficient to confirm whether a gene (gene locus) involved in barley resistance to yellow mosaic disease has been properly screened for. Under these circumstances, genetic markers would be very effective for the breeding of yellow mosaic disease-resistant barley. If genetic markers linked to gene loci involved in barley resistance to yellow mosaic disease were developed for use, it would be possible to greatly improve the efficiency of breeding yellow mosaic disease-resistant barley.

The present invention was made in view of the foregoing problem, and an object of the present invention is to provide novel genetic markers that are linked to gene loci involved in barley resistance to yellow mosaic disease, and representative use of such genetic markers.

### DISCLOSURE OF INVENTION

In order to achieve the foregoing object, the inventors of the present invention designed primer sets based on inventors' barley EST sequences, and developed genetic markers (DNA markers) based on the presence or absence of polymorphism in the fragments of barley genomic DNA amplified with the primer sets. Further, the inventors produced a double haploid population (hereinafter, double haploid lines will be abbreviated to "DH", and a population of double haploid lines will be referred to as a "DHHS population") from the F1 hybrid of malting barley "Haruna Nijo" and "wildtype barley "H602", and constructed a linkage map of the DHHS population by detecting linkage between the genetic markers, and performed a QTL analysis for the gene loci involved in barley resistance to yellow mosaic disease, based on the linkage map. As a result, a gene locus involved in barley resistance to yellow mosaic disease was found on 1H chromosome and 3H chromosome. Upon further analysis, the inventors found novel genetic markers that respectively linked to the gene loci involved in barley resistance to yellow mosaic disease.

The inventors continued the QTL analysis further using barley varieties with significantly different levels of resistance to yellow mosaic disease. Specifically, the following populations were used for the QTL analysis: RI line obtained from the cross between Russia 6 (two-row, resistant) and H.E.S. 4 (six-row, susceptible) ("RI1 population" hereinafter); RI line obtained from the cross between Harbin 2-row (resistant) and Turkey 6 (susceptible) ("RI2 population" hereinafter); and DHHS population obtained from the cross between Haruna Nijo (resistant) and H602 (susceptible). As a result, two QTLs were found on 4H chromosome in RI1 population, one on 2H, 3H, and 5H chromosomes in RI2 population, and one on 1H chromosome in DHHS population. Genetic markers linked to these QTLs were also found.

The present invention was made based on these new findings. Specifically, the present invention provides:
A genetic marker that resides in 1H chromosome of barley and is linked to a gene locus involved in barley resistance to yellow mosaic disease, wherein the genetic marker is amplified with a first primer set that comprises a primer having the base sequence of SEQ ID NO: 1 and a primer having the base sequence of SEQ ID NO: 2;
A genetic marker that resides in 1H chromosome of barley and is linked to a gene locus involved in barley resistance to yellow mosaic disease, wherein the genetic marker is amplified with a second primer set that comprises a primer having the base sequence of SEQ ID NO: 3 and a primer having the base sequence of SEQ ID NO: 4;
A genetic marker that resides in 1H chromosome of barley and is linked to a gene locus involved in barley resistance to yellow mosaic disease, wherein the genetic marker is amplified with a fifth primer set that comprises a primer having the base sequence of SEQ ID NO: 19 and a primer having the base sequence of SEQ ID NO: 20;
A genetic marker that resides in 1H chromosome of barley and is linked to a gene locus involved in barley resistance to yellow mosaic disease, wherein the genetic marker is amplified with a sixth primer set that comprises a primer having the base sequence of SEQ ID NO: 21 and a primer having the base sequence of SEQ ID NO: 22;
A genetic marker that resides in 1H chromosome of barley and is linked to a gene locus involved in barley resistance to yellow mosaic disease, wherein the genetic marker is amplified with a seventh primer set that comprises a primer having the base sequence of SEQ ID NO: 23 and a primer having the base sequence of SEQ ID NO: 24;
A genetic marker that resides in 2H chromosome of barley and is linked to barley resistance to yellow mosaic disease, wherein the genetic marker is amplified by: ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50; pre-amplifying the ligated DNA fragment with an MseI universal primer having the base sequence of SEQ ID NO: 51, and an EcoRI universal primer having the base sequence of SEQ ID NO: 52; and amplifying the pre-amplified fragment with an eighth primer set that comprises a primer having the base sequence of SEQ ID NO: 25 and a primer having the base sequence of SEQ ID NO: 26;
A genetic marker that resides in 2H chromosome of barley and is linked to barley resistance to yellow mosaic disease, wherein the genetic marker is amplified a ninth primer set that comprises a primer having the base sequence of SEQ ID NO: 27 and a primer having the base sequence of SEQ ID NO: 28;
A genetic marker that resides in 3H chromosome of barley and is linked to barley resistance to yellow mosaic disease, wherein the genetic marker is amplified a third primer set that comprises a primer having the base sequence of SEQ ID NO: 5 and a primer having the base sequence of SEQ ID NO: 6;
A genetic marker that resides in 3H chromosome of barley and is linked to barley resistance to yellow mosaic disease, wherein the genetic marker is amplified a fourth primer set that comprises a primer having the base sequence of SEQ ID NO: 7 and a primer having the base sequence of SEQ ID NO: 8;
A genetic marker that resides in 3H chromosome of barley and is linked to barley resistance to yellow mosaic disease, wherein the genetic marker is amplified by: ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50; pre-amplifying the ligated DNA fragment with an MseI universal primer having the base sequence of SEQ ID NO: 51, and an EcoRI universal primer having the base sequence of SEQ ID NO: 52; and amplifying the pre-amplified fragment with a tenth primer set that comprises a primer having the base sequence of SEQ ID NO: 29 and a primer having the base sequence of SEQ ID NO: 30;
A genetic marker that resides in 3H chromosome of barley and is linked to barley resistance to yellow mosaic disease, wherein the genetic marker is amplified by: ligating a DNA fragment, obtained by digesting genomic DNA of barley with restriction enzymes MseI and EcoRI, to an MseI adapter having the base sequences of SEQ ID NO: 47 and 48, and an EcoRI. adapter having the base sequences of SEQ ID NO: 49 and 50; pre-amplifying the ligated DNA fragment with an MseI universal primer having the base sequence of SEQ ID NO: 51, and an EcoRI universal primer having the base sequence of SEQ ID NO: 52; and amplifying the pre-amplified fragment with an eleventh primer set that comprises a primer having the base sequence of SEQ ID NO: 31 and a primer having the base sequence of SEQ ID NO: 32;
A genetic marker that resides in 3H chromosome of barley and is linked to barley resistance to yellow mosaic disease, wherein the genetic marker is amplified a twelfth primer set that comprises a primer having the base sequence of SEQ ID NO: 33 and a primer having the base sequence of SEQ ID NO: 34;
A genetic marker that resides in 4H chromosome of barley and is linked to barley resistance to yellow mosaic disease, wherein the genetic marker is amplified by: ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50; pre-amplifying the ligated DNA fragment with an MseI universal primer having the base sequence of SEQ ID NO: 51, and an EcoRI universal primer having the base sequence of SEQ ID NO: 52; and amplifying the pre-amplified fragment with a thirteenth primer set that comprises a primer having the base sequence of SEQ ID NO: 35 and a primer having the base sequence of SEQ ID NO: 36;
A genetic marker that resides in 4H chromosome of barley and is linked to barley resistance to yellow mosaic disease, wherein the genetic marker is amplified by: ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50; pre-amplifying the ligated DNA fragment with an MseI universal primer having the base sequence of SEQ ID NO: 51, and an EcoRI universal primer having the base sequence of SEQ ID NO: 52; and amplifying the pre-amplified fragment with a fourteenth primer set that comprises a primer having the base sequence of SEQ ID NO: 37 and a primer having the base sequence of SEQ ID NO: 38;
A genetic marker that resides in 4H chromosome of barley and is linked to barley resistance to yellow mosaic disease, wherein the genetic marker is amplified by: ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50; pre-amplifying the ligated DNA fragment with an MseI universal primer having the base sequence of SEQ ID NO: 51, and an EcoRI universal primer having the base sequence of SEQ ID NO: 52; and amplifying the pre-amplified fragment with a fifteenth primer set that comprises a primer having the base sequence of SEQ ID NO: 39 and a primer having the base sequence of SEQ ID NO: 40;
A genetic marker that resides in 4H chromosome of barley and is linked to barley resistance to yellow mosaic disease, wherein the genetic marker is amplified by: ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50; pre-amplifying the ligated DNA fragment with an MseI universal primer having the base sequence of SEQ ID NO: 51, and an EcoRI universal primer having the base sequence of SEQ ID NO: 52; and amplifying the pre-amplified fragment with a sixteenth primer set that comprises a primer having the base sequence of SEQ ID NO: 41 and a primer having the base sequence of SEQ ID NO: 42;
A genetic marker that resides in 5H chromosome of barley and is linked to barley resistance to yellow mosaic disease, wherein the genetic marker is amplified by: ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50; pre-amplifying the ligated DNA fragment with an MseI universal primer having the base sequence of SEQ ID NO: 51, and an EcoRI universal primer having the base sequence of SEQ ID NO: 52; and amplifying the pre-amplified fragment with a seventeenth primer set that comprises a primer having the base sequence of SEQ ID NO: 43 and a primer having the base sequence of SEQ ID NO: 44;
A genetic marker that resides in 5H chromosome of barley and is linked to barley resistance to yellow mosaic disease, wherein the genetic marker is amplified by: ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50; pre-amplifying the ligated DNA fragment with an MseI universal primer having the base sequence of SEQ ID NO: 51, and an EcoRI universal primer having the base sequence of SEQ ID NO: 52; and amplifying the pre-amplified fragment with an eighteenth primer set that comprises a primer having the base sequence of SEQ ID NO: 45 and a primer having the base sequence of SEQ ID NO: 46.
A method for isolating a DNA fragment that includes a gene locus involved in barley resistance to yellow mosaic disease, using any of the foregoing genetic markers;
A method for producing a yellow mosaic disease-resistant barley, which comprises introducing a DNA fragment, isolated by the isolation method of the present invention and including a gene locus involved in barley resistance to yellow mosaic disease, into genomic DNA of barley;
A yellow mosaic disease-resistant barley produced by the producing method of the present invention;

A method for screening for a yellow mosaic disease-resistant barley, using any of the foregoing genetic markers as an index.

In another aspect, a genetic marker according to the present invention resides in the genomic DNA of barley and is linked to a gene locus involved in barley resistance to yellow mosaic disease, wherein the genetic marker is located within 0 to 18 centiMorgan of the gene locus involved in yellow mosaic disease. The genetic marker is strongly linked to the gene locus involved in barley resistance to yellow mosaic disease, and therefore the probability of recombination occurring between the gene locus of the genetic marker and the gene locus involved in barley resistance to yellow mosaic disease is small. The genetic marker can therefore be used to obtain a DNA fragment that includes a gene locus involved in barley resistance to yellow mosaic disease, for example. The genetic marker can also be used for the production or selection of yellow mosaic disease-resistant barley.

Further, a genetic marker according to the present invention resides in the genomic DNA of barley and is linked to a gene locus involved in barley resistance to yellow mosaic disease, wherein the genetic marker is located within 0 to 14 centiMorgan of the gene locus involved in yellow mosaic disease. The genetic marker is strongly linked to the gene locus involved in barley resistance to yellow mosaic disease, and therefore the probability of recombination occurring between the gene locus of the genetic marker and the gene locus involved in barley resistance to yellow mosaic disease is small. The genetic marker can therefore be used to obtain a DNA fragment that includes a gene locus involved in barley resistance to yellow mosaic disease, for example. The genetic marker can also be used for the production or selection of yellow mosaic disease-resistant barley.

Further, in a genetic marker according to the present invention, the genomic DNA comprises 1H chromosome. The genetic marker can therefore be used to obtain a DNA fragment that includes a 1H chromosome gene locus involved in barley resistance to yellow mosaic disease. The genetic marker can also be used for the production or selection of yellow mosaic disease-resistant barley.

Further, a genetic marker according to the present invention is amplified with a first primer set that comprises a primer having the base sequence of SEQ ID NO: 1 and a primer having the base sequence of SEQ ID NO: 2. Performing PCR or other amplification reactions with the first primer set conveniently allows for amplification and detection of the genetic marker, and thereby easily screen for yellow mosaic disease-resistant barley.

Further, a genetic marker according to the present invention is amplified with a second primer set that comprises a primer having the base sequence of SEQ ID NO: 3 and a primer having the base sequence of SEQ ID NO: 4. Performing PCR or other amplification reactions with the first primer set conveniently allows for amplification and detection of the genetic marker, and thereby easily screen for yellow mosaic disease-resistant barley.

Further, a genetic marker according to the present invention is amplified with a fifth primer set that comprises a primer having the base sequence of SEQ ID NO: 19 and a primer having the base sequence of SEQ ID NO: 20. Performing PCR or other amplification reactions with the first primer set conveniently allows for amplification and detection of the genetic marker, and thereby easily screen for yellow mosaic disease-resistant barley.

Further, a genetic marker according to the present invention is amplified with a sixth primer set that comprises a primer having the base sequence of SEQ ID NO: 21 and a primer having the base sequence of SEQ ID NO: 22. Performing PCR or other amplification reactions with the first primer set conveniently allows for amplification and detection of the genetic marker, and thereby easily screen for yellow mosaic disease-resistant barley.

Further, a genetic marker according to the present invention is amplified with a seventh primer set that comprises a primer having the base sequence of SEQ ID NO: 23 and a primer having the base sequence of SEQ ID NO: 24. Performing PCR or other amplification reactions with the first primer set conveniently allows for amplification and detection of the genetic marker, and thereby easily screen for yellow mosaic disease-resistant barley.

Further, in a genetic marker according to the present invention, the genomic DNA comprises 2H chromosome. The genetic marker can therefore be used to obtain a DNA fragment that includes a 2H chromosome gene locus involved in barley resistance to yellow mosaic disease. The genetic marker can also be used for the production or selection of yellow mosaic disease-resistant barley.

In order to achieve the foregoing object, a genetic marker according to the present invention is amplified by: ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50; pre-amplifying the ligated DNA fragment with an MseI universal primer having the base sequence of SEQ ID NO: 51, and an EcoRI universal primer having the base sequence of SEQ ID NO: 52; and amplifying the pre-amplified fragment with an eighth primer set that comprises a primer having the base sequence of SEQ ID NO: 25 and a primer having the base sequence of SEQ ID NO: 26. Performing PCR or other amplification reactions with the eighth primer set conveniently allows for amplification and detection of the genetic marker, and thereby easily screen for yellow mosaic disease-resistant barley.

Further, a genetic marker according to the present invention is amplified with a ninth primer set that comprises a primer having the base sequence of SEQ ID NO: 27 and a primer having the base sequence of SEQ ID NO: 28. Performing PCR or other amplification reactions with the ninth primer set conveniently allows for amplification and detection of the genetic marker, and thereby easily screen for yellow mosaic disease-resistant barley.

Further, in a genetic marker according to the present invention, the genomic DNA comprises 3H chromosome. The genetic marker can therefore be used to obtain a DNA fragment that includes a 3H chromosome gene locus involved in barley resistance to yellow mosaic disease. The genetic marker can also be used for the production or selection of yellow mosaic disease-resistant barley.

Further, a genetic marker according to the present invention is amplified with a third primer set that comprises a primer having the base sequence of SEQ ID NO: 5 and a primer having the base sequence of SEQ ID NO: 6. Performing PCR or other amplification reactions with the third primer set conveniently allows for amplification and detection of the genetic marker, and thereby easily screen for yellow mosaic disease-resistant barley.

Further, a genetic marker according to the present invention is amplified with a fourth primer set that comprises a primer having the base sequence of SEQ ID NO: 7 and a primer having the base sequence of SEQ ID NO: 8. Performing PCR or other amplification reactions with the ninth primer set conveniently allows for amplification and detection of the genetic marker, and thereby easily screen for yellow mosaic disease-resistant barley.

In order to achieve the foregoing object, a genetic marker according to the present invention is amplified by: ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50; pre-amplifying the ligated DNA fragment with an MseI universal primer having the base sequence of SEQ ID NO: 51, and an EcoRI universal primer having the base sequence of SEQ ID NO: 52; and amplifying the pre-amplified fragment with a tenth primer set that comprises a primer having the base sequence of SEQ ID NO: 29 and a primer having the base sequence of SEQ ID NO: 30.

Performing PCR or other amplification reactions with the tenth primer set conveniently allows for amplification and detection of the genetic marker, and thereby easily screen for yellow mosaic disease-resistant barley.

In order to achieve the foregoing object, a genetic marker according to the present invention is amplified by: ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50; pre-amplifying the ligated DNA fragment with an MseI universal primer having the base sequence of SEQ ID NO: 51, and an EcoRI universal primer having the base sequence of SEQ ID NO: 52; and amplifying the pre-amplified fragment with an eleventh primer set that comprises a primer having the base sequence of SEQ ID NO: 31 and a primer having the base sequence of SEQ ID NO: 32.

Performing PCR or other amplification reactions with the eleventh primer set conveniently allows for amplification and detection of the genetic marker, and thereby easily screen for yellow mosaic disease-resistant barley.

Further, a genetic marker according to the present invention is amplified with a twelfth primer set that comprises a primer having the base sequence of SEQ ID NO: 33 and a primer having the base sequence of SEQ ID NO: 34. Performing PCR or other amplification reactions with the twelfth primer set conveniently allows for amplification and detection of the genetic marker, and thereby easily screen for yellow mosaic disease-resistant barley.

Further, in a genetic marker according to the present invention, the genomic DNA comprises 4H chromosome. The genetic marker can therefore be used to obtain a DNA fragment that includes a 4H chromosome gene locus involved in barley resistance to yellow mosaic disease. The genetic marker can also be used for the production or selection of yellow mosaic disease-resistant barley.

In order to achieve the foregoing object, a genetic marker according to the present invention is amplified by: ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50; pre-amplifying the ligated DNA fragment with an MseI universal primer having the base sequence of SEQ ID NO: 51, and an EcoRI universal primer having the base sequence of SEQ ID NO: 52; and amplifying the pre-amplified fragment with a thirteenth primer set that comprises a primer having the base sequence of SEQ ID NO: 35 and a primer having the base sequence of SEQ ID NO: 36.

Performing PCR or other amplification reactions with the thirteenth primer set conveniently allows for amplification and detection of the genetic marker, and thereby easily screen for yellow mosaic disease-resistant barley.

In order to achieve the foregoing object, a genetic marker according to the present invention is amplified by: ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50; pre-amplifying the ligated DNA fragment with an MseI universal primer having the base sequence of SEQ ID NO: 51, and an EcoRI universal primer having the base sequence of SEQ ID NO: 52; and amplifying the pre-amplified fragment with a fourteenth primer set that comprises a primer having the base sequence of SEQ ID NO: 37 and a primer having the base sequence of SEQ ID NO: 38.

Performing PCR or other amplification reactions with the fourteenth primer set conveniently allows for amplification and detection of the genetic marker, and thereby easily screen for yellow mosaic disease-resistant barley.

In order to achieve the foregoing object, a genetic marker according to the present invention is amplified by: ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50; pre-amplifying the ligated DNA fragment with an MseI universal primer having the base sequence of SEQ ID NO: 51, and an EcoRI universal primer having the base sequence of SEQ ID NO: 52; and amplifying the pre-amplified fragment with a fifteenth primer set that comprises a primer having the base sequence of SEQ ID NO: 39 and a primer having the base sequence of SEQ ID NO: 40.

Performing PCR or other amplification reactions with the fifteenth primer set conveniently allows for amplification and detection of the genetic marker, and thereby easily screen for yellow mosaic disease-resistant barley.

In order to achieve the foregoing object, a genetic marker according to the present invention is amplified by: ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50; pre-amplifying the ligated DNA fragment with an MseI universal primer having the base sequence of SEQ ID NO: 51, and an EcoRI universal primer having the base sequence of SEQ ID NO: 52; and amplifying the pre-amplified fragment with a sixteenth primer set that comprises a primer having the base sequence of SEQ ID NO: 41 and a primer having the base sequence of SEQ ID NO: 42.

Performing PCR or other amplification reactions with the sixteenth primer set conveniently allows for amplification and detection of the genetic marker, and thereby easily screen for yellow mosaic disease-resistant barley.

Further, in a genetic marker according to the present invention, the genomic DNA comprises 5H chromosome. The genetic marker can therefore be used to obtain a DNA fragment that includes a 5H chromosome gene locus involved in barley resistance to yellow mosaic disease. The genetic marker can also be used for the production or selection of yellow mosaic disease-resistant barley.

In order to achieve the foregoing object, a genetic marker according to the present invention is amplified by: ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50; pre-amplifying the ligated DNA fragment with an MseI universal primer having the base sequence of SEQ ID NO: 51, and an EcoRI universal primer having the base sequence of SEQ ID NO: 52; and amplifying the pre-amplified fragment with a seventeenth primer set that comprises a primer having the base sequence of SEQ ID NO: 43 and a primer having the base sequence of SEQ ID NO: 44.

Performing PCR or other amplification reactions with the seventeenth primer set conveniently allows for amplification and detection of the genetic marker, and thereby easily screen for yellow mosaic disease-resistant barley.

In order to achieve the foregoing object, a genetic marker according to the present invention is amplified by: ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50; pre-amplifying the ligated DNA fragment with an MseI universal primer having the base sequence of SEQ ID NO: 51, and an EcoRI universal primer having the base sequence of SEQ ID NO: 52; and amplifying the pre-amplified fragment with an eighteenth primer set that comprises a primer having the base sequence of SEQ ID NO: 45 and a primer having the base sequence of SEQ ID NO: 46.

Performing PCR or other amplification reactions with the eighteenth primer set conveniently allows for amplification and detection of the genetic marker, and thereby easily screen for yellow mosaic disease-resistant barley.

An isolation method of a DNA fragment according to the present invention is a method for isolating a DNA fragment that includes a gene locus involved in barley resistance to yellow mosaic disease, using a genetic marker of the present invention. A genetic marker according to the present invention is strongly linked to a gene locus involved in barley resistance to yellow mosaic disease. Thus, cloning DNA fragments with the genetic marker used as a target conveniently allows for isolation of a DNA fragment that includes a gene locus involved in barley resistance to yellow mosaic disease.

A producing method of a yellow mosaic disease-resistant barley according to the present invention is a method for introducing a DNA fragment, isolated by the isolation method of the present invention and including a gene locus involved in barley resistance to yellow mosaic disease, into genomic DNA of barley. The producing method of the present invention can therefore be used to produce a yellow mosaic disease-resistant barley.

A yellow mosaic disease-resistant barley according to the present invention is produced by the producing method of a yellow mosaic disease-resistant barley according to the present invention. In the yellow mosaic disease-resistant barley according to the present invention, it is ensured that individuals that are resistant to yellow mosaic disease are screened for both conveniently and reliably. This prevents the reduction in barley yield caused by yellow mosaic disease.

A screening method of a yellow mosaic disease-resistant barley according to the present invention is a method for screening for a yellow mosaic disease-resistant barley, using a genetic marker of the present invention as an index. The genetic marker is strongly linked to the gene locus involved in barley resistance to yellow mosaic disease, and therefore the probability of recombination occurring between the gene locus of the genetic marker and the gene locus involved in barley resistance to yellow mosaic disease is very small. Thus, by detecting the genetic marker, the genotype of a tested barley can easily be found with regard to the gene locus involved in barley resistance to yellow mosaic disease, with the result that individuals of interest are easily screened for.

A genetic marker according to the present invention is linked to a gene locus involved in barley resistance to yellow mosaic disease. The genetic marker can therefore be used as an index to breed barley with yellow mosaic disease-resistance. In other words, the genetic marker allows for efficient breeding of yellow mosaic disease-resistant barley. More specifically, since the target individuals can be screened for at the seeding stage, there is no need to cultivate barley in a soil that has actually been infected with yellow mosaic virus and then screen for target individuals by inspecting each individual in regard to the yellow mosaic disease-resistance. This reduces the breeding time. Further, since multiple gene loci can be screened for simultaneously, the genotype can be found more reliably as compared with the screening that relies on inspection. Another advantage is that the labor and cultivation area can be reduced.

Further, since the yellow mosaic disease-resistant barley that was selected with the use of the genetic marker as an index can be cultivated in a soil that is contaminated by yellow mosaic virus, a stable yield can be ensured.

For a fuller understanding of the nature and advantages of the invention, reference should be made to the ensuing detailed description taken in conjunction with the accompanying drawings.

### BRIEF DESCRIPTION OF DRAWINGS

Figure 1 is a view showing a base sequence of barley EST clone, baaklj14, and locations of primer sequences designed based on the base sequence of baaklj 14.
Figure 2 is a view showing SNP-containing portions of base sequences of H602 and Haruna Nijo in relation to genetic marker k00256.
Figure 3 is a view showing a base sequence of barley EST clone, bags32m16, and locations of primer sequences designed based on the base sequence of bags32m16.
Figure 4 is an electrophoretic image showing fragment length polymorphism of H602 and Haruna Nijo in relation to genetic marker k02948, in connection with Example 13.
Figure 5 is a view showing a base sequence of barley EST clone, bah41103, and locations of primer sequences designed based on the base sequence of bah41103.
Figure 6 is a view showing SNP-containing portions of base sequences of H602 and Haruna Nijo in relation to genetic marker k04143.
Figure 7 is a view showing a base sequence of barley EST clone, baak14i02, and locations of primer sequences designed based on the base sequence of baak14i02.
Figure 8 is a view showing SNP-containing portions of base sequences of H602 and Haruna Nijo in relation to genetic marker k00169.
Figure 9 is a view showing SNP-containing portions of base sequences of H602 and Haruna Nijo in relation to genetic marker k03616.
Figure 10 is a view showing SNP-containing portions of base sequences of H602 and Haruna Nijo in relation to genetic marker k02325.
Figure 11 is a view showing SNP-containing portions of base sequences of H602 and Haruna Nijo in relation to genetic marker k07966.
Figure 12 is an electrophoretic image showing the result of polymorphism detection that was performed on genetic marker FEggaMtgg116 in Example 2.
Figure 13 is an electrophoretic image showing the result of polymorphism detection that was performed on genetic marker FEgggMcaa585 in Example 3.
Figure 14 is an electrophoretic image showing the result of polymorphism detection that was performed on genetic marker MEcatMagc467 in Example 4.
Figure 15 is an electrophoretic image showing the result of polymorphism detection that was performed on genetic marker MEataMatg396 in Example 5.
Figure 16 is an electrophoretic image showing the result of polymorphism detection that was performed on genetic marker MMattEacg 162 in Example 6.
Figure 17 is an electrophoretic image showing the result of polymorphism detection that was performed on genetic marker FMataEgga331 in Example 7.
Figure 18 is an electrophoretic image showing the result of polymorphism detection that was performed on genetic marker FMacgEgat88 in Example 8.
Figure 19 is an electrophoretic image showing the result of polymorphism detection that was performed on genetic marker MMacgEgga74 in Example 9.
Figure 20 is an electrophoretic image showing the result of polymorphism detection that was performed on genetic marker FMaccEacg402 in Example 10.
Figure 21 is an electrophoretic image showing the result of polymorphism detection that was performed on genetic marker HVM36 in Example 11.
Figure 22 is an electrophoretic image showing the result of polymorphism detection that was performed on genetic marker k00256 in Example 12.
Figure 23 is an electrophoretic image showing the result of polymorphism detection that was performed on genetic marker k03861 in Example 14.
Figure 24 is an electrophoretic image showing the result of polymorphism detection that was performed on genetic marker k03616 in Example 15.
Figure 25 is an electrophoretic image showing the result of polymorphism detection that was performed on genetic marker k02325 in Example 16.
Figure 26 is an electrophoretic image showing the result of polymorphism detection that was performed on genetic marker k00169 in Example 17.
Figure 27 is an electrophoretic image showing the result of polymorphism detection that was performed on genetic marker k07966 in Example 18.
Figure 28 is an electrophoretic image showing the result of polymorphism detection that was performed on genetic marker k04143 in Example 19.

### BEST MODE FOR CARRYING OUT THE INVENTION

The following will describe one embodiment of the present invention. It should be appreciated that the present invention is not limited in any way by the following description.

The present invention relates to genetic markers linked to gene loci involved in barley resistance to yellow mosaic disease, and exemplary use thereof. In the following, description is made as to genetic markers of the invention, and exemplary use thereof.

### (1) Genetic Markers according to the Present Invention

Genetic markers according to the present invention may be any genetic markers that reside in the genomic DNA of barley and are linked to gene loci involved in resistance to yellow mosaic disease.

As mentioned above, barley yellow mosaic disease is a soilborne viral disease caused by BaYMV or BaMMV, with the involvement of the Phycomycete, *Polymyxa graminis,* acting as a carrier. When developed, it causes necrotic spots or yellowing on the leaves, in addition to other abnormalities such as a slow tillering rate or stunt growth, or even death. The seriousness of the disease is that once it occurs the soils stays contaminated even if the soil is rested for 4 to 5 years. Today, resistance to yellow mosaic disease has become a very important subject in breeding.

Barley resistance to yellow mosaic disease is determined by both quantitative traits and qualitative traits, and more than one gene locus is responsible for determining the resistance. Chromosomal locations of gene loci involved in quantitative traits can be estimated by a QTL analysis. The inventors of the present invention have previously developed barley genetic markers linked to gene loci involved in barley resistance to yellow mosaic disease. The following specifically describes such genetic markers. It should be noted here that genetic markers according to the present invention are not limited to those described below.

The inventors of the present invention constructed a high-density linkage map using a DHHS population obtained from the cross (F1) between malting barley "Haruna Nijo" and wildtype barley "H602." Specifically, genomic DNA of barley was amplified with primer sets that had been designed based on the barley EST (expressed sequence tag) sequences owned by the inventors, and the amplified fragments that had polymorphism in the fragment length or digestion patterns of restriction enzyme were specified between Haruna Nijo and H602. As a result, a linkage map was constructed that had about 490 gene loci with these DNA markers. Based on the linkage map and the data concerning yellow mosaic disease resistance of 93 individuals of the DHHS population observed by the inventors, a QTL analysis was performed for gene loci involved in barley resistance to yellow mosaic disease. As a result, a gene locus involved in barley resistance to yellow mosaic disease was found on 1H chromosome and 3H chromosome. Genetic markers according to the present invention include the closest two genetic markers flanking the 1H chromosome gene locus involved in barley resistance to yellow mosaic disease, and the closest two genetic markers flanking the 3H chromosome gene locus involved in barley resistance to yellow mosaic disease. By the inventors, the genetic markers linked to the 1H chromosome gene locus involved in barley resistance to yellow mosaic disease were named "k00256" and "k02948," and the genetic markers linked to the 3H chromosome gene locus involved in barley resistance to yellow mosaic disease "k04143" and "k00169."

The genetic marker k00256 is a DNA marker that is amplified with the genomic DNA of barley as a template, using a first primer set that includes:
a primer of the base sequence CTTGGCCTTGATCTTCTGCT (SEQ ID NO: 1); and
a primer of the base sequence GACCGTGTCAGGAAAGCAAT (SEQ ID NO: 2).

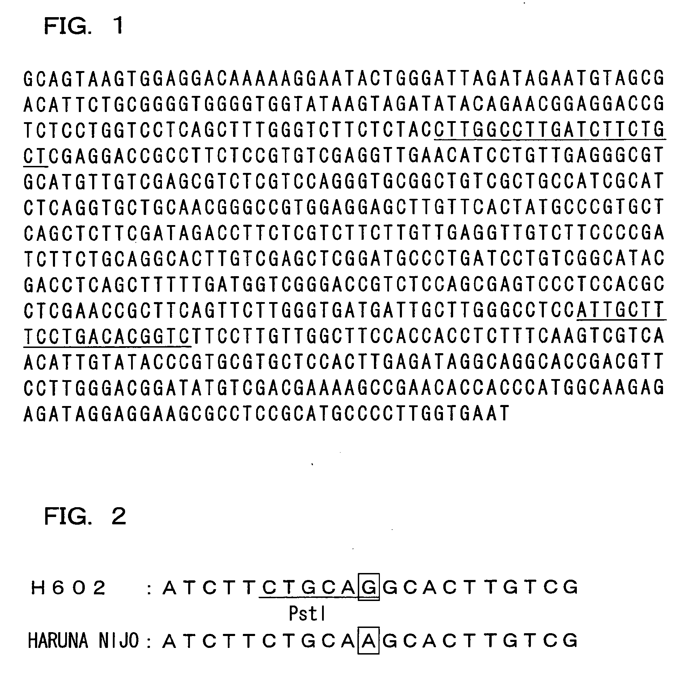

The DNA marker k00256 is located on the short-arm side of 1H chromosome about 7.8 centiMorgan (hereinafter "cM") from the gene locus involved in barley resistance to yellow mosaic disease. The primer sequences are based on one of the barley EST sequences (EST clone: baaklj14, SEQ ID NO: 15) independently developed by the inventors. Figure 1 shows the EST base sequence. The complementary sequences of the primer sequences (SEQ ID NO: 1) (SEQ ID NO: 2) are indicated by underline. Haruna Nijo and H602 have SNP (single nucleotide polymorphism) in the amplification products. Figure 2 shows SNP-containing portions of the base sequences of the amplification products of Haruna Nijo and H602. The base sequence (SEQ ID NO: 9) of the resistant strain (H602) is shown on the top, and the base sequence (SEQ ID NO: 10) of the susceptible strain (Haruna Nijo) is shown on the bottom. SNP is indicated by square. The recognition sequence (CTGCAG) of restriction enzyme PstI is indicated by underline. As can be seen in Figure 2, while the amplification product of the resistant strain (H602) is excised by PstI due to the presence of the recognition sequence (CTGCAG) of restriction enzyme PstI, the restriction enzyme PstI does not cut the amplification product of the susceptible strain (Haruna Nijo) because the recognition sequence has been changed to CTGCAA by the G-A mutation. That is, the genetic marker k00256 is a CAPS (cleaved amplified polymorphic sequence) marker, which, by the amplification with the first primer set and the excision by restriction enzyme PstI, can determine the genotype of an individual of interest, whether it is resistant or susceptible, in regard to the allele located on the 1H chromosome gene locus involved in barley resistance to yellow mosaic disease.

Note that, the genetic marker is linked to the gene locus involved in the yellow mosaic disease resistance of H602, which is a barley variety susceptible to yellow mosaic disease. Thus, in the detection of the genetic marker, Haruna Nijo is the susceptible strain, and H602 is the resistant strain.

The genetic marker k02948 is a DNA marker that is amplified with the genomic DNA of barley as a template, using a second primer set that includes:
a primer of the base sequence TCTTTCCTGGGTTGGTGAAC (SEQ ID NO: 3); and
a primer of the base sequence GCAGCTTTTGAGTTCGTTCC (SEQ ID NO: 4).

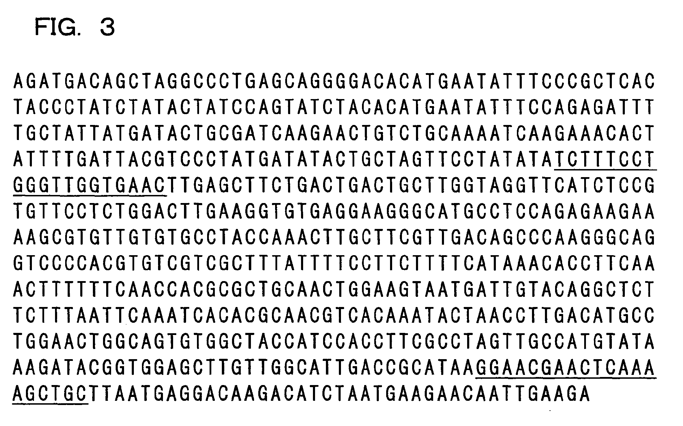

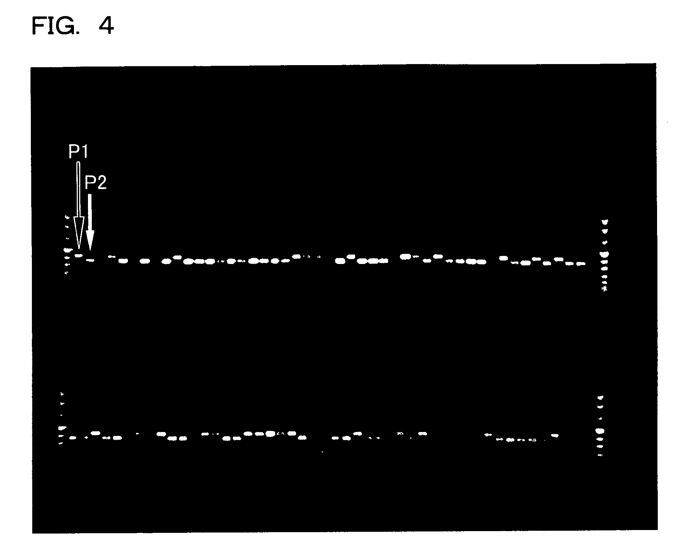

The DNA marker k02948 is located on the long-arm side of 1H chromosome about 0.0 cM from the gene locus involved in barley resistance to yellow mosaic disease. The primer sequences are based on one of the barley EST sequences (EST clone: bags32m16, SEQ ID NO: 16) independently developed by the inventors. Figure 3 shows the EST base sequence. The complementary sequences of the primer sequences (SEQ ID NO: 3) (SEQ ID NO: 4) are indicated by underline. Haruna Nijo and H602 have fragment length polymorphism in the amplification products. The size of amplified fragment obtained by using the genomic DNA of the susceptible strain (Haruna Nijo) as a template is about 389 bp, and the size of amplified fragment obtained by using the genomic DNA of the resistant strain (H602) as a template is about 358 bp. Figure 4 shows an electrophoretic image of fragments amplified by PCR using the second primer set. In the upper and lower parts of Figure 4, the both ends are molecular weight markers. The upper part of Figure 4 shows amplified fragments of the susceptible strain (Haruna Nijo), the resistant strain (H602), the F1 cross between Haruna Nijo and H602, and the DHHS population (1-45), in this order from the left (excluding the molecular weight marker). The lower part of Figure 4 shows amplified fragments of the DHHS population (46-93), in this order from the left (excluding the molecular weight marker). As can be seen in Figure 4, by checking the size of amplified fragments, the genotype of an individual of interest can be determined, whether it is susceptible (Haruna Nijo) or resistant (H602), in regard to the allele located on the 1H chromosome gene locus involved in barley resistance to yellow mosaic disease.

Note that, the genetic marker is linked to the gene locus involved in the yellow mosaic disease resistance of H602, which is a barley variety susceptible to yellow mosaic disease. Thus, in the detection of the genetic marker, Haruna Nijo is the susceptible strain, and H602 is the resistant strain.

The genetic marker k04143 is a DNA marker that is amplified with the genomic DNA of barley as a template, using a third primer set that includes:
a primer of the base sequence CTGTTTGGATGACTGCGAGA (SEQ ID NO: 5); and
a primer of the base sequence ATTACGCAACCTGATGGAGC (SEQ ID NO: 6).

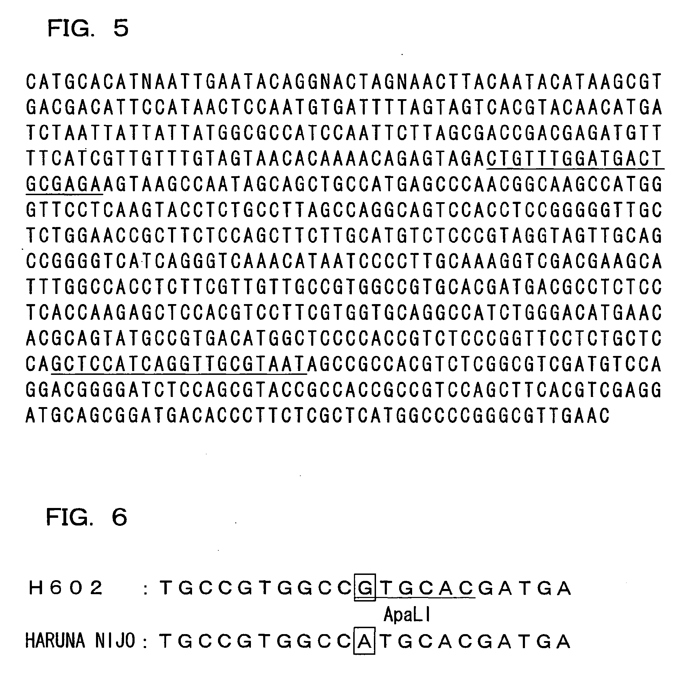

The DNA marker k04143 is located on the short-arm side of 3H chromosome about 0.0 cM to 13.1 cM from the gene locus involved in barley resistance to yellow mosaic disease. The primer sequences are based on one of the barley EST sequences (EST clone: bah41103, SEQ ID NO: 17) independently developed by the inventors. Figure 5 shows the EST base sequence. The complementary sequences of the primer sequences (SEQ ID NO: 5) (SEQ ID NO: 6) are indicated by underline. Haruna Nijo and H602 have SNP in the amplification products. Figure 6 shows SNP-containing portions of the base sequences of the amplification products of Haruna Nijo and H602. The base sequence (SEQ ID NO: 11) of the susceptible strain (H602) is shown on the top, and the base sequence (SEQ ID NO: 12) of the resistant strain (Haruna Nijo) is shown on the bottom. SNP is indicated by square. The recognition sequence (GTGCAC) of restriction enzyme ApaLI is indicated by underline. As can be seen in Figure 6, while the amplification product of the susceptible strain (H602) is excised by ApaLI due to the presence of the recognition sequence (GTGCAC) of restriction enzyme ApaLI, the restriction enzyme ApaLI does not cut the amplification product of the resistant strain (Haruna Nijo) because the recognition sequence has been changed to ATGCAC by the G-A mutation. That is, the genetic marker k04143 is a CAPS marker, which, by the amplification with the third primer set and the excision by restriction enzyme ApaLI, can determine the genotype of an individual of interest, whether it is resistant (Haruna Nijo) or susceptible (H602), in regard to the allele located on the 3H chromosome gene locus involved in barley resistance to yellow mosaic disease.

The genetic marker k00169 is a DNA marker that is amplified with the genomic DNA of barley as a template, using a fourth primer set that includes:
a primer of the base sequence ACCCCGGAAGCTAAGATGAT (SEQ ID NO: 7); and
a primer of the base sequence AGTCGGAACATGCGGTACAC (SEQ ID NO: 8).

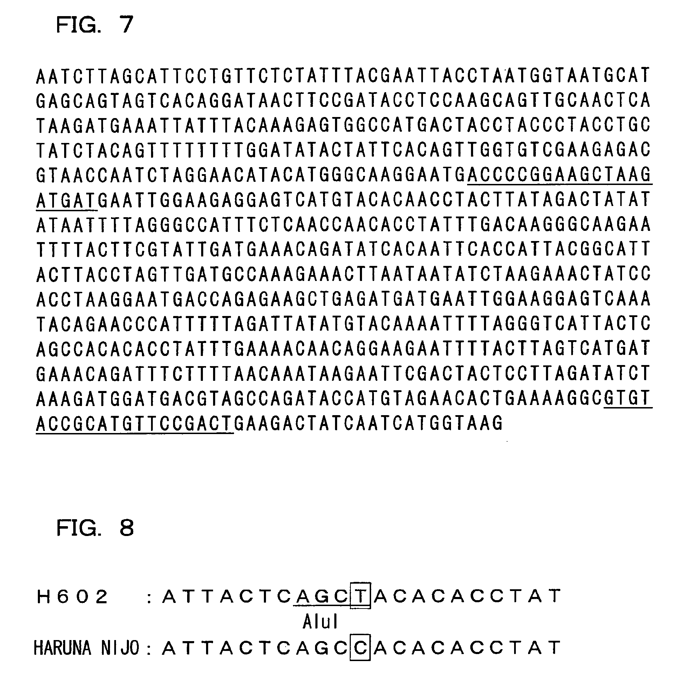

The DNA marker k00169 is located on the long-arm side of 3H chromosome about 0.0 cM to 13.4 cM from the gene locus involved in barley resistance to yellow mosaic disease. The primer sequences are based on one of the barley EST sequences (EST clone: baak14i02, SEQ ID NO: 18) independently developed by the inventors. Figure 7 shows the EST base sequence. The complementary sequences of the primer sequences (SEQ ID NO: 7) (SEQ ID NO: 8) are indicated by underline. Haruna Nijo and H602 have SNP in the amplification products. Figure 8 shows SNP-containing portions of the base sequences of the amplification products of Haruna Nijo and H602. The base sequence (SEQ ID NO: 13) of the susceptible strain (H602) is shown on the top, and the base sequence (SEQ ID NO: 14) of the resistant strain (Haruna Nijo) is shown on the bottom. SNP is indicated by square. The recognition sequence (AGCT) of restriction enzyme AluI is indicated by underline. As can be seen in Figure 8, while the amplification product of the susceptible strain (H602) is excised by AluI due to the presence of the recognition sequence (AGCT) of restriction enzyme AluI, the restriction enzyme AluI does not cut the amplification product of the resistant strain (Haruna Nijo) because the recognition sequence has been changed to AGCC by the T-C mutation. That is, the genetic marker k00169 is a CAPS marker, which, by the amplification with the fourth primer set and the excision by restriction enzyme AluI, can determine the genotype of an individual of interest, whether it is resistant (Haruna Nijo) or susceptible (H602), in regard to the allele located on the 3H chromosome gene locus involved in barley resistance to yellow mosaic disease.

Further, the inventors performed a QTL analysis on barley resistance to yellow mosaic disease, using RI line (RII population) obtained from the cross between Russia 6 (two-row, resistant) and H.E.S. 4 (six-row, susceptible); RI line (RI2 population) obtained from the cross between Harbin 2-row (resistant) and Turkey 6 (susceptible); and RI line (DHHS population) obtained from the cross between Haruna Nijo (resistant) and H602 (susceptible).

By the QTL analysis, QTLs were found in the following quantities: two on 4H chromosome in RI1 population; one each on 2H, 3H, and 5H chromosomes in RI2 population; and one on 1H chromosome in DHHS population. The QTL analysis also found novel genetic markers linked to these QTLs.

From the RI1 population, genetic markers FEggaMtgg116, FEgggMcaa585, MEcatMagc467, and MEataMatg396 were detected as novel genetic markers linked to the 4H chromosome gene locus involved in barley resistance to yellow mosaic disease, for example.

The genetic marker FEggaMtgg116 is detected by so-called AFLP (Amplified Fragment Length Polymorphism). For the detection, FEggaMtgg116 is amplified according to the following procedure. First, DNA fragments obtained by digesting the genomic DNA of barley with restriction enzymes MseI and EcoRI are ligated to MseI adapter having the base sequences GACGATGAGTCCTGAG (SEQ ID NO: 47) and TACTCAGGACTCAT (SEQ ID NO: 48), and EcoRI adapter having the base sequences CTCGTAGACTGCGTACC (SEQ ID NO: 49) and AATTGGTACGCAGTCTAC (SEQ ID NO: 50). The DNA fragments with these adopters are then pre-amplified with MseI universal primer having the base sequence GATGAGTCCTGAGTAA (SEQ ID NO: 51), and EcoRI universal primer having the base sequence GACTGCGTACCAATTC (SEQ ID NO: 52). Finally, the resulting fragments are amplified with a thirteenth primer set that includes a primer having the base sequence GATGAGTCCTGAGTAATGG (SEQ ID NO: 35) and a primer having the base sequence GACTGCGTACCAATTCGGA (SEQ ID NO: 36).

The genetic marker FEggaMtgg116 is located on the short arm side (closer to the 5' end) of 4H chromosome about 4.0 cM from the gene locus involved in barley resistance to yellow mosaic disease.

Note that, the AFLP and the procedure of detecting AFLP are not particularly limited. For example, the method, or a modification thereof, described in the following publication can be used. Pieter Vos, Rene Hogers, Marjo Bleeker, Martin Reijans, Theo van de Lee, Miranda Hornes, Adrie Frijters, Jerina Pot, Johan Peleman, Martin Kuiper and Marc Zabeau. (1995) AFLP: a new technique for DNA fingerprinting. Nucleic Acids Research. 23:21:4407-4414. Further, PCR or other amplification reactions may be performed under ordinary conditions, or by setting suitable conditions.

The amplified fragments obtained with the thirteenth primer set include those from the resistant strain (Russia 6) and those from the susceptible strain (H.E.S. 4). The length of amplified fragment was 0 bp for the resistant strain (Russia 6), and about 116 bp for the susceptible strain (H.E.S. 4). In other words, the resistant strain (Russia 6) does not yield the amplified fragment of about 116 bp, and it is only obtained in the susceptible strain (H.E.S. 4). Thus, if the fragment length of the amplified fragments obtained by the AFLP detecting procedure were checked by a conventional method such as electrophoresis, it would be possible to determine the genotype of a barley individual of interest, whether it is resistant or susceptible, in regard to the allele of the 4H chromosome gene locus involved in barley resistance to yellow mosaic disease.

The genetic marker FEgggMcaa585 is detected by so-called AFLP (Amplified Fragment Length Polymorphism). For the detection, FEgggMcaa585 is amplified according to the following procedure. First, DNA fragments obtained by digesting the genomic DNA of barley with restriction enzymes MseI and EcoRI are ligated to MseI adapter having the base sequences GACGATGAGTCCTGAG (SEQ ID NO: 47) and TACTCAGGACTCAT (SEQ ID NO: 48), and EcoRI adapter having the base sequences CTCGTAGACTGCGTACC (SEQ ID NO: 49) and AATTGGTACGCAGTCTAC (SEQ ID NO: 50). The DNA fragments with these adopters are then pre-amplified with MseI universal primer having the base sequence GATGAGTCCTGAGTAA (SEQ ID NO: 51), and EcoRI universal primer having the base sequence GACTGCGTACCAATTC (SEQ ID NO: 52). Finally, the resulting fragments are amplified with a fourteenth primer set that includes a primer having the base sequence GATGAGTCCTGAGTAACAA (SEQ ID NO: 37) and a primer having the base sequence GACTGCGTACCAATTCGGG (SEQ ID NO: 38).

The genetic marker FEgggMcaa585 is located on the long arm side (closer to the 3' end) of 4H chromosome about 13.9 cM from the gene locus involved in barley resistance to yellow mosaic disease.

The amplified fragments obtained with the fourteenth primer set include those from the resistant strain (Russia 6) and those from the susceptible strain (H.E.S. 4). The length of amplified fragment was 0 bp for the resistant strain (Russia 6), and about 585 bp for the susceptible strain (H.E.S. 4). In other words, the resistant strain (Russia 6) does not yield the amplified fragment of about 585 bp, and it is only obtained in the susceptible strain (H.E.S. 4). Thus, if the fragment length of the amplified fragments obtained by the AFLP detecting procedure were checked by a conventional method such as electrophoresis, it would be possible to determine the genotype of a barley individual of interest, whether it is resistant or susceptible, in regard to the allele of the 4H chromosome gene locus involved in barley resistance to yellow mosaic disease.

The genetic marker MEcatMagc467 is detected by so-called AFLP (Amplified Fragment Length Polymorphism). For the detection, MEcatMagc467 is amplified according to the following procedure. First, DNA fragments obtained by digesting the genomic DNA of barley with restriction enzymes MseI and EcoRI are ligated to MseI adapter having the base sequences GACGATGAGTCCTGAG (SEQ ID NO: 47) and TACTCAGGACTCAT (SEQ ID NO: 48), and EcoRI adapter having the base sequences CTCGTAGACTGCGTACC (SEQ ID NO: 49) and AATTGGTACGCAGTCTAC (SEQ ID NO: 50). The DNA fragments with these adopters are then pre-amplified with MseI universal primer having the base sequence GATGAGTCCTGAGTAA (SEQ ID NO: 51), and EcoRI universal primer having the base sequence GACTGCGTACCAATTC (SEQ ID NO: 52). Finally, the resulting fragments are amplified with a fifteenth primer set that includes a primer having the base sequence GATGAGTCCTGAGTAAACG (SEQ ID NO: 39) and a primer having the base sequence GACTGCGTACCAATTCCAT (SEQ ID NO: 40).

The genetic marker MEcatMagc467 is located on the short arm side (closer to the 5' end) of 4H chromosome about 7.4 cM from the gene locus involved in barley resistance to yellow mosaic disease.

The amplified fragments obtained with the fifteenth primer set include those from the resistant strain (Russia 6) and those from the susceptible strain (H.E.S. 4). The length of amplified fragment was 467 bp for the resistant strain (Russia 6), and about 0 bp for the susceptible strain (H.E.S. 4). In other words, the susceptible strain (H.E.S. 4) does not yield the amplified fragment of about 467 bp, and it is only obtained in the resistant strain (Russia 6). Thus, if the fragment length of the amplified fragments obtained by the AFLP detecting procedure were checked by a conventional method such as electrophoresis, it would be possible to determine the genotype of a barley individual of interest, whether it is resistant or susceptible, in regard to the allele of the 4H chromosome gene locus involved in barley resistance to yellow mosaic disease.

The genetic marker MEataMatg396 is detected by so-called AFLP (Amplified Fragment Length Polymorphism). For the detection, MEataMatg396 is amplified according to the following procedure. First, DNA fragments obtained by digesting the genomic DNA of barley with restriction enzymes MseI and EcoRI are ligated to MseI adapter having the base sequences GACGATGAGTCCTGAG (SEQ ID NO: 47) and TACTCAGGACTCAT (SEQ ID NO: 48), and EcoRI adapter having the base sequences CTCGTAGACTGCGTACC (SEQ ID NO: 49) and AATTGGTACGCAGTCTAC (SEQ ID NO: 50). The DNA fragments with these adopters are then pre-amplified with MseI universal primer having the base sequence GATGAGTCCTGAGTAA (SEQ ID NO: 51), and EcoRI universal primer having the base sequence GACTGCGTACCAATTC (SEQ ID NO: 52). Finally, the resulting fragments are amplified with a sixteenth primer set that includes a primer having the base sequence GATGAGTCCTGAGTAAATG (SEQ ID NO: 41) and a primer having the base sequence GACTGCGTACCAATTCATA (SEQ ID NO: 42).

The genetic marker MEataMatg396 is located on the long arm side (closer to the 3' end) of 4H chromosome about 0.6 cM from the gene locus involved in barley resistance to yellow mosaic disease.

The amplified fragments obtained with the sixteenth primer set include those from the resistant strain (Russia 6) and those from the susceptible strain (H.E.S. 4). The length of amplified fragment was 396 bp for the resistant strain (Russia 6), and about 0 bp for the susceptible strain (H.E.S. 4). In other words, the susceptible strain (H.E.S. 4) does not yield the amplified fragment of about 396 bp, and it is only obtained in the resistant strain (Russia 6). Thus, if the fragment length of the amplified fragments obtained by the AFLP detecting procedure were checked by a conventional method such as electrophoresis, it would be possible to determine the genotype of a barley individual of interest, whether it is resistant or susceptible, in regard to the allele of the 4H chromosome gene locus involved in barley resistance to yellow mosaic disease.

From the RI2 population, genetic markers FMaccEacg402 and HVM36 were detected as novel genetic markers linked to the 2H chromosome gene locus involved in barley resistance to yellow mosaic disease, for example.

The genetic marker FMaccEacg402 is detected by so-called AFLP (Amplified Fragment Length Polymorphism). For the detection, FMaccEacg402 is amplified according to the following procedure. First, DNA fragments obtained by digesting the genomic DNA of barley with restriction enzymes MseI and EcoRI are ligated to MseI adapter having the base sequences GACGATGAGTCCTGAG (SEQ ID NO: 47) and TACTCAGGACTCAT (SEQ ID NO: 48), and EcoRI adapter having the base sequences CTCGTAGACTGCGTACC (SEQ ID NO: 49) and AATTGGTACGCAGTCTAC (SEQ ID NO: 50). The DNA fragments with these adopters are then pre-amplified with MseI universal primer having the base sequence GATGAGTCCTGAGTAA (SEQ ID NO: 51), and EcoRI universal primer having the base sequence GACTGCGTACCAATTC (SEQ ID NO: 52). Finally, the resulting fragments are amplified with an eighth primer set that includes a primer having the base sequence GATGAGTCCTGAGTAAACC (SEQ ID NO: 25) and a primer having the base sequence GACTGCGTACCAATTCACG (SEQ ID NO: 26). The genetic marker FMaccEacg402 is located on the short arm side (closer to the 5' end) of 2H chromosome about 2.3 cM from the gene locus involved in barley resistance to yellow mosaic disease.

The amplified fragments obtained with the eighth primer set include those from the susceptible strain (Harbin 2-row) and those from the resistant strain (Turkey 6). The length of amplified fragment was 0 bp for the susceptible strain (Harbin 2-row), and about 402 bp for the resistant strain (Turkey 6). In other words, the susceptible strain (Harbin 2-row) does not yield the amplified fragment of about 402 bp, and it is only obtained in the resistant strain (Turkey 6). Thus, if the fragment length of the amplified fragments obtained by the AFLP detecting procedure were checked by a conventional method such as electrophoresis, it would be possible to determine the genotype of a barley individual of interest, whether it is resistant or susceptible, in regard to the allele of the 2H chromosome gene locus involved in barley resistance to yellow mosaic disease.

Note that, the genetic marker is linked to the gene locus involved in the yellow mosaic disease resistance of Turkey 6, which is a barley variety resistant to yellow mosaic disease. Thus, in the detection of the genetic marker, Harbin 2-row is the susceptible strain, and Turkey 6 is the resistant strain.

The genetic marker HVM36 is amplified with a ninth primer set that includes a primer having the base sequence TCCAGCCGAACAATTTCTTG (SEQ ID NO: 27) and a primer having the base sequence AGTACTCCGACACCACGTCC (SEQ ID NO: 28), using the genomic DNA of barley as a template. The genetic marker HVM36 is located on the long arm side of 2H chromosome about 6.0 cM from the gene locus involved in barley resistance to yellow mosaic disease. The genetic marker HVM36 is known as a SSR (simple sequence repeat) marker. Further, the amplification method of HVM36 is not particularly limited and may be performed under suitable conditions.

The amplified fragments obtained with the ninth primer set include those from the susceptible strain (Harbin 2-row) and those from the resistant strain (Turkey 6). The length of amplified fragment was about 60 bp to 30 bp for the susceptible strain (Harbin 2-row), and about 90 bp to 60 bp for the resistant strain (Turkey 6). In other words, the susceptible strain (Harbin 2-row) yields the amplified fragment of about 60 bp to 30 bp, and the resistant strain (Turkey6) yields the amplified fragment of about 90 bp to 60 bp. Thus, if the fragment length of the amplified fragments obtained by the AFLP detecting procedure were checked by a conventional method such as electrophoresis, it would be possible to determine the genotype of a barley individual of interest, whether it is resistant or susceptible, in regard to the allele of the 2H chromosome gene locus involved in barley resistance to yellow mosaic disease.

Note that, the genetic marker is linked to the gene locus involved in the yellow mosaic disease resistance of Turkey 6, which is a barley variety susceptible to yellow mosaic disease. Thus, in the detection of the genetic marker, Harbin 2-row is the susceptible strain, and Turkey 6 is the resistant strain.

From the RI2 population, genetic markers MMattEacg162 and FMataEgga331 were detected as novel genetic markers linked to the 3H chromosome gene locus involved in barley resistance to yellow mosaic disease, for example.

The genetic marker MMattEacg162 is detected by so-called AFLP (Amplified Fragment Length Polymorphism). For the detection, MMattEacg162 is amplified according to the following procedure. First, DNA fragments obtained by digesting the genomic DNA of barley with restriction enzymes MseI and EcoRI are ligated to MseI adapter having the base sequences GACGATGAGTCCTGAG (SEQ ID NO: 47) and TACTCAGGACTCAT (SEQ ID NO: 48), and EcoRI adapter having the base sequences CTCGTAGACTGCGTACC (SEQ ID NO: 49) and AATTGGTACGCAGTCTAC (SEQ ID NO: 50). The DNA fragments with these adopters are then pre-amplified with MseI universal primer having the base sequence GATGAGTCCTGAGTAA (SEQ ID NO: 51), and EcoRI universal primer having the base sequence GACTGCGTACCAATTC (SEQ ID NO: 52). Finally, the resulting fragments are amplified with a tenth primer set that includes a primer having the base sequence GATGAGTCCTGAGTAAATT (SEQ ID NO: 29) and a primer having the base sequence GACTGCGTACCAATTCACG (SEQ ID NO: 30). The genetic marker MMattEacg162 is located on the short arm side (closer to the 5' end) of 3H chromosome about 0.6 cM from the gene locus involved in barley resistance to yellow mosaic disease.

The amplified fragments obtained with the tenth primer set include those from the resistant strain (Harbin 2-row) and those from the susceptible strain (Turkey 6). The length of amplified fragment was about 162 bp for the resistant strain (Harbin 2-row), and about 170 bp for the susceptible strain (Turkey 6). In other words, the resistant strain (Harbin 2-row) yields the amplified fragment of about 162 bp, and the susceptible strain (Turkey 6) yields the amplified fragment of about 170 bp. Thus, if the fragment length of the amplified fragments obtained by the AFLP detecting procedure were checked by a conventional method such as electrophoresis, it would be possible to determine the genotype of a barley individual of interest, whether it is resistant or susceptible, in regard to the allele of the 3H chromosome gene locus involved in barley resistance to yellow mosaic disease.

The genetic marker FMataEgga331 is detected by so-called AFLP (Amplified Fragment Length Polymorphism). For the detection, FMataEgga331 is amplified according to the following procedure. First, DNA fragments obtained by digesting the genomic DNA of barley with restriction enzymes MseI and EcoRI are ligated to MseI adapter having the base sequences GACGATGAGTCCTGAG (SEQ ID NO: 47) and TACTCAGGACTCAT (SEQ ID NO: 48), and EcoRI adapter having the base sequences CTCGTAGACTGCGTACC (SEQ ID NO: 49) and AATTGGTACGCAGTCTAC (SEQ ID NO: 50). The DNA fragments with these adopters are then pre-amplified with MseI universal primer having the base sequence GATGAGTCCTGAGTAA (SEQ ID NO: 51), and EcoRI universal primer having the base sequence GACTGCGTACCAATTC (SEQ ID NO: 52). Finally, the resulting fragments are amplified with an eleventh primer set that includes a primer having the base sequence GATGAGTCCTGAGTAAATA (SEQ ID NO: 31) and a primer having the base sequence GACTGCGTACCAATTCGGA (SEQ ID NO: 32). The genetic marker FMataEgga331 is located on the long arm side (closer to the 3' end) of 4H chromosome about 6.2 cM from the gene locus involved in barley resistance to yellow mosaic disease.

The amplified fragments obtained with the eleventh primer set include those from the resistant strain (Harbin 2-row) and those from the susceptible strain (Turkey 6). The length of amplified fragment was about 0 bp for the resistant strain (Harbin 2-row), and about 331 bp for the susceptible strain (Turkey 6). In other words, the resistant strain (Harbin 2-row) does not yield the amplified fragment of about 331 bp, and it is obtained only in the susceptible strain (Turkey 6). Thus, if the fragment length of the amplified fragments obtained by the AFLP detecting procedure were checked by a conventional method such as electrophoresis, it would be possible to determine the genotype of a barley individual of interest, whether it is resistant or susceptible, in regard to the allele of the 3H chromosome gene locus involved in barley resistance to yellow mosaic disease.

From the RI2 population, genetic markers FMacgEgat88 and MMacgEgga74 were detected as novel genetic markers linked to the 5H chromosome gene locus involved in barley resistance to yellow mosaic disease, for example.

The genetic marker FMacgEgat88 is detected by so-called AFLP (Amplified Fragment Length Polymorphism). For the detection, FMacgEgat88 is amplified according to the following procedure. First, DNA fragments obtained by digesting the genomic DNA of barley with restriction enzymes MseI and EcoRI are ligated to MseI adapter having the base sequences GACGATGAGTCCTGAG (SEQ ID NO: 47) and TACTCAGGACTCAT (SEQ ID NO: 48), and EcoRI adapter having the base sequences CTCGTAGACTGCGTACC (SEQ ID NO: 49) and AATTGGTACGCAGTCTAC (SEQ ID NO: 50). The DNA fragments with these adopters are then pre-amplified with MseI universal primer having the base sequence GATGAGTCCTGAGTAA (SEQ ID NO: 51), and EcoRI universal primer having the base sequence GACTGCGTACCAATTC (SEQ ID NO: 52). Finally, the resulting fragments are amplified with a seventeenth primer set that includes a primer having the base sequence GATGAGTCCTGAGTAAACG (SEQ ID NO: 43) and a primer having the base sequence GACTGCGTACCAATTCGAT (SEQ ID NO: 44). The genetic marker FMacgEgat88 is located on the short arm side (closer to the 5' end) of 5H chromosome about 3.8 cM from the gene locus involved in barley resistance to yellow mosaic disease.

The amplified fragments obtained with the seventeenth primer set include those from the resistant strain (Harbin 2-row) and those from the susceptible strain (Turkey 6). The length of amplified fragment was 0 bp for the resistant strain (Harbin 2-row), and about 88 bp for the susceptible strain (Turkey 6). In other words, the resistant strain (Harbin 2-row) does not yield the amplified fragment of about 88 bp, and it is only obtained in the susceptible strain (Turkey 6). Thus, if the fragment length of the amplified fragments obtained by the AFLP detecting procedure were checked by a conventional method such as electrophoresis, it would be possible to determine the genotype of a barley individual of interest, whether it is resistant or susceptible, in regard to the allele of the 5H chromosome gene locus involved in barley resistance to yellow mosaic disease.

The genetic marker MMacgEgga74 is detected by so-called AFLP (Amplified Fragment Length Polymorphism). For the detection, MMacgEgga74 is amplified according to the following procedure. First, DNA fragments obtained by digesting the genomic DNA of barley with restriction enzymes MseI and EcoRI are ligated to MseI adapter having the base sequences GACGATGAGTCCTGAG (SEQ ID NO: 47) and TACTCAGGACTCAT (SEQ ID NO: 48), and EcoRI adapter having the base sequences CTCGTAGACTGCGTACC (SEQ ID NO: 49) and AATTGGTACGCAGTCTAC (SEQ ID NO: 50). The DNA fragments with these adopters are then pre-amplified with MseI universal primer having the base sequence GATGAGTCCTGAGTAA (SEQ ID NO: 51), and EcoRI universal primer having the base sequence GACTGCGTACCAATTC (SEQ ID NO: 52). Finally, the resulting fragments are amplified with an eighteenth primer set that includes a primer having the base sequence GATGAGTCCTGAGTAAACG (SEQ ID NO: 45) and a primer having the base sequence GACTGCGTACCAATTCGGA (SEQ ID NO: 46). The genetic marker MMacgEgga74 is located on the long arm side (closer to the 3' end) of 5H chromosome about 17.1 cM from the gene locus involved in barley resistance to yellow mosaic disease.

The amplified fragments obtained with the eighteenth primer set include those from the resistant strain (Harbin 2-row) and those from the susceptible strain (Turkey 6). The length of amplified fragment was about 74 bp for the resistant strain (Harbin 2-row), and about 0 bp for the susceptible strain (Turkey 6). In other words, the susceptible strain (Turkey 6) does not yield the amplified fragment of about 74 bp, and it is obtained only in the resistant strain (Harbin 2-row). Thus, if the fragment length of the amplified fragments obtained by the AFLP detecting procedure were checked by a conventional method such as electrophoresis, it would be possible to determine the genotype of a barley individual of interest, whether it is resistant or susceptible, in regard to the allele of the 5H chromosome gene locus involved in barley resistance to yellow mosaic disease.

From the DHHS population, genetic markers k03861, k03616, and k02325 were detected as novel genetic markers linked to the 1H chromosome gene locus involved in barley resistance to yellow mosaic disease, for example.

The genetic marker k03861 is amplified with the fifth primer set that includes a primer having the base sequences ACGATCGATCAAAAGGACCA (SEQ ID NO: 19) and a primer having the base sequences AATCCGACGAAATCAACGAG (SEQ ID NO: 20), using the genomic DNA of barley as a template. The genetic marker k03861 is located on the long arm side of 1H chromosome about 15.5 cM from the gene locus involved in barley resistance to yellow mosaic disease. The primer sequences are based on one of the barley EST sequences (EST clone: bah27k23, SEQ ID NO: 53) independently developed by the inventors.

The amplification method of k03861 is not particularly limited and may be performed under suitable conditions.

The amplified fragments obtained with the fifth primer set include those from the susceptible strain (Haruna Nijo) and those from the resistant strain (H602). The length of amplified fragment was about 379 bp for the susceptible strain (Haruna Nijo), and about 353 bp for the resistant strain (H602). Thus, if the fragment length of the amplified fragments obtained by the AFLP detecting procedure were checked by a conventional method such as electrophoresis, it would be possible to determine the genotype of a barley individual of interest, whether it is resistant or susceptible, in regard to the allele of the 1H chromosome gene locus involved in barley resistance to yellow mosaic disease.

Note that, the genetic marker is linked to the gene locus involved in the yellow mosaic disease resistance of H602, which is a barley variety susceptible to yellow mosaic disease. Thus, in the detection of the genetic marker, Haruna Nijo is the susceptible strain, and H602 is the resistant strain.

The genetic marker k03616 is amplified with the sixth primer set that includes a primer having the base sequences CTCGATCATCAGCGACTTCA (SEQ ID NO: 21) and a primer having the base sequences GAAGAGGCACCTTCTGCAAC (SEQ ID NO: 22), using the genomic DNA of barley as a template. The genetic marker k03616 is located on the short arm side of 1H chromosome about 1.4 cM from the gene locus involved in barley resistance to yellow mosaic disease. The primer sequences are based on one of the barley EST sequences (EST clone: bah 13e15, SEQ ID NO: 54) independently developed by the inventors.

The amplification method of k03616 is not particularly limited and may be performed under suitable conditions.

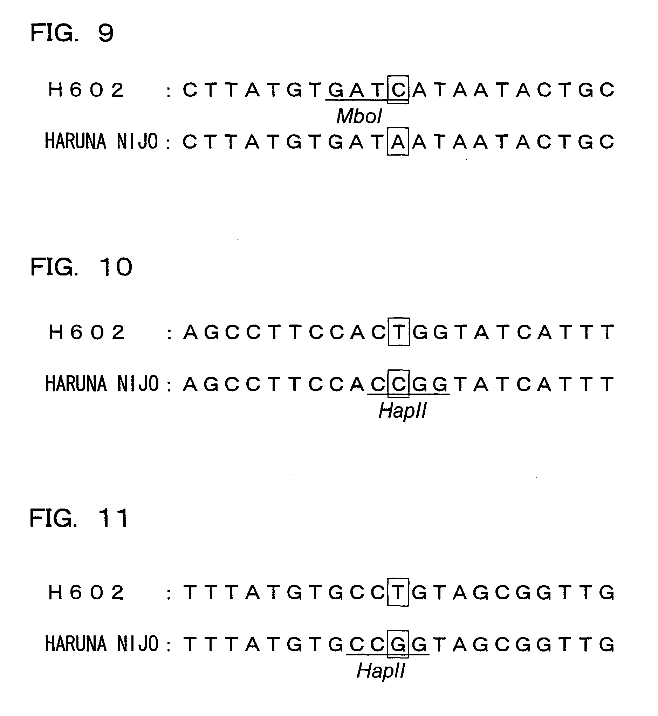

The amplified fragments obtained with the sixth primer set include those from the resistant strain (Haruna Nijo) and those from the susceptible strain (H602). Haruna Nijo and H602 have SNP. Figure 9 shows SNP-containing portions of the base sequences of the amplification products of Haruna Nijo and H602. The base sequence (SEQ ID NO: 55) of the susceptible strain (H602) is shown on the top, and the base sequence (SEQ ID NO: 56) of the resistant strain (Haruna Nijo) is shown on the bottom. SNP is indicated by square. The recognition sequence (GATC) of restriction enzyme MboI is indicated by underline. As can be seen in Figure 9, while the amplification product of the susceptible strain is excised by MboI due to the presence of the recognition sequence (GATC) of restriction enzyme MboI, the restriction enzyme MboI does not cut the amplification product of the resistant strain because the recognition sequence has been changed to GATA by the C-A mutation. That is, the genetic marker k03616 is a CAPS marker, which, by the amplification with the sixth primer set and the excision by restriction enzyme MboI, can determine the genotype of an individual of interest, whether it is resistant or susceptible, in regard to the allele located on the 1H chromosome gene locus involved in barley resistance to yellow mosaic disease.

The genetic marker k02325 is amplified with the seventh primer set that includes a primer having the base sequence AATGTGCACACCAAGGTTGA (SEQ ID NO: 23) and a primer having the base sequence AGACAACAACCGCCTGTACC (SEQ ID NO: 24), using the genomic DNA of barley as a template. The genetic marker k02325 is located on the long arm side of 1H chromosome about 4.3 cM from the gene locus involved in barley resistance to yellow mosaic disease. The primer sequences are based on one of the barley EST sequences (EST clone: bags1f22, SEQ ID NO: 57) independently developed by the inventors.

The amplification method of k02325 is not particularly limited and may be performed under suitable conditions.

The amplified fragments obtained with the seventh primer set include those from the resistant strain (Haruna Nijo) and those from the susceptible strain (H602). Haruna Nijo and H602 have SNP. Figure 10 shows SNP-containing portions of the base sequences of the amplification products of Haruna Nijo and H602. The base sequence (SEQ ID NO: 58) of the susceptible strain (H602) is shown on the top, and the base sequence (SEQ ID NO: 59) of the resistant strain (Haruna Nijo) is shown on the bottom. SNP is indicated by square. The recognition sequence (CCGG) of restriction enzyme HapII is indicated by underline. As can be seen in Figure 10, while the amplification product of the susceptible strain is excised by HapII due to the presence of the recognition sequence (CCGG) of restriction enzyme HapII, the restriction enzyme HapII does not cut the amplification product of the resistant strain because the recognition sequence has been changed to CTGG by the C-T mutation. That is, the genetic marker k02325 is a CAPS marker, which, by the amplification with the seventh primer set and the excision by restriction enzyme HapII, can determine the genotype of an individual of interest, whether it is resistant or susceptible, in regard to the allele located on the 1H chromosome gene locus involved in barley resistance to yellow mosaic disease.

The genetic marker k07966 is amplified with a twelfth primer set that includes a primer having the base sequence ATGGACCCAACAAGTGGAAG (SEQ ID NO: 33) and a primer having the base sequence AGGAAGACTTTGGAGGCCAT (SEQ ID NO: 34), using the genomic DNA of barley as a template. The genetic marker k07966 is located on the long arm side of 3H chromosome about 4.8 cM from the gene locus involved in barley resistance to yellow mosaic disease. The primer sequences are based on one of the barley EST sequences (EST clone: BaGS7G14, SEQ ID NO: 60) independently developed by the inventors.

The amplification method of k07966 is not particularly limited and may be performed under suitable conditions.

The amplified fragments obtained with the twelfth primer set include those from the resistant strain (Haruna Nijo) and those from the susceptible strain (H602). Haruna Nijo and H602 have SNP. Figure 11 shows SNP-containing portions of the base sequences of the amplification products of Haruna Nijo and H602. The base sequence (SEQ ID NO: 61) of the susceptible strain (H602) is shown on the top, and the base sequence (SEQ ID NO: 62) of the resistant strain (Haruna Nijo) is shown on the bottom. SNP is indicated by square. The recognition sequence (CCGG) of restriction enzyme HapII is indicated by underline. As can be seen in Figure 11, while the amplification product of the resistant strain is excised by HapII due to the presence of the recognition sequence (CCGG) of restriction enzyme HapII, the restriction enzyme HapII does not cut the amplification product of the susceptible strain because the recognition sequence has been changed to CCTG by the G-T mutation. That is, the genetic marker k07966 is a CAPS marker, which, by the amplification with the twelfth primer set and the excision by restriction enzyme HapII, can determine the genotype of an individual of interest, whether it is resistant or susceptible, in regard to the allele located on the 3H chromosome gene locus involved in barley resistance to yellow mosaic disease.

One centiMorgan (cM) refers to the distance between two gene loci with the crossover frequency of 1%. For example, 1.4 cM indicates that a recombination on a chromosome occurs on average at 14/ 1000 between a genetic marker and a gene locus involved in barley resistance to yellow mosaic disease. In other words, a recombination rate of about 1.4%.

The genomic DNA used for amplification can be extracted from plants, using conventional methods. For a preferable example of an ordinary method for extracting genomic DNA from plants, refer to Murray, M.G. and W.F. Thompson (1980) Nucleic Acids Res.8: 4321-4325, for example. The genomic DNA can be extracted from any tissue of barley, including root, stem, leaf, and reproductive organs. Under certain conditions, the genomic DNA may be extracted from the callus of barley. The reproductive organs include floral organs (both male and female reproductive organs) and seeds. For example, extraction of genomic DNA is performed with a barley leaf obtained from the seeding stage of development. This is because the leaf obtained from the seeding stage (i) allows the tissue to be ground relatively easily, (ii) contains a relatively small amount of impurity such as polysaccharides, and (iii) is easy to grow from the seed in a short time period. Another advantage is that it allows for screening of individuals at the seeding stage, and therefore greatly reduces the breeding time.

The method of amplification whereby the genomic DNA of barley is used as a template and the primers are used in the foregoing combinations can be performed by conventional DNA amplification methods. Generally, a PCR method (polymerase chain reaction) or a modification of PCR is used. Reaction conditions of PCR, or a modification thereof, are not particularly limited, and the method can be performed under ordinary conditions.

With the genetic markers according to the present invention, DNA fragments can be isolated that include gene loci involved in barley resistance to yellow mosaic disease. The DNA fragments can therefore be used to elucidate the mechanism of genes involved in barley resistance to yellow mosaic disease, and the mechanism of barley resistance to yellow mosaic disease. Further, by introducing the DNA fragments in the genomic DNA of barley, barley can be produced (bred) that is resistant to yellow mosaic disease.

Further, the genetic markers are linked to a gene locus involved in barley resistance to yellow mosaic disease. Thus, by detecting polymorphism of the genetic markers in the genomic DNA of tested barley, it is possible to determine whether the barley has a gene locus involved in resistance to yellow mosaic disease. Further, in this way, whether or not the barley is resistant to yellow mosaic disease can be determined. The primers for amplifying the genetic markers, or a DNA microarray with the genetic markers immobilized thereon may be provided as a kit, which can then be used for determining the presence or absence of a gene locus involved in barley resistance to yellow mosaic disease, or for determining a yellow mosaic disease-resistant barley.

As described above, genetic markers according to the present invention have many uses. Exemplary uses of genetic markers of the present invention will be described later in detail.

### (1) Use of the Present Invention

### [Isolation Method of DNA Fragments including Gene Locus involved in Barley Resistance to Yellow Mosaic Disease]

As described above, the genetic markers (k00256, k02948, k03861, k03616, k02325) according to the present invention are linked to a barley 1H chromosome gene locus involved in barley resistance to yellow mosaic disease. The genetic markers (FMaccEacg402, HVM36) according to the present invention are linked to a barley 2H chromosome gene locus involved in barley resistance to yellow mosaic disease. The genetic markers (k04143, k00169, k07966, MMattEacg162, FMataEgga331) according to the present invention are linked to a barley 3H chromosome gene locus involved in barley resistance to yellow mosaic disease. The genetic markers (FEggaMtgg116, FEgggMcaa585, MEcatMagc467, MEataMatg396) according to the present invention are linked to a barley 4H chromosome gene locus involved in barley resistance to yellow mosaic disease. The genetic markers (FMacgEgat88, MMacgEgga74) according to the present invention are linked to a barley 5H chromosome gene locus involved in barley resistance to yellow mosaic disease.

The genetic markers k00256, k02948, k03861, k03616, and k02325 can be used to isolate DNA fragments including a barley 1H chromosome gene locus involved in yellow mosaic disease. The genetic markers FMaccEacg402 and HVM36 can be used to isolate DNA fragments including a barley 2H chromosome gene locus involved in barley resistance to yellow mosaic disease. The genetic markers k04143, k00169, k07966, MMattEacg162, and FMataEgga331 can be used to isolate DNA fragments including a barley 3H chromosome gene locus involved in barley resistance to yellow mosaic disease. The genetic markers FEggaMtgg116, FEgggMcaa585, MEcatMagc467, and MEataMatg396 can be used to isolate DNA fragments including a barley 4H chromosome gene locus involved in barley resistance to yellow mosaic disease. The genetic markers FMacgEgat88 and MMacgEgga74 can be used to isolate DNA fragments including a barley 5H chromosome gene locus involved in barley resistance to yellow mosaic disease.

As used herein, the term "isolate" means not only cloning target DNA fragments including a gene locus involved in barley resistance to yellow mosaic disease, but the term is also used more broadly in situations where, for example, a backcross of the F1 population is made to screen for individuals that have DNA fragments including the gene locus involved in barley resistance to yellow mosaic disease of one of the parents, and then only these gene locus regions are introduced into a target variety to produce an isogenic line.

The method by which DNA fragments including a gene locus involved in barley resistance to yellow mosaic disease is isolated using the genetic markers is not particularly limited. For example, the following methods are available.

Currently, two kinds of BAC libraries of barley genomic DNA have been constructed, including one for Haruna Nijo being developed by the inventors of the present invention. Some more BAC libraries are under development as well. Such BAC libraries are used to find a gene locus involved in barley resistance to yellow mosaic disease. This can be performed according to- the following procedure. First, according to a known map base cloning technique, BAC clones including the genetic markers of the present invention are identified based on a gene locus involved in barley resistance to yellow mosaic disease and the genetic markers of the present invention linked thereto. Then, from the BAC clones so prepared, BAC contigs are prepared and sequenced.

Alternatively, as described above, by the backcross of F1 with one of the parents, DNA fragments may be obtained that include the gene locus involved in barley resistance to yellow mosaic disease of one of the parents. The DNA fragments can then be introduced into a target variety to achieve isolation in a broad sense.

For example, in using the foregoing methods to isolate DNA fragments that include a gene locus involved in barley resistance to yellow mosaic disease, it is preferable that genetic markers be used that are closer to the target gene locus involved in barley resistance to yellow mosaic disease. This reduces the chance of recombination between the genetic markers and the target gene locus involved in barley resistance to yellow mosaic disease, and thereby reliably isolates the gene locus involved in barley resistance to yellow mosaic disease.

### [Producing Method of Yellow Mosaic Disease-Resistant Barley, and Yellow Mosaic Disease-Resistant Barley Obtained by the Method]

A DNA fragment having a gene locus involved in barley resistance to yellow mosaic disease, obtained by the isolation method of DNA fragments including a gene locus involved in barley resistance to yellow mosaic disease, can be introduced into the genomic DNA of barley to produce a modified barley resistant to yellow mosaic disease. For the production of yellow mosaic disease-resistant barley, DNA fragments including a gene locus involved in barley resistance to yellow mosaic disease need to be isolated and introduced into a barley variety sensitive to yellow mosaic disease.

The method of introducing a DNA fragment is not particularly limited, and conventional methods can suitably be used. For example, an Agrobacterium method or a particle gun method can be used. Specifically, a transformed barley may be produced using *Agrobacterium tumefaciens*, according to the method described in Sonia Tingay et al., The Plant Journal (1997) 11(6), 1369-1376, for example.

The DNA fragment including a gene locus involved in barley resistance to yellow mosaic disease, introduced into barley to produce yellow mosaic disease-resistant barley may be any of DNA fragments of 1H, 2H, 3H, 4H, and 5H chromosomes. However, by introducing different kinds of DNA fragments into the genomic DNA of the same barley, the barley resistance to yellow mosaic disease can be improved.

Further, a DNA fragment including a gene locus involved in barley resistance to yellow mosaic disease may be introduced by crossing a genotypically resistant strain having a gene locus involved in barley resistance to yellow mosaic disease with a recipient strain sensitive to yellow mosaic disease.

A yellow mosaic disease-resistant barley according to the present invention is obtained by a producing method of the present invention. A producing method according to the present invention enables a yellow mosaic disease-resistant barley to be produced both easily and reliably, and therefore prevents yellow mosaic disease and provides a stable yield.

### [Determination Method (Screening Method) of Yellow Mosaic Disease-Resistant Barley]

A method for determining (screening for) a yellow mosaic disease-resistant barley according to the present invention is not particularly limited as long as it determines (screens for) a yellow mosaic disease-resistant barley using genetic markers of the present invention as an index. As such, the steps, conditions, materials, etc. are not particularly limited. For example, known crop breeding methods can be used.

More specifically, the genomic DNA of barley produced by a crossing etc. may be extracted, and a barley may be determined (screened for) using the genotype of genetic markers of the present invention as an index. For the detection of genetic markers, the fragment length or digestion patterns of restriction enzyme may be observed with regard to DNA fragments that have been amplified with any of the first through eighteenth primer sets using the extracted genomic DNA of tested barley as a template. From the amplified fragments, it is determined whether the tested barley is genotypically resistant or susceptible, with regard to the allele of the gene locus involved in barley resistance to yellow mosaic disease, using the genetic markers as an index. This was already described above in conjunction with the genetic markers according to the present invention.

The following description deals with accuracy (probability) of the determination method. Think of a genetic marker located 1.4 cM away from the gene locus involved in barley resistance to yellow mosaic disease. Then, chances of recombination occurring between the genetic marker and the gene locus involved in barley resistance to yellow mosaic disease on the chromatids are on average 14/1000. That is, the probability of recombination is about 1.4%. Thus, if the genetic marker for the gene locus involved in barley resistance to yellow mosaic disease were detected, there is a 98.6% chance that the gene locus involved in barley resistance to yellow mosaic disease is present. That is, the presence or absence of a gene locus involved in barley resistance to yellow mosaic disease can be determined more accurately if the distance between the genetic marker and the gene locus involved in barley resistance to yellow mosaic disease is close together. In other words, a yellow mosaic disease-resistant barley can be determined (screened for).

For the reasons set forth above, though any of the genetic markers for detecting polymorphism can be used to determine the presence or absence of yellow mosaic disease-resistance, it is preferable to detect genetic markers that are closer to the gene locus involved in barley resistance to yellow mosaic disease. For example, when a genetic marker linked to the 1H chromosome gene locus involved in barley resistance to yellow mosaic disease is detected by the determination method of the present invention, it is more preferable to detect k03616 (located about 1.4 cM from the gene locus involved in barley resistance to yellow mosaic disease) than k02325 (located about 4.3 cM from the gene locus involved in barley resistance to yellow mosaic disease).

Further, in the detection method, polymorphism of more than one genetic marker may be detected. Particularly, the accuracy (probability) of determination can be improved if polymorphism were detected by selecting genetic markers that flank a gene locus involved in barley resistance to yellow mosaic disease. For example, detection can be made with a combination of k03616 (located on the short arm side about 1.4 cM from the gene locus involved in barley resistance to yellow mosaic disease) and k02325 (located about 4.3 cM from the gene locus involved in barley resistance to yellow mosaic disease). By taking this as an example, the following specifically describes the accuracy (probability) of the determination method of the invention, in cases where polymorphism is detected with a single genetic marker and the both genetic markers.

Genetic marker k03616 is located on the short arm side about 1.4 cM from the gene locus involved in barley resistance to yellow mosaic disease, and the accuracy (probability) of the determination method of the invention is (1-14/ 1000) x 100 = about 98.6% when this genetic marker is solely used to detect polymorphism. Genetic marker k02325 is located about 4.3 cM from the gene locus involved in barley resistance to yellow mosaic disease, and the accuracy (probability) of the determination method of the invention is 95.7% when this genetic marker is solely used to detect polymorphism. The accuracy improves to (1-(14/1000) x (43/1000)) x 100 = 99.940% if polymorphism of the both genetic markers were detected, with the result that the presence or absence of a gene locus involved in barley resistance to yellow mosaic disease is detected with improved accuracy.

It is therefore preferable that determination be made by detecting genetic markers that flank a gene locus involved in barley resistance to yellow mosaic disease. Other than the combination of k03616 and k02325, the genetic markers may be used, for example, in the following combinations: FEggaMtgg116 and FEgggMcaa585; MEcatMagc467 and MEataMatg396; MMattEacg162 and FMataEgga331; FMacgEgat88 and MMacgEgga74; FMaccEacg402 and HVM36; k02948 and k03861; k00169 and k07966; k04143 and k00169; and k00256 and k02948.

The determination method of the present invention can be used as effective screening means for the breeding of yellow mosaic disease-resistant barley. For example, in performing the producing method of a yellow mosaic disease-resistant barley according to the present invention, the determination method readily allows large numbers of candidate transformants to be screened for individuals that have incorporated a DNA fragment including a gene locus involved in barley resistance to yellow mosaic disease. The determination method can also be used as means for screening barley individuals that have been subjected to chemical treatment or the like for causing mutation, or that have been bred by a crossing for example.

### <Determination Kit of Yellow Mosaic Disease-Resistant Barley>

The reagents, enzymes, and other materials necessary for the determination method of a yellow mosaic disease-resistant barley may be provided as a kit to provide a determination kit for determining a yellow mosaic disease-resistant barley (hereinafter referred to as "determination kit"). As described above in conjunction with the determination method of a yellow mosaic disease-resistant barley, genetic markers according to the present invention can be used to determine a genotype of a tested barley individual, whether it is resistant or susceptible, with regard to the allele of a gene locus involved in barley resistance to yellow mosaic disease. That is, it is possible to determine whether a tested barley individual is resistant or susceptible to yellow mosaic disease. Thus, with the determination kit, a yellow mosaic disease-resistant barley can be distinguished more easily.

Preferably, the determination kit includes one or more primer sets (first through eighteenth primer sets) that are used to detect at least genetic markers of the present invention. More preferably, the determination kit includes all of the primer sets (first through eighteenth primer sets). The determination kit may additionally include primers that are necessary for detecting known markers linked to barley resistance to yellow mosaic disease.

The determination kit may include enzymes, reagents, and the like for performing PCR. The kit may also include reagents, buffers, and a centrifugal tube that are necessary for preparing the genomic DNA used as a template. Further, the kit may include genetic markers (k00256, K02948, k04143, k00169, FEggaMtgg116, FEgggMcaa585, MEcatMagc467, MEataMatg396, MMattEacg162, FMataEgga331, FMacgEgat88, MMacgEgga74, FMaccEacg402, HVM36, k03861, k03616, k02325, k07966) that are necessary for the detection of target DNA size bands. Alternatively, the kit may include suitable DNA size markers.

### <Gene Detecting Instrument (DNA microarray)>

Genetic markers according to the present invention (k00256, K02948, k04143, k00169, FEggaMtgg116, FEgggMcaa585, MEcatMagc467, MEataMatg396, MMattEacg162, FMataEgga331, FMacgEgat88, MMacgEgga74, FMaccEacg402, HVM36, k03861, k03616, k02325, k07966) may be fixed on a suitable substrate (glass, silicon wafer, nylon membrane, etc.) to provide a gene detecting instrument such as a DNA microarray. By allowing the gene detecting instrument (DNA microarray) to react with a probe prepared from a tested barley and detecting a signal of the reaction, genetic markers of the present invention can be detected both easily and simultaneously. Thus, the gene detecting instrument (DNA microarray) can be used as means for detecting polymorphism of genetic markers of the present invention. The gene detecting instrument can therefore be used as detecting means in the determination method of a yellow mosaic disease-resistant barley. Further, the DNA microarray may be included in the determination kit for a yellow mosaic disease-resistant barley. In this case, the kit may include reagents, instruments, devices, and the like that are used to detect a signal from the gene detecting instrument (DNA microarray).

On the substrate of the gene detecting instrument (DNA microarray) of the present invention, there is fixed at least one of the genetic markers of the present invention (k00256, K02948, k04143, k00169, FEggaMtgg116, FEgggMcaa585, MEcatMagc467, MEataMatg396, MMattEacg162, FMataEgga331, FMacgEgat88, MMacgEgga74, FMaccEacg402, HVM36, k03861, k03616, k02325, k07966). The genetic marker may be either resistant or susceptible, or both. In order to more accurately (reliably) determine the presence or absence of a gene locus involved in barley resistance to yellow mosaic disease, it is preferable that a plurality of genetic markers flanking a gene locus involved in barley resistance to yellow mosaic disease be fixed in combinations. For example, the genetic markers may be fixed in the following combinations: k00256 and K02948; k04143 and k00169; FEggaMtgg116 and FEgggMcaa585; MEcatMagc467 and MEataMatg396; MMattEacg162 and FMataEgga331; FMacgEgat88 and MMacgEgga74; FMaccEacg402 and HVM36; K02948 and k038611; k03616 and k02325; and k00169 and k07966. Most preferably, all of the genetic markers (resistant and susceptible) are fixed.

With a gene detecting instrument (DNA microarray) having fixed thereon more than one genetic marker, a multiplicity of genetic markers can be easily detected in a single run. Further, the presence or absence of a gene locus involved in barley resistance to yellow mosaic disease can be detected with good accuracy (probability).

In addition to the genetic markers of the present invention, the gene detecting instrument (DNA microarray) may include other genetic markers fixed in the vicinity of the genetic markers of the present invention.

Further, in the gene detecting instrument (DNA microaray), it is preferable that the genetic markers of the present invention be fixed in the order they are aligned on barley chromosomes, or with sequence position information indicative of the order in which the genetic markers are aligned on barley chromosomes. This further improves detection accuracy. To describe more specifically, in analyses using a conventional microarray, one cannot be certain if failure to obtain a signal from a given spot is due to the absence of a genetic marker being detected, or if it is an experimental error. This necessitates further analysis. However, if the genetic markers were fixed on the gene detecting instrument (DNA microarray) in the chromosomal order or with sequence position information indicative of the chromosomal order, the order of the genetic markers fixed on the gene detecting instrument as they are aligned on the chromosomes can be checked to easily determine whether the result is due to an experimental error.

More specifically, assume that a signal was obtained in spots on the both sides of a spot where no signal was obtained. Meanwhile, in the gene detecting instrument (DNA microarray), the spots are disposed as they are aligned on the chromosomes. As a rule, in order for a recombination to occur in only one of closely aligned genes on a chromosome, two recombinations need to occur at very close locations. However, since the probability of such recombination is extremely small, failure to obtain a signal can be attributed to an experimental error. In this manner, with the gene detecting instrument (DNA microarray), whether the result is due to an experimental error can easily be determined even when a signal is not present in a given spot. As a result, the accuracy of analysis can be improved.

The present invention is not limited to the description of the embodiments above, but may be altered by a skilled person within the scope of the claims. An embodiment based on a proper combination of technical means disclosed in different embodiments is encompassed in the technical scope of the present invention.

The primers used in the description of the invention may have base sequences with the substitution, deletion, and/or addition of one or more bases, so long as the genetic markers of the present invention are amplified.

The following describes the present invention in more detail by way of Examples. It should be appreciated, however, that the present invention is not just limited to the following description.

### [Plants]

The pollens of F1 generation obtained from the cross between malting barley "Haruna Nijo" (Hordeum vulgare ssp. vulgare variety Harunanijo, resistant) and wildtype barley "H602" (Hordeum vulgare ssp. spontaneum H602, susceptible) were cultured to produce haploids. For the experiments, a naturally grown population (DHHS population) of 93 individuals of doubled haploids was used.

In addition, the following two different lines obtained from barley varieties with different levels of resistance to yellow mosaic disease were used: RI line (RI1 population) grown from the cross between Russia 6 (two-row, resistance) and H.E.S. 4 (six-row, susceptible); and RI line (RI2 population) grown from the cross between Harbin 2-row (resistance) and Turkey 6 (susceptible).

### [Construction of Linkage Map]

About 120,000 barley EST sequences owned by the inventors were basecalled again with phred. After trimming the sequences with a quality score of 20, about 60,000 sequences at the 3' end were obtained by vector masking. From these sequences, Unigene with 8,753 contigs and 6,686 siglets was obtained with phrap. Using the primer constructing software Primer 3, about 11,000 primer sets for amplifying a cDNA sequence of 149 bp to 490 bp centered on 400 bp were constructed. In order to detect polymorphism in the amplified fragments of Haruna Nijo and H602 genomes, the genomic DNA of Haruna Nijo and H602 was amplified by PCR, and for about 5,100 of the primer sets, agarose gel electrophoresis was performed to investigate the presence or absence of bands, the number of bands, and the band size. From the result of agarose gel electrophoresis, potential markers were selected. For fragments that did not have polymorphism in band size, the amplified fragments were directly sequenced to detect differences in the base sequences, and sequences that could be converted into CAPS (cleaved amplified polymorphic sequences) for 39 kinds of restriction enzymes were selected as markers.

In this manner, a linkage map with a total of 499 gene locus markers was constructed for the DHHS population obtained from the F1 hybrid between Haruna Nijo and H602. The linkage map had an average marker density of 3.0 cM/locus, and a total length of 1,470 cM.

### [Determination of Genotype]

The genotype of each individual of the DHHS population was determined with the markers mapped on the linkage map of the DHHS population. Specifically, PCR was performed with primer sets that had been designed based on the EST sequence. As a template, DNA separated from the tissue of a fresh leaf of each individual was used. For the fragment length polymorphism markers, the genotype was determined by performing electrophoresis on the PCR products. For the CAPS (cleaved amplified polymorphic sequence) markers, the genotype was determined by electrophoresis, after digesting the PCR product with restriction enzyme.

### [Assay for Yellow Mosaic Disease Resistance]

The following lines of barley were cultivated in a heavily contaminated field at the Research Institute for Bioresources, Okayama University: a group consisting of Russia 6, H.E.S. 4, and RI1 population; a group consisting of Harbin 2-row, Turkey 6, and RI2 population; and a group consisting of H602, Haruna Nijo, and DHHS population. In early March, mosaic lesions in each line were observed by naked eye. The results of observation were then scored to provide a scale for yellow mosaic disease resistance. The scores were classified as follows according to the extent of mosaic lesion.
Score 1 (Highly Resistant): No mosaic
Score 2 (Resistant): Some mosaics
Score 3 (Mildly Resistant): Mosaics are noticeable
Score 4 (Susceptible): Strong mosaic
Score 5 (Highly Susceptible): Mosaics are prominent

As controls, the following variants were used: six-row barley Mokusekko 3, native to China, highly resistant to yellow mosaic disease; and malting two-row barley "Amagi Nijo" highly susceptible to yellow mosaic disease.

### [Example 1: QTL Analysis on Barley Resistance to Yellow Mosaic Disease]

As the algorithms of QTL analysis, simple interval mapping ("SIM" hereinafter) and composite interval mapping ("CIM" hereinafter) were used. As the analysis software, MAPMAKER/QTL and QTL Cartographer were used, respectively. The threshold of LOD score was set to 2. For an LOD score exceeding 2, the presence of a QTL was estimated at a position between two markers where the LOD score was the greatest.

### [Results]

Tables 1, 2, and 3 show the results for the DHHS population, RI1 population, and RI2 population, respectively. In Tables 1 through 3, "Population" denotes the name of a population subjected to the QTL analysis, "Trait" means characteristic, "Chromosome" means the chromosome on which the genetic markers are located, "Marker interval (M1-A-QTL-B-M2)" means two genetic markers flanking a QTL in the vicinity thereof, "Distance (cM) A+B" means the distance between two genetic markers flanking a QTL, "Position^{a)} (cM)A" means the distance between genetic marker M1 and QTL, "Position (cM)B" means the distance between genetic marker M2 and QTL, "LOD^{b)} Score" means the peak value of LOD score, "Var. (%)^{c)} is the value that indicates what proportion (%) of the phenotype variance is accounted for by the presence of a QTL, and "Weight^{d)}" is the value indicative of the margin by which the score for the cut-spike test is increased due to the presence of a QTL.

**[Table 1]**

| Population | Trait | Algorithm | Chromosome | Marker interval (M1-A-QTL-B-M2) | Distance (cM)A+B | Position^{a)} (cM)A | Position (cM)B | LOD^{b)} score | Var.^{c)} (%) | Weight^{d)} |
|---|---|---|---|---|---|---|---|---|---|---|
| DHHS | Resistance to BaYMV | | | | | | | | | |
| | | SIM | 1H | k00256-k02948 | 7.8 | 7.8 | 0.0 | 2.5 | 12.5 | 0.4 |
| | | SIM | 3H | k04143-k00169 | 13.4 | 0.0 | 13.4 | 4.4 | 2.1 | -0.5 |
| | | CIM | 3H | k04143-k00169 | 13.4 | 13.1 | 0.3 | 2.2 | 34.7 | -0.9 |
| | | SIM | 1H | k02948-k03861 | 15.5 | 0.0 | 15.5 | 2.5 | 12.5 | 0.4 |
| | | CIM | 1H | k03616-k02325 | 5.7 | 1.4 | 4.3 | 2.6 | 16.3 | -0.6 |
| | | SIM | 3H | k00169-k07966 | 4.8 | 0.0 | 4.8 | 4.4 | 2.1 | -0.5 |
| | | CIM | 3H | k04143-k00169 | 13.1 | 13.1 | 0.0 | 3.5 | 30.6 | -0.8 |

| | | | | | | | | | | |
|---|---|---|---|---|---|---|---|---|---|---|
| a): Distance of peak LOD score position from the left side marker b): Peak LOD score of significant marker interval c): Explained variance of peak LOD score d): Estimated additive effect | | | | | | | | | | |

As can be seen from Table 1, SIM detected a QTL located between k00256 and k02948 on 1H chromosome, and a QTL located between k02948 and k03861 on 1H chromosome. It should be noted here that, since the linkage map has the markers k00256, k02948, and k03861 aligned in this order from the short arm side, the QTL detected by k00256 and k02948 and the QTL detected by k02948 and k03861 are on the same position (on k02948).

Further, SIM detected a QTL located between k04143 and k00169 on 3H chromosome, and a QTL located between k00169 and k07966 on 3H chromosome. Further, CIM detected a QTL located between k04143 and k00169 on 3H chromosome, and a QTL located between k03616 and k02325 on 1H chromosome. It should be noted here that, since the linkage map has the markers k04143, k00169, and k07966 aligned in this order from the short arm side of 3H chromosome, and since CIM indicates the presence of a QTL in very close proximity to K00169 (about 0.3 cM toward the long arm side from k00169), it is believed that the QTLs detected by SIM and CIM on 3H chromosome are the same QTL.

That is, the QTL analysis of gene loci involved in barley resistance to yellow mosaic disease revealed two QTLs on 1H chromosome: one located between k00256, k02948, and k03861, and one located between k03616 and k02325. The QTL analysis also detected a QTL on 3H chromosome, located between k04143, k00169, and k07966.

The QTL detected on 1H chromosome by SIM was located between genetic markers k00256, k02948, and k03861, at the distances of about 7.8 cM from the genetic marker k00256 on the short arm side, 0.0 cM from the genetic marker k02948, and 15.5 cM from the genetic marker k03861 on the short arm side. The LOD score was 2.5. The phenotype variance of 12.5% can be explained by this QTL. By the QTL, the score for yellow mosaic resistance decreased by 0.4.

The QTL detected on 3H chromosome by SIM was located between genetic markers k04143 and k00169, at the distances of 0.0 cM from the genetic marker k04143, and 13.4 cM from the genetic marker k00169 on the short arm side. The LOD score was 4.4. The phenotype variance of 2.1 % can be explained by this QTL. By the QTL, the score for yellow mosaic resistance increased by 0.5.

The other QTL detected on 3H chromosome by SIM was located between genetic markers k00169 and k07966, at the distances of 0.0 cM from the genetic marker k00169, and 4.8 cM from the genetic marker k07966 on the short arm side. The LOD score was 4.4. The phenotype variance of 2.1% can be explained by this QTL. By the QTL, the score for yellow mosaic resistance increased by 0.5.

The QTL detected on 1H chromosome by CIM was located between genetic markers k03616 and k02325, at the distances of 1.4 cM from the genetic marker k03616 on the long arm side, and 4.3 cM from the genetic marker k02325 on the short arm side. The LOD score was 2.6. The phenotype variance of 16.3% can be explained by this QTL. By the QTL, the score for yellow mosaic resistance increased by 0.6.

The detection of QTL on 3H chromosome by CIM was performed twice. In the first detection, the QTL was located between the genetic markers k04143 and k00169, at the distances of 13.1 cM from the genetic marker k04143 on the long arm side, and 0.0 cM from the genetic marker k00169. The LOD score was 2.2. The phenotype variance of 34.7% can be explained by this QTL. By the QTL, the score for yellow mosaic resistance increased by 0.9. In the second detection, the QTL was located between the genetic markers k04143 and k00169, at the distances of 13.1 cM from the genetic marker k04143 on the long arm side, and 0.0 cM from the genetic marker k00169. The LOD score was 3.5. The phenotype variance of 30.6% can be explained by this QTL. By the QTL, the score for yellow mosaic resistance increased by 0.8.

**[Table 2]**

| Population | Trait | Algorithm | Chromosome | Marker interval (M1-A-QTL-B-M2) | Distance (cM)A+B | Position^{a)} (cM)A | Position (cM)B | LOD^{b)} score | Var.^{c)} (%) | Weight^{d)} |
|---|---|---|---|---|---|---|---|---|---|---|
| R11 | Resistance to BaYMV | | | | | | | | | |
| | | SIM | 4H | FEggaMtgg116-FEggMcaa585 | 17.9 | 4.0 | 13.9 | 2.9 | 15.0 | -0.4 |
| | | CIM | 4H | MEcatMagc467-MEataMatg396 | 8.0 | 7.4 | 0.6 | 4.2 | 21.9 | -0.6 |

| | | | | | | | | | | |
|---|---|---|---|---|---|---|---|---|---|---|
| a): Distance of peak LOD score position from the left side marker b): Peak LOD score of significant marker interval c): Explained variance of peak LOD score d): Estimated additive effect | | | | | | | | | | |

As can be seen from Table 2, two QTLs involved in barley resistance to yellow mosaic disease were detected on 4H chromosome in the RI1 population.

One of the QTLs was located between the genetic markers FEggaMtgg116 and FEgggMcaa585, at the distances of 4.0 cM from the genetic marker FEggaMtgg116 on the long arm side, and 13.9 cM from the genetic marker FEgggMcaa585 on the short arm side. The LOD score was 2.9. The phenotype variance of 15.0% can be explained by this QTL. By the QTL, the score for yellow mosaic resistance increased by 0.4.

The other QTL was located between the genetic markers MEcatMagc467 and MEataMatg396, at the distances of 7.4 cM from the genetic marker MEcatMagc467 on the long arm side, and 0.6 cM from the genetic marker MEataMatg396 on the short arm side. The LOD score was 4.2. The phenotype variance of 21.9% can be explained by this QTL. By the QTL, the score for yellow mosaic resistance increased by 0.6.

**[Table 3]**

| Population | Trait | Algorithm | Chromosome | Marker interval (M1-A-QTL-B-M2) | Distance (cM)A+B | Position^{a)} (cM)A | Position (cM)B | LOD^{b)} score | Var.^{c)} (%) | Weight^{d)} |
|---|---|---|---|---|---|---|---|---|---|---|
| RI2 | Resistance to BaYMV | | | | | | | | | |
| | | SIM | 3H | MMattEacg162-FMataEgga331 | 6.8 | 0.6 | 6.2 | 2.5 | 11.6 | -0.6 |
| | | CIM | 5H | FMacgEgat88-MMacgEgga74 | 20.9 | 3.8 | 17.1 | 2.8 | 9.5 | -0.6 |
| | | CIM | 2H | FMaccEacg402-HVM36 | 9.3 | 2.3 | 6.0 | 3.4 | 18.7 | 0.6 |

| | | | | | | | | | | |
|---|---|---|---|---|---|---|---|---|---|---|
| a): Distance of peak LOD score position from the left side marker b): Peak LOD score of significant marker interval c): Explained variance of peak LOD score d): Estimated additive effect | | | | | | | | | | |

As can be seen from Table 3, the QTL involved in barley resistance to yellow mosaic disease was detected on each of 2H, 3H, and 5H chromosomes in the RI2 population.

The QTL on 2H chromosome was detected by CIM, and it was located between the genetic markers FMaccEacg402 and HVM36, at the distances of 2.3 cM from the genetic marker FMaccEacg402 on the long arm side, and 6.0 cM from the genetic marker HVM36 on the short arm side. The LOD score was 3.4. The phenotype variance of 18.7% can be explained by this QTL. By the QTL, the score for yellow mosaic resistance decreased by 0.6.

The QTL on 3H chromosome was detected by SIM, and it was located between the genetic markers MMattEacg162 and FMataEgga331, at the distances of 0.6 cM from the genetic marker MMattEacg162 on the long arm side, and 6.2 cM from the genetic marker FMataEgga331 on the short arm side. The LOD score was 2.5. The phenotype variance of 11.6% can be explained by this QTL. By the QTL, the score for yellow mosaic resistance increased by 0.6.

The QTL on 5H chromosome was detected by CIM, and it was located between the genetic markers FMacgEgat88 and MMacgEgga74, at the distances of 3.8 cM from the genetic marker FMacgEgat88 on the long arm side, and 17.1 cM from the genetic marker MMacgEgga74 on the short arm side. The LOD score was 2.8. The phenotype variance of 9.5% can be explained by this QTL. By the QTL, the score for yellow mosaic resistance increased by 0.6.

### [Example 2: Detection of Genetic Marker FEggaMtgg116]

### (Method)

The genomic DNA (50 ng) of tested barley was double digested at 37°C for 12 hours in a 25 µl reaction system including 1.5 U of EcoRI (TAKARA BIO INC.) and 1.5 U of MseI (NEW ENGLAND BioLabs). After the restriction enzyme treatment, the DNA was ligated at 37°C for 3 hours to 5 µM EcoRI adapter (base sequences of SEQ ID NO: 49 and 50) and 50 µM MseI adapter (base sequences of SEQ ID NO: 47 and 48), using 25 U T4 ligase (TAKARA BIO INC.). The DNA fragments after ligation were pre-amplified with a universal primer for EcoRI (base sequence of SEQ ID NO: 52) and a universal primer for MseI (base sequence of SEQ ID NO: 51). For 0.07 mg/ml of pre-amplification reaction solution, an amplification reaction (final amplification) was performed with a selective primer for EcoRI (base sequence of SEQ ID No: 36) and a selective primer for MseI (base sequence of SEQ ID No: 35). For the amplification, TaKaRa Ex Taq (TAKARA BIO INC.) was used.

The pre-amplification was performed with the following cycling parameters: one cycle consisting of 94°C for 2 minutes; and 20 cycles consisting of 94°C for 30 seconds, 56°C for 1 minute, and 72°C for 1 minute.

The final amplification was performed with the following cycling parameters: one cycle consisting of 94°C for 30 seconds, 68°C for 30 seconds, and 72°C for 1 minute; one cycle consisting of: of 94°C for 30 seconds, 68°C for 30 seconds, 72°C for 30 seconds, 94°C for 30 seconds, 67.3°C for 30 seconds, 72°C for 1 minute, 94°C for 30 seconds, 66.6°C for 30 seconds, 72°C for 1 minute, 94°C for 30 seconds, 65.9°C for 30 seconds, 72°C for 1 minute, 94°C for 30 seconds, 65.2°C for 30 seconds, 72°C for 1 minute, 94°C for 30 seconds, 64.5°C for 30 seconds, 72°C for 1 minute, 94°C for 30 seconds, 63.8°C for 30 seconds, 72°C for 1 minute, 94°C for 30 seconds, 63.1°C for 30 seconds, 72°C for 1 minute, 94°C for 30 seconds, 62.4°C for 30 seconds, 72°C for 1 minute, 94°C for 30 seconds, 61.7°C for 30 seconds, 72°C for 1 minute, 94°C for 30 seconds, 61.0°C for 30 seconds, 72°C for 1 minute, 94°C for 30 seconds, 60.3°C for 30 seconds, 72°C for 1 minute, 94°C for 30 seconds, 59.6°C for 30 seconds, 72°C for 1 minute, 94°C for 30 seconds, 58.9°C for 30 seconds, 72°C for 1 minute, 94°C for 30 seconds, 58.2°C for 30 seconds, 72°C for 1 minute, 94°C for 30 seconds, 57.5°C for 30 seconds, 72°C for 1 minute, 94°C for 30 seconds, 56.8°C for 30 seconds, and 72°C for 1 minute; 23 cycles consisting of 94°C for 30 seconds, 56.0°C for 30 seconds, and 72°C for 1 minute.

### (Results)

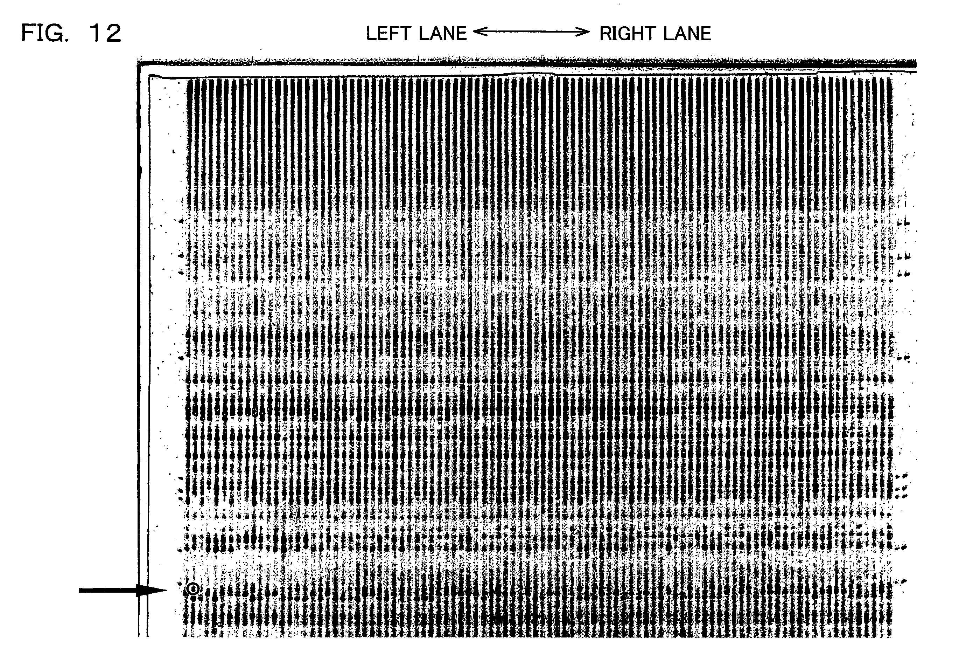

Figure 12 shows the result of electrophoresis performed on the amplified fragments of the amplification reaction. In Figure 12, the leftmost and rightmost lanes show size markers. The second lane from the left shows the result of AFLP detection on Russia 6, which is a barley variety resistant to yellow mosaic disease. The third lane from the left is the result of AFLP detection on H.E.S. 4, which is a barley variety susceptible to yellow mosaic disease. The other lanes are the results of AFLP detection on the RI1 population of inbred lines (RI lines) obtained from the cross between Russia 6 and H.E.S. 4.

The results shown in Figure 12 revealed that the amplified fragments obtained by the foregoing procedures were from both the resistant strain (Russia 6) and the susceptible strain (H.E.S. 4). The fragment length was 0 bp for the resistant strain (Russia 6), and about 116 bp for the susceptible strain (H.E.S. 4) (indicated by arrow in Figure 12). In other words, the amplified fragment of about 116 bp was obtained only for the susceptible strain (H.E.S. 4) but not for the resistant strain (Russia 6).

Thus, if AFLP were detected in the tested barley and the presence or absence of the amplified fragment of about 116 bp were used as an index, it would be possible to determine the genotype of tested barley, whether it is resistant or susceptible, with regard to the allele of the 4H chromosome gene locus involved in barley resistance to yellow mosaic disease.

### [Example 3: Detection of Genetic Marker FEgggMcaa585]

The methodology of Example 2 was used except that the amplification reaction (final amplification) was performed with selective primer for EcoPI (base sequence of SEQ ID NO: 38) and selective primer for MseI (base sequence of SEQ ID NO: 37).

### (Results)

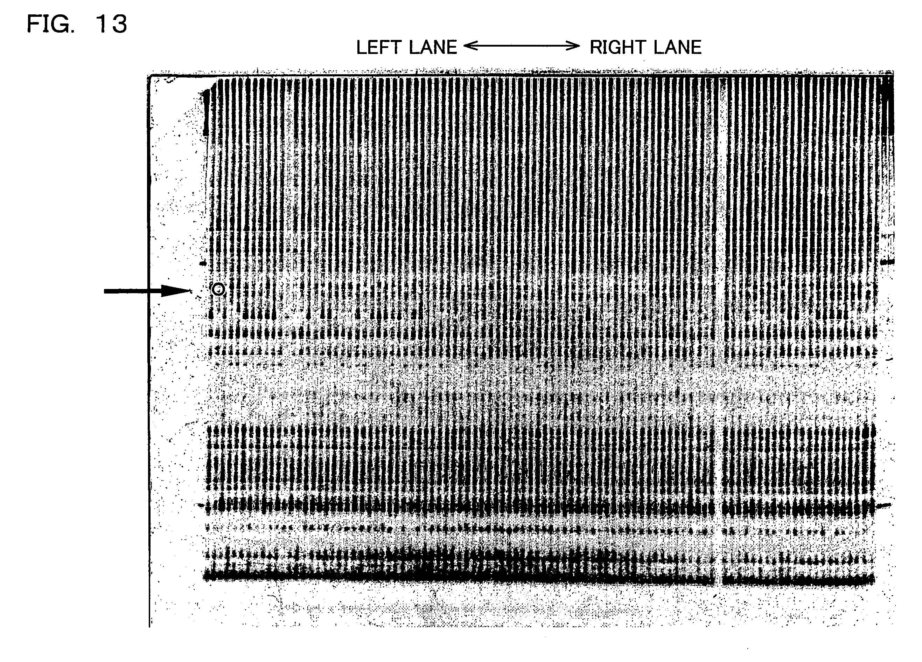

Figure 13 shows the result of electrophoresis performed on the amplified fragments of the amplification reaction. In Figure 13, the leftmost and rightmost lanes show size markers. The second lane from the left shows the result of AFLP detection on Russia 6, which is a barley variety resistant to yellow mosaic disease. The third lane from the left is the result of AFLP detection on H.E.S. 4, which is a barley variety susceptible to yellow mosaic disease. The other lanes are the results of AFLP detection on the RI1 population of inbred lines (RI lines) obtained from the cross between Russia 6 and H.E.S. 4.

The results shown in Figure 13 revealed that the amplified fragments obtained by the foregoing procedures were from both the resistant strain (Russia 6) and the susceptible strain (H.E.S. 4). The fragment length was 0 bp for the resistant strain (Russia 6), and about 585 bp for the susceptible strain (H.E.S. 4) (indicated by arrow in Figure 13). In other words, the amplified fragment of about 585 bp was obtained only for the susceptible strain (H.E.S. 4) but not for the resistant strain (Russia 6).

Thus, if AFLP were detected in the tested barley and the presence or absence of the amplified fragment of about 585 bp were used as an index, it would be possible to determine the genotype of the tested barley, whether it is resistant or susceptible, with regard to the allele of the 4H chromosome gene locus involved in barley resistance to yellow mosaic disease.

### [Example 4: Detection of Genetic Marker MEcatMagc467]

The methodology of Example 2 was used except that the amplification reaction (final amplification) was performed with selective primer for EcoPI (base sequence of SEQ ID NO: 40) and selective primer for MseI (base sequence of SEQ ID NO: 39).

### (Results)

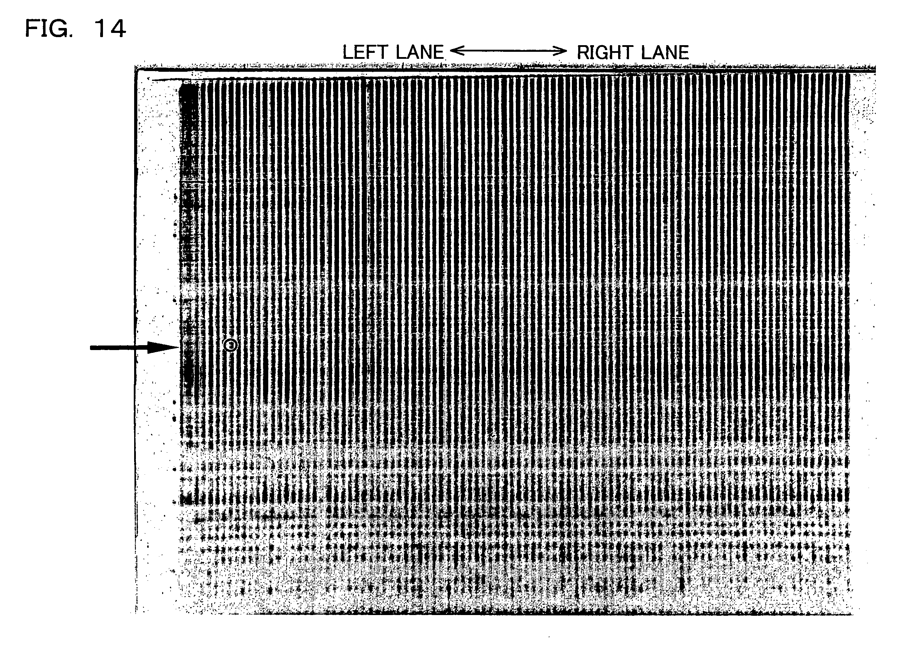

Figure 14 shows the result of electrophoresis performed on the amplified fragments of the amplification reaction. In Figure 14, the leftmost lane shows a size marker. The second lane from the left shows the result of AFLP detection on Russia 6, which is a barley variety resistant to yellow mosaic disease. The third lane from the left is the result of AFLP detection on H.E.S. 4, which is a barley variety susceptible to yellow mosaic disease. The other lanes are the results of AFLP detection on the RI1 population of inbred lines (RI lines) obtained from the cross between Russia 6 and H.E.S. 4.

The results shown in Figure 14 revealed that the amplified fragments obtained by the foregoing procedures were from both the resistant strain (Russia 6) and the susceptible strain (H.E.S. 4). The fragment length was 0 bp for the susceptible strain (H.E.S. 4) and about 467 bp for the resistant strain (Russia 6) (indicated by arrow in Figure 14). In other words, the amplified fragment of about 467 bp was obtained only for the resistant strain (Russia 6) but not for the susceptible strain (H.E.S. 4).

Thus, if AFLP were detected in the tested barley and the presence or absence of the amplified fragment of about 467 bp were used as an index, it would be possible to determine the genotype of the tested barley, whether it is resistant or susceptible, with regard to the allele of the 4H chromosome gene locus involved in barley resistance to yellow mosaic disease.

### [Example 5: Detection of Genetic Marker MEataMatg396]

The methodology of Example 2 was used except that the amplification reaction (final amplification) was performed with selective primer for EcoPI (base sequence of SEQ ID NO: 42) and selective primer for MseI (base sequence of SEQ ID NO: 41).

### (Results)

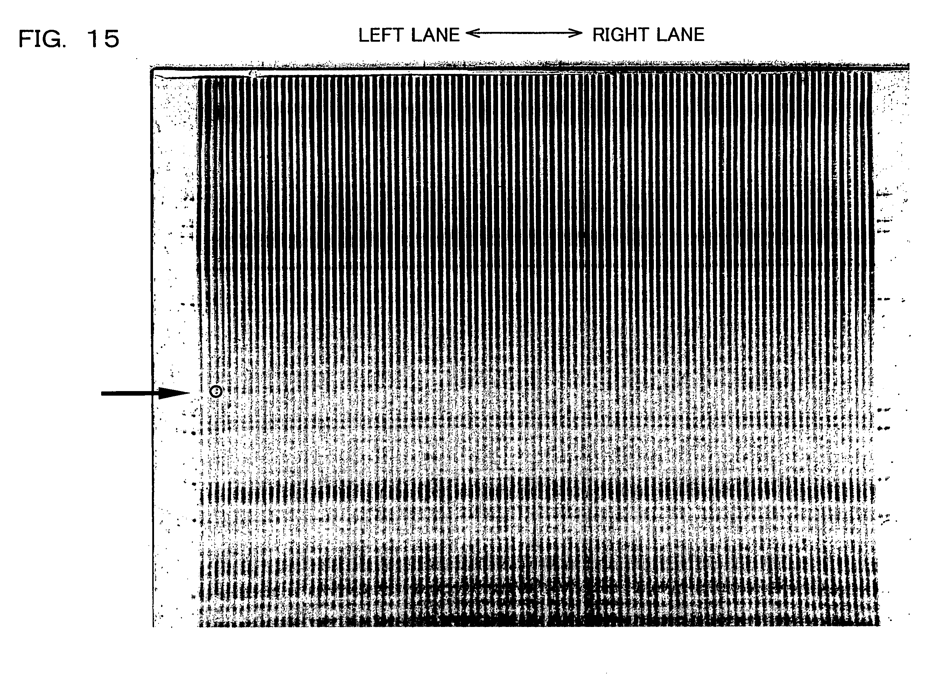

Figure 15 shows the result of electrophoresis performed on the amplified fragments of the amplification reaction. In Figure 15, the leftmost two lanes and the rightmost two lanes show size markers. The third lane from the left shows the result of AFLP detection on Russia 6, which is a barley variety resistant to yellow mosaic disease. The fourth lane from the left is the result of AFLP detection on H.E.S. 4, which is a barley variety susceptible to yellow mosaic disease. The electrophoretic image of these results is not clearly shown in Figure 15, however. The other lanes are the results of AFLP detection on the RI1 population of inbred lines (RI lines) obtained from the cross between Russia 6 and H.E.S. 4.

The results shown in Figure 15 revealed that the amplified fragments obtained by the foregoing procedures were from both the resistant strain (Russia 6) and the susceptible strain (H.E.S. 4). The fragment length was 0 bp for the susceptible strain (H.E.S. 4) and about 396 bp for the resistant strain (Russia 6) (indicated by arrow in Figure 15). In other words, the amplified fragment of about 396 bp was obtained only for the resistant strain (Russia 6) but not for the susceptible strain (H.E.S. 4).

Thus, if AFLP were detected in the tested barley and the presence or absence of the amplified fragment of about 396 bp were used as an index, it would be possible to determine the genotype of the tested barley, whether it is resistant or susceptible, with regard to the allele of the 4H chromosome gene locus involved in barley resistance to yellow mosaic disease.

### [Example 6: Detection of Genetic Marker MMattEacg162]

The methodology of Example 2 was used except that the amplification reaction (final amplification) was performed with selective primer for EcoPI (base sequence of SEQ ID NO: 30) and selective primer for MseI (base sequence of SEQ ID NO: 29).

### (Results)

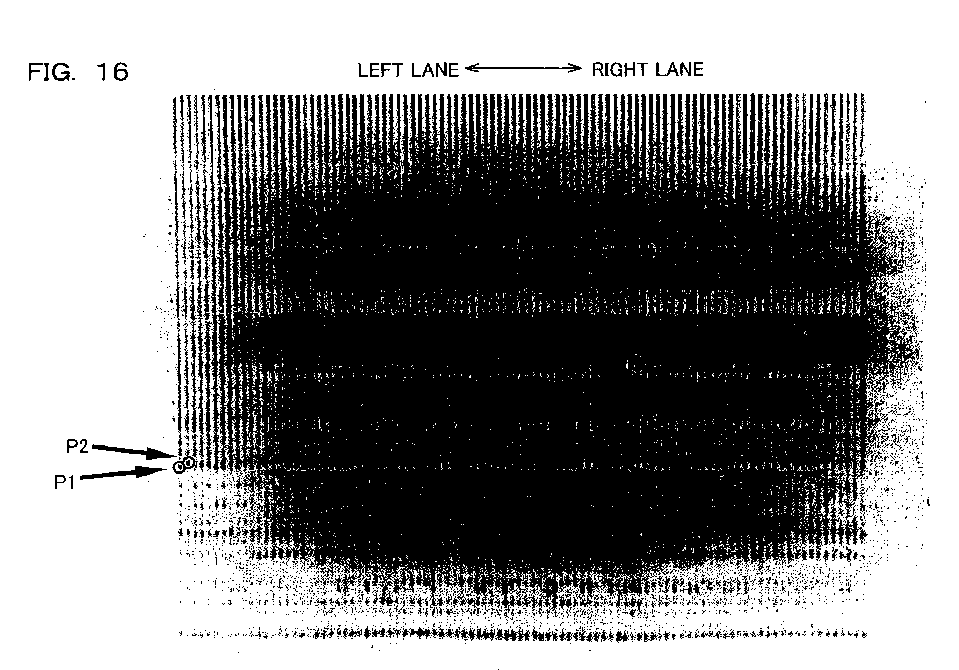

Figure 16 shows the result of electrophoresis performed on the amplified fragments of the amplification reaction. In Figure 16, the leftmost lane and the rightmost two lanes show size markers. The second lane from the left shows the result of AFLP detection on Harbin 2-row, which is a barley variety resistant to yellow mosaic disease. The third lane from the left is the result of AFLP detection on Turkey 6, which is a barley variety susceptible to yellow mosaic disease. The other lanes are the results of AFLP detection on the RI2 population of inbred lines (RI lines) obtained from the cross between Harbin 2-row and Turkey 6.

The results shown in Figure 16 revealed that the amplified fragments obtained by the foregoing procedures were from both the resistant strain (Harbin 2-row) and the susceptible strain (Turkey 6). The fragment length was about 162 bp for the resistant strain (Harbin 2-row) (indicated by arrow P1 in Figure 16) and about 170 bp for the susceptible strain (Turkey 6) (indicated by arrow P2 in Figure 16). In other words, the amplified fragment of about 162 bp was obtained only for the resistant strain (Harbin 2-row), and the amplified fragment of about 170 bp was obtained only for the susceptible strain (Turkey 6).

Thus, if AFLP were detected in the tested barley and the presence or absence of the amplified fragments of about 162 bp and about 170 bp were used as indices, it would be possible to determine the genotype of the tested barley, whether it is resistant or susceptible, with regard to the allele of the 3H chromosome gene locus involved in barley resistance to yellow mosaic disease.

### [Example 7: Detection of Genetic Marker FMataEgga331]

The methodology of Example 2 was used except that the amplification reaction (final amplification) was performed with selective primer for EcoPI (base sequence of SEQ ID NO: 32) and selective primer for MseI (base sequence of SEQ ID NO: 31).

### (Results)

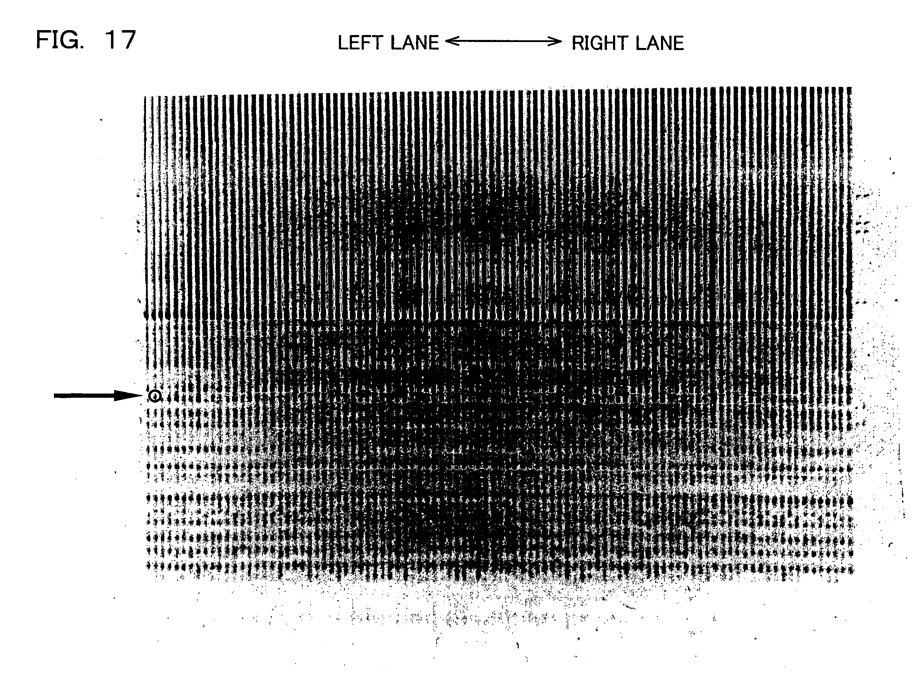

Figure 17 shows the result of electrophoresis performed on the amplified fragments of the amplification reaction. In Figure 17, the leftmost lane and the rightmost two lanes show size markers. The second lane from the left shows the result of AFLP detection on Harbin 2-row, which is a barley variety resistant to yellow mosaic disease. The third lane from the left is the result of AFLP detection on Turkey 6, which is a barley variety susceptible to yellow mosaic disease. The other lanes are the results of AFLP detection on the RI2 population of inbred lines (RI lines) obtained from the cross between Harbin 2-row and Turkey 6.

The results shown in Figure 17 revealed that the amplified fragments obtained by the foregoing procedures were from both the resistant strain (Harbin 2-row) and the susceptible strain (Turkey 6). The fragment length was about 331 bp for the susceptible strain (Turkey 6) (indicated by arrow in Figure 16), and 0 bp for the resistant strain (Harbin 2-row). In other words, the amplified fragment of about 331 bp was obtained only for the susceptible strain (Turkey 6) but not for the resistant strain (Harbin 2-row).

Thus, if AFLP were detected in the tested barley and the presence or absence of the amplified fragments of about 331 bp as an index, it would be possible to determine the genotype of the tested barley, whether it is resistant or susceptible, with regard to the allele of the 3H chromosome gene locus involved in barley resistance to yellow mosaic disease.

### [Example 8: Detection of Genetic Marker FMacgEgat88]

The methodology of Example 2 was used except that the amplification reaction (final amplification) was performed with selective primer for EcoPI (base sequence of SEQ ID NO: 44) and selective primer for MseI (base sequence of SEQ ID NO: 43).

### (Results)

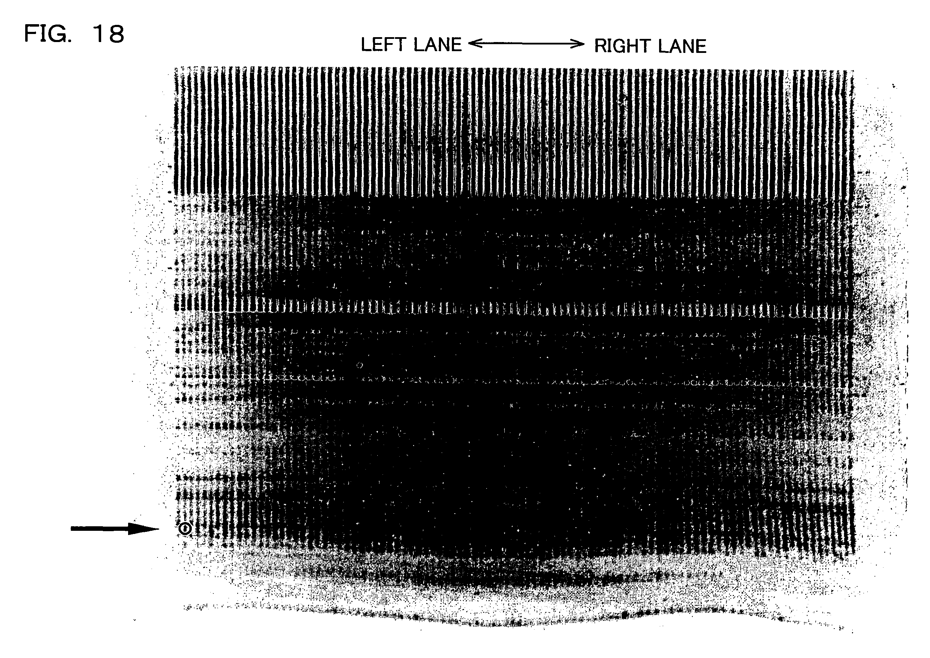

Figure 18 shows the result of electrophoresis performed on the amplified fragments of the amplification reaction. In Figure 18, the leftmost lane and the rightmost two lanes show size markers. The second lane from the left shows the result of AFLP detection on Harbin 2-row, which is a barley variety resistant to yellow mosaic disease. The third lane from the left is the result of AFLP detection on Turkey 6, which is a barley variety susceptible to yellow mosaic disease. The other lanes are the results of AFLP detection on the RI2 population of inbred lines (RI lines) obtained from the cross between Harbin 2-row and Turkey 6.

The results shown in Figure 18 revealed that the amplified fragments obtained by the foregoing procedures were from both the resistant strain (Harbin 2-row) and the susceptible strain (Turkey 6). The fragment length was 0 bp for the resistant strain (Harbin 2-row), and about 88 bp for the susceptible strain (Turkey 6) (indicated by arrow in Figure 18). In other words, the amplified fragment of about 88 bp was obtained only for the susceptible strain (Turkey 6) but not for the resistant strain (Harbin 2-row).

Thus, if AFLP were detected in the tested barley and the presence or absence of the amplified fragments of about 88 bp as an index, it would be possible to determine the genotype of the tested barley, whether it is resistant or susceptible, with regard to the allele of the 5H chromosome gene locus involved in barley resistance to yellow mosaic disease.

### [Example 9: Detection of Genetic Marker MMacgEgga74]

The methodology of Example 2 was used except that the amplification reaction (final amplification) was performed with selective primer for EcoPI (base sequence of SEQ ID NO: 46) and selective primer for MseI (base sequence of SEQ ID NO: 45).

### (Results)

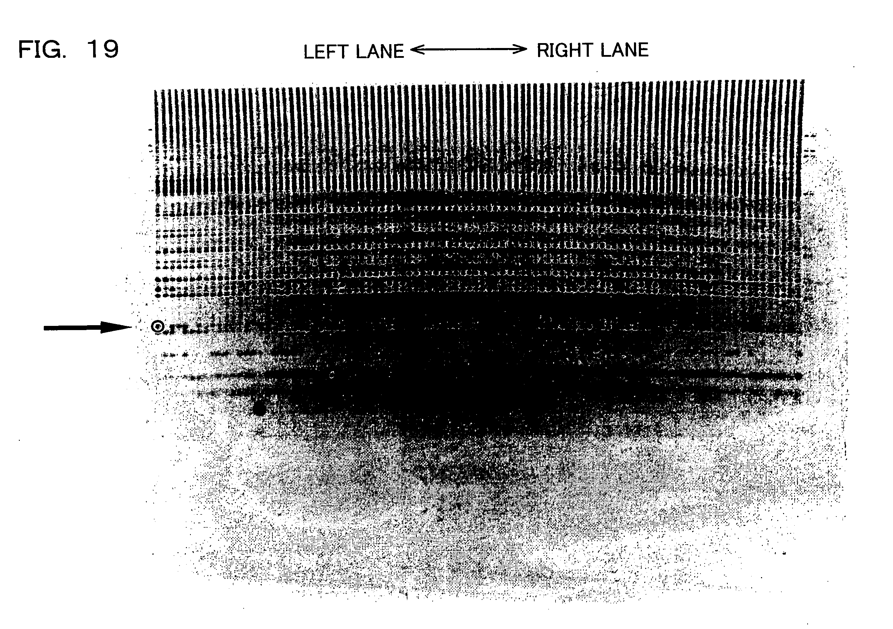

Figure 19 shows the result of electrophoresis performed on the amplified fragments of the amplification reaction. In Figure 19, the leftmost lane and the rightmost two lanes show size markers. The second lane from the left shows the result of AFLP detection on Harbin 2-row, which is a barley variety resistant to yellow mosaic disease. The third lane from the left is the result of AFLP detection on Turkey 6, which is a barley variety susceptible to yellow mosaic disease. The other lanes are the results of AFLP detection on the RI2 population of inbred lines (RI lines) obtained from the cross between Harbin 2-row and Turkey 6.

The results shown in Figure 19 revealed that the amplified fragments obtained by the foregoing procedures were from both the resistant strain (Harbin 2-row) and the susceptible strain (Turkey 6). The fragment length was about 74 bp for the resistant strain (Harbin 2-row) (indicated by arrow in Figure 19), and 0 bp for the susceptible strain (Turkey 6). In other words, the amplified fragment of about 74 bp was obtained only for the resistant strain (Harbin 2-row) but not for the susceptible strain (Turkey 6).

Thus, if AFLP were detected in the tested barley and the presence or absence of the amplified fragments of about 74 bp as an index, it would be possible to determine the genotype of the tested barley, whether it is resistant or susceptible, with regard to the allele of the 5H chromosome gene locus involved in barley resistance to yellow mosaic disease.

### [Example 10: Detection of Genetic Marker FMaccEacg402]

The methodology of Example 2 was used except that the amplification reaction (final amplification) was performed with selective primer for EcoPI (base sequence of SEQ ID NO: 26) and selective primer for MseI (base sequence of SEQ ID NO: 25).

### (Results)

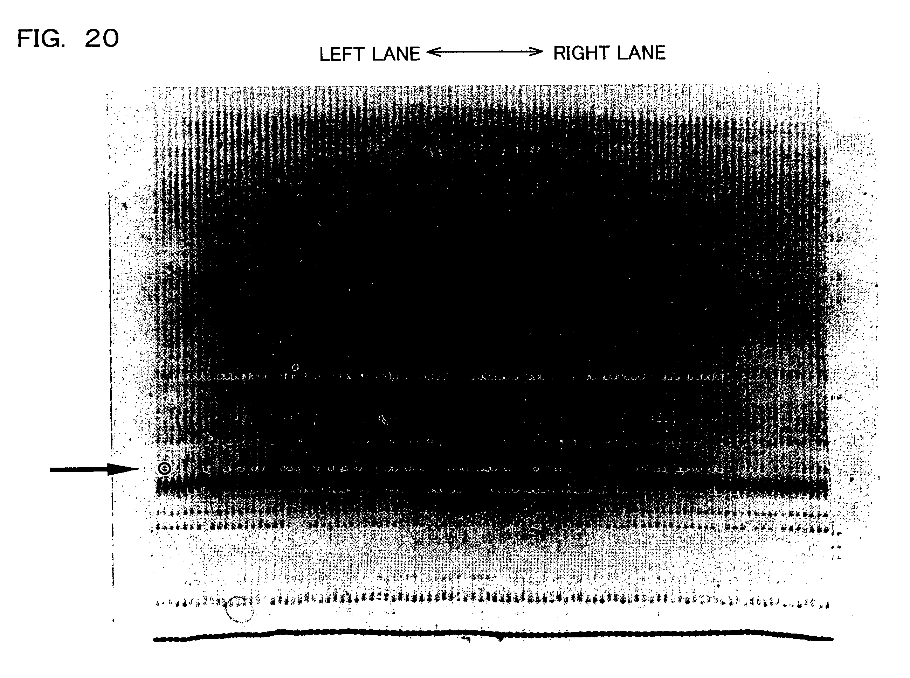

Figure 20 shows the result of electrophoresis performed on the amplified fragments of the amplification reaction. In Figure 20, the leftmost lane and the rightmost two lanes show size markers. The second lane from the left shows the result of AFLP detection on Harbin 2-row, which is a barley variety resistant to yellow mosaic disease. The third lane from the left is the result of AFLP detection on Turkey 6, which is a barley variety susceptible to yellow mosaic disease. The other lanes are the results of AFLP detection on the RI2 population of inbred lines (RI lines) obtained from the cross between Harbin 2-row and Turkey 6.

The results shown in Figure 20 revealed that the amplified fragments obtained by the foregoing procedures were from both the susceptible strain (Harbin 2-row) and the resistant strain (Turkey 6). The fragment length was 0 bp for the susceptible strain (Harbin 2-row), and about 402 bp for the resistant strain (Turkey 6) (indicated by arrow in Figure 20). In other words, the amplified fragment of about 402 bp was obtained only for the resistant strain (Turkey 6) but not for the susceptible strain (Harbin 2-row).

Thus, if AFLP were detected in the tested barley and the presence or absence of the amplified fragments of about 402 bp were used as an index, it would be possible to determine the genotype of the tested barley, whether it is resistant or susceptible, with regard to the allele of the 2H chromosome gene locus involved in barley resistance to yellow mosaic disease.

Note that, the genetic marker is linked to the gene locus involved in the yellow mosaic disease resistance of Turkey 6, which is a variety susceptible to yellow mosaic disease. Thus, in the detection of the genetic marker, Harbin 2-row is the susceptible strain, and Turkey 6 is the resistant strain.

### [Example 11: Detection of Genetic Marker HVM36]

### (Method)

An amplification reaction was performed with primers of the base sequences of SEQ ID NO: 27 and 28, using the genomic DNA of a tested barley as a template. The reaction was performed with the following cycling parameters: one cycle consisting of 94°C for 3 minutes; 10 cycles consisting of 94°C for 1 minute, 64°C for 1 minute (with the temperature lowered by 1°C in each cycle), and 72°C for 1 minute; 30 cycles consisting of 94°C for 1 minute, 55°C for 1 minute, and 72°C for 1 minute; and one cycle consisting of 72°C for 5 minutes.

### (Results)

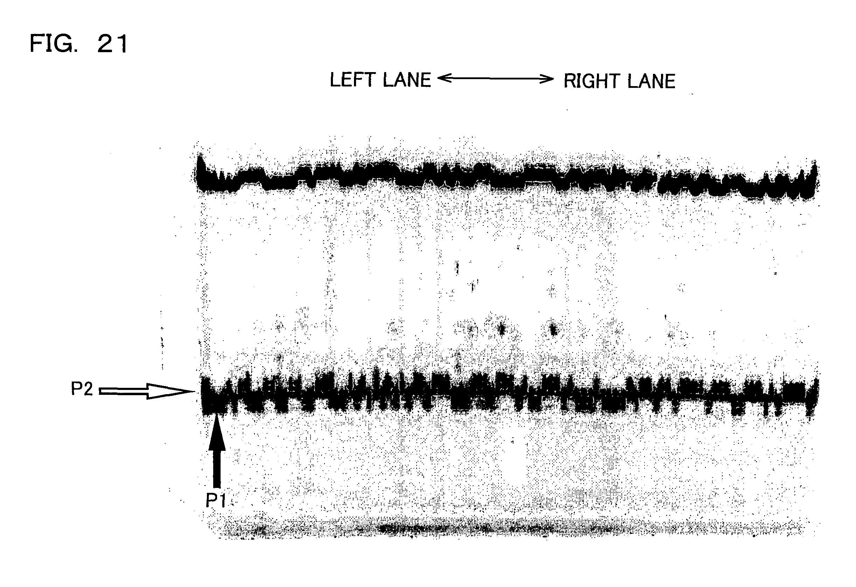

Figure 21 shows the result of electrophoresis performed on the amplified fragments of the amplification reaction. In Figure 21, the leftmost lane shows the result of AFLP detection on Turkey 6, which is a barley variety susceptible to yellow mosaic disease. The rightmost lane is the result of AFLP detection on Harbin 2-row, which is a barley variety resistant to yellow mosaic disease. The other lanes are the results of AFLP detection on the RI2 population of inbred lines (RI lines) obtained from the cross between Harbin 2-row (resistant) and Turkey 6 (susceptible).

The results shown in Figure 21 revealed that the amplified fragments obtained by the foregoing procedures were from both the susceptible strain (Harbin 2-row) and the resistant strain (Turkey 6). The fragment length was about 60 bp to 30 bp for the susceptible strain (Harbin 2-row) the second lane from the left, indicated by arrow P1 in Figure 21), and about 90 bp to 60 bp for the resistant strain (Turkey 6) (the leftmost lane, indicated by arrow P2 in Figure 21).

Thus, if AFLP were detected in the tested barley and the presence or absence of the amplified fragments of about 60 bp to 30 bp, or about 90 bp to 60 bp were used as an index, it would be possible to determine the genotype of the tested barley, whether it is resistant or susceptible, with regard to the allele of the 2H chromosome gene locus involved in barley resistance to yellow mosaic disease.

Note that, the genetic marker is linked to the gene locus involved in the yellow mosaic disease resistance of Turkey 6, which is a variety susceptible to yellow mosaic disease. Thus, in the detection of the genetic markers, Harbin 2-row is the susceptible strain, and Turkey 6 is the resistant strain.

### [Example 12: Detection of Genetic Marker k00256]

### (Method)

An amplification reaction was performed with primers of the base sequences of SEQ ID NO: 1 and 2, using the genomic DNA of a tested barley as a template. The reaction was performed with the following cycling parameters: one cycle consisting of 94°C for 2 minutes; 5 cycles consisting of 94°C for 30 seconds, 65°C for 30 seconds (with the temperature lowered by 1°C in each cycle), and 72°C for 2 minute; 35 cycles consisting of 94°C for 30 seconds, 60°C for 30 seconds, and 72°C for 2 minute; and one cycle consisting of 72°C for 7 minutes.

The amplified products were digested with restriction enzyme at 37°C for 15 hours, using 1.6 U PstI (TAKARA BIO INC.).

### (Results)

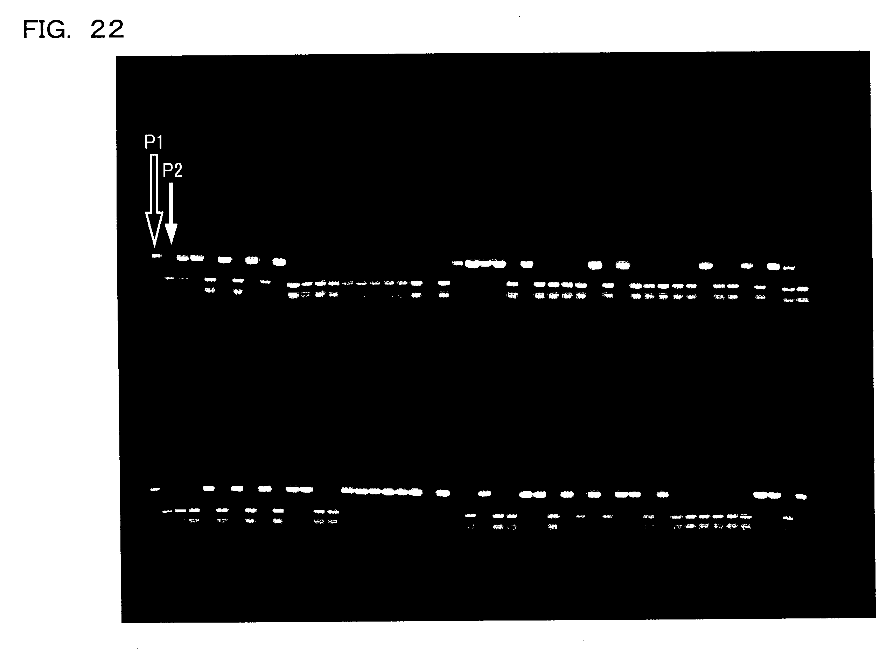

Figure 22 shows the result of electrophoresis performed on amplified fragments after the restriction enzyme treatment. The upper part of Figure 22 shows amplified fragments of Haruna Nijo, H602, F1 cross between Haruna Nijo and H602, and lines 1 through 45 of the DH population, in this order from the left. The lower part of Figure 4 shows amplified fragments of lines of 46 through 93 of the DH population, in this order from the left (except for the molecular weight marker). The leftmost lane in the upper part of Figure 22 shows the result for the amplified fragment of Haruna Nijo, which is a barley variety resistant to yellow mosaic disease. The second lane from the left in the upper part of Figure 22 is the result for the amplified fragment of H602, which is a barley variety susceptible to yellow mosaic disease. Further, in the upper part of Figure 22, the third lane from the left is the result for the amplified fragment of the F1 hybrids. The other lanes are the results for the amplified fragments of the DHHS population of double haploid line (DH line) grown from the cross between Haruna Nijo and H602.

Since the genetic marker is a CAPS marker, restriction enzyme (PstI) shows different digestion patterns. The results shown in Figure 22 revealed that the digested fragments obtained by the foregoing procedures were from both the susceptible strain (Haruna Nijo) and the resistant strain (H602). The fragment length was about 362 bp for Haruna Nijo (indicated by arrow P1 in Figure 22), and about 156 bp and about 206 bp for H602 (indicated by arrow P2 in Figure 22). Thus, if the pattern of digested fragment were used as an index, it would be possible to determine the genotype of the tested barley, whether it is resistant or susceptible, with regard to the allele of the 1H chromosome gene locus involved in barley resistance to yellow mosaic disease.

As noted above, the genetic marker is linked to the gene locus involved in the yellow mosaic disease resistance of H602, which is a variety susceptible to yellow mosaic disease. Thus, in the detection of the genetic marker, Haruna Nijo is the susceptible strain, and H602 is the resistant strain.

### [Example 13: Detection of Genetic Marker k02948]

### (Method)

An amplification reaction was performed with primers of the base sequences of SEQ ID NO: 3 and 4, using the genomic DNA of a tested barley as a template. The reaction was performed with the following cycling parameters: one cycle consisting of 94°C for 2 minutes; 5 cycles consisting of 94°C for 30 seconds, 65°C for 30 seconds (with the temperature lowered by 1°C in each cycle), and 72°C for 2 minute; 35 cycles consisting of 94°C for 30 seconds, 60°C for 30 seconds, and 72°C for 2 minute; and one cycle consisting of 72°C for 7 minutes.

### (Results)

Figure 4 shows the result of electrophoresis performed on the amplified fragments of the amplification reaction. In the upper part and lower part of Figure 4, the both ends are molecular weight markers. The upper part of Figure 4 shows amplified fragments of Haruna Nijo, H602, F1 cross between Haruna Nijo and H602, and lines 1 through 45 of the DH population, in this order from the left (except for the molecular weight marker). The lower part of Figure 4 shows amplified fragments of lines 46 through 93 of the DH population, in this order from the left (except for the molecular weight marker). As can be seen from Figure 4, if the size of the amplified fragments were checked, it would be possible to determine the genotype of the tested barley, whether it is resistant or susceptible, in regard to the allele of the 1H chromosome gene locus involved in barley resistance to yellow mosaic disease.

The results shown in Figure 4 revealed that the amplified fragments obtained by the foregoing procedures were from both the susceptible strain (Haruna Nijo) and the resistant strain (H602). The fragment length was about 389 bp for the susceptible strain (Haruna Nijo) (indicated by arrow P1 in Figure 4), and about 358 bp for the resistant strain (H602) (indicated by arrow P2 in Figure 4).

Thus, if the foregoing detecting procedures were performed on the tested barley and the presence or absence of the amplified fragments of about 389 bp or about 358 bp were used as an index, it would be possible to determine the genotype of the tested barley, whether it is resistant or susceptible, with regard to the allele of the 1H chromosome gene locus involved in barley resistance to yellow mosaic disease.

Note that, the genetic marker is linked to the gene locus involved in the yellow mosaic disease resistance of H602, which is a variety susceptible to yellow mosaic disease. Thus, in the detection of the genetic marker, Haruna Nijo is the susceptible strain, and H602 is the resistant strain.

### [Example 14: Detection of Genetic Marker k03861]

### (Method)

An amplification reaction was performed with primers of the base sequences of SEQ ID NO: 19 and 20, using the genomic DNA of a tested barley as a template. The reaction was performed with the following cycling parameters: one cycle consisting of 94°C for 2 minutes; 5 cycles consisting of 94°C for 30 seconds, 65°C for 30 seconds (with the temperature lowered by 1°C in each cycle), and 72°C for 2 minute; 35 cycles consisting of 94°C for 30 seconds, 60°C for 30 seconds, and 72°C for 2 minute; and one cycle consisting of 72°C for 7 minutes.

### (Results)

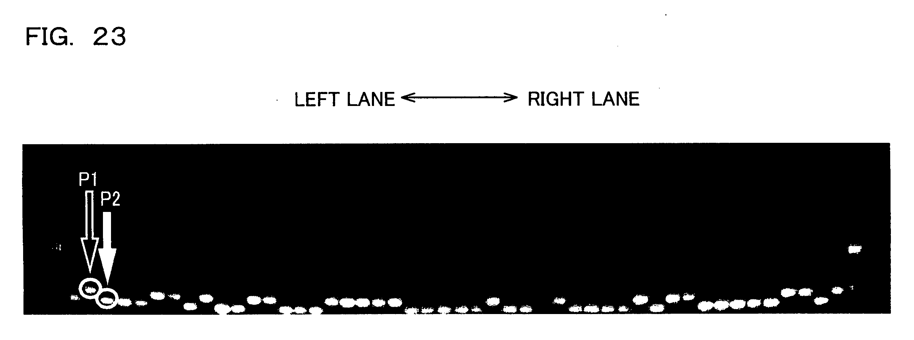

Figure 23 shows the result of electrophoresis performed on the amplified fragments of the amplification reaction. The leftmost and rightmost lane are molecular weight markers. The third lane from the left shows the result for the amplified fragment of Haruna Nijo, which is a barley variety resistant to yellow mosaic disease. The fourth lane from the left is the result for the amplified fragment of H602, which is a barley variety susceptible to yellow mosaic disease. The other lanes are the results for the amplified fragments of the DHHS population of double haploid line (DH line) grown from the cross between Haruna Nijo and H602.

The results shown in Figure 23 revealed that the amplified fragments obtained by the foregoing procedures were from both the susceptible strain (Haruna Nijo) and the resistant strain (H602). The fragment length was about 379 bp for the susceptible strain (Haruna Nijo) (indicated by arrow P1 in Figure 23), and about 353 bp for the resistant strain (H602) (indicated by arrow P2 in Figure 23).

Thus, if the foregoing detecting procedures were performed on the tested barley and the presence or absence of the amplified fragments of about 379 bp or about 353 bp were used as an index, it would be possible to determine the genotype of the tested barley, whether it is resistant or susceptible, with regard to the allele of the 1H chromosome gene locus involved in barley resistance to yellow mosaic disease.

Note that, the genetic marker is linked to the gene locus involved in the yellow mosaic disease resistance of H602, which is a variety susceptible to yellow mosaic disease. Thus, in the detection of the genetic marker, Haruna Nijo is the susceptible strain, and H602 is the resistant strain.

### [Example 15: Detection of Genetic Marker k03616]

### (Method)

An amplification reaction was performed with primers of the base sequences of SEQ ID NO: 21 and 22, using the genomic DNA of a tested barley as a template. The reaction was performed with the following cycling parameters: one cycle consisting of 94°C for 2 minutes; 5 cycles consisting of 94°C for 30 seconds, 65°C for 30 seconds (with the temperature lowered by 1°C in each cycle), and 72°C for 2 minute; 35 cycles consisting of 94°C for 30 seconds, 60°C for 30 seconds, and 72°C for 2 minute; and one cycle consisting of 72°C for 7 minutes.

The amplified products were digested with restriction enzyme at 37°C for 15 hours, using 1.6 U MboI (TAKARA BIO INC.).

### (Results)

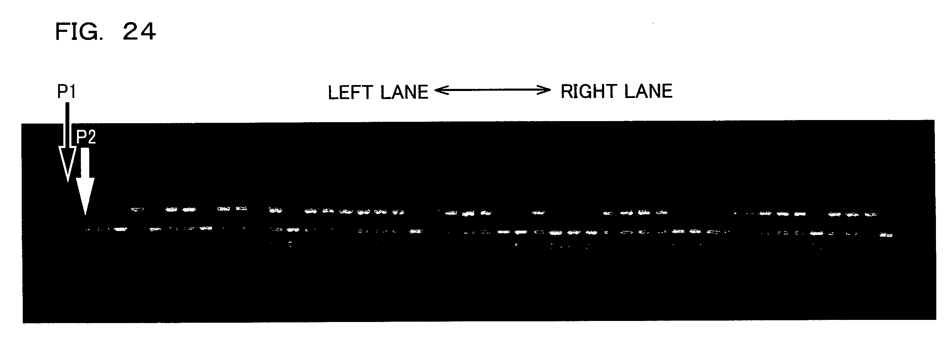

Figure 24 shows the result of electrophoresis performed on amplified fragments after the restriction enzyme treatment. In Figure 24, the leftmost and rightmost lanes are molecular weight markers. The second lane from the left is the result for the amplified fragment of Haruna Nijo, which is a barley variety resistant to yellow mosaic disease. The third lane from the left is the result from the amplified fragment of H602, which is a barley variety susceptible to yellow mosaic disease. The other lanes are the results for the amplified fragments of the DHHS population of double haploid line (DH line) grown from the cross between Haruna Nijo and H602.

Since the genetic marker is a CAPS marker, restriction enzyme (MboI) shows different digestion patterns. The results shown in Figure 24 revealed that the digested fragments obtained by the foregoing procedures were from both the resistant strain (Haruna Nijo) and the susceptible strain (H602). The fragment length was about 323 bp, about 155 bp, about 135 bp, about 85 bp, about 79 bp, and about 50 bp for Haruna Nijo (indicated by arrow P1 in Figure 24), and about 172 bp, about 155 bp, about 151 bp, about 135 bp, about 85 bp, about 79 bp, and about 50 bp for H602 (indicated by arrow P2 in Figure 24). It should be noted here that the amplified fragments of about 172 bp, about 155 bp, and about 151 bp for H602 may overlap one another if the resolution of electrophoresis is low.

Thus, if the pattern of digested fragment were used as an index, it would be possible to determine the genotype of the tested barley, whether it is resistant or susceptible, with regard to the allele of the 1H chromosome gene locus involved in barley resistance to yellow mosaic disease.

### [Example 16: Detection of Genetic Marker k02325]

### (Method)

An amplification reaction was performed with primers of the base sequences of SEQ ID NO: 23 and 24, using the genomic DNA of a tested barley as a template. The reaction was performed with the following cycling parameters: one cycle consisting of 94°C for 2 minutes; 5 cycles consisting of 94°C for 30 seconds, 65°C for 30 seconds (with the temperature lowered by 1°C in each cycle), and 72°C for 2 minute; 35 cycles consisting of 94°C for 30 seconds, 60°C for 30 seconds, and 72°C for 2 minute; and one cycle consisting of 72°C for 7 minutes.

The amplified products were digested with restriction enzyme at 37°C for 15 hours, using 1.6 U HapII (TAKARA BIO INC.).

### (Results)

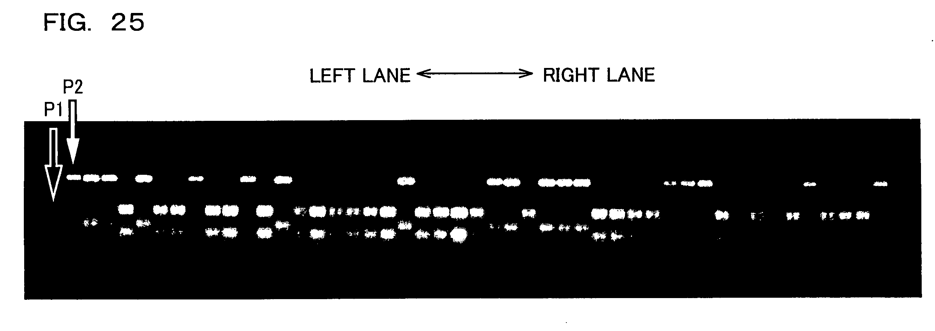

Figure 25 shows the result of electrophoresis performed on amplified fragments after the restriction enzyme treatment. In Figure 25, the leftmost lane is the result for the amplified fragment of Haruna Nijo, which is a barley variety resistant to yellow mosaic disease. The second lane from the left is the result from the amplified fragment of H602, which is a barley variety susceptible to yellow mosaic disease. The third lane from the left is the result for the amplified fragment of F1 hybrids of Haruna Nijo and H602. The other lanes are the results for the amplified fragments of the DHHS population of double haploid line (DH line) grown from the cross between Haruna Nijo and H602.

Since the genetic marker is a CAPS marker, restriction enzyme (HapII) shows different digestion patterns. The results shown in Figure 25 revealed that the digested fragments obtained by the foregoing procedures were from both the resistant strain (Haruna Nijo) and the susceptible strain (H602). The fragment length was about 153 bp, about 145 bp, about 57 bp, about 56 bp, and about 36 bp for Haruna Nijo (indicated by arrow P1 in Figure 25), and about 353 bp and about 94 bp for H602 (indicated by arrow P2 in Figure 25). It should be noted here that the amplified fragments of about 153 bp and about 145 bp for Haruna Nijo, and about 57 bp, about 56, and about 36 bp for Haruna Nijo may overlap one another if the resolution of electrophoresis is low.

Thus, if the pattern of digested fragment were used as an index, it would be possible to determine the genotype of the tested barley, whether it is resistant or susceptible, with regard to the allele of the 1H chromosome gene locus involved in barley resistance to yellow mosaic disease.

### [Example 17: Detection of Genetic Marker k00169]

### (Method)

An amplification reaction was performed with primers of the base sequences of SEQ ID NO: 7 and 8, using the genomic DNA of a tested barley as a template. The reaction was performed with the following cycling parameters: one cycle consisting of 94°C for 2 minutes; 5 cycles consisting of 94°C for 30 seconds, 65°C for 30 seconds (with the temperature lowered by 1°C in each cycle), and 72°C for 2 minute; 35 cycles consisting of 94°C for 30 seconds, 60°C for 30 seconds, and 72°C for 2 minute; and one cycle consisting of 72°C for 7 minutes.

The amplified products were digested with restriction enzyme at 37°C for 15 hours, using 1.6 U AluI (TAKARA BIO INC.).

### (Results)

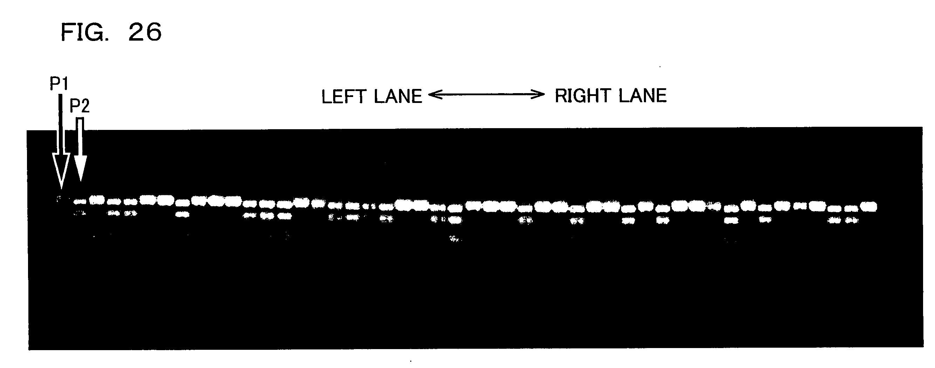

Figure 26 shows the result of electrophoresis performed on amplified fragments after the restriction enzyme treatment. In Figure 26, the leftmost lane is the result for the amplified fragment of Haruna Nijo, which is a barley variety resistant to yellow mosaic disease. The second lane from the left is the result from the amplified fragment of H602, which is a barley variety susceptible to yellow mosaic disease. The other lanes are the results for the amplified fragments of the DHHS population of double haploid line (DH line) grown from the cross between Haruna Nijo and H602.

Since the genetic marker is a CAPS marker, restriction enzyme (AluI) shows different digestion patterns. The results shown in Figure 26 revealed that the digested fragments obtained by the foregoing procedures were from both the resistant strain (Haruna Nijo) and the susceptible strain (H602). The fragment length was about 245 bp and about 215 bp for Haruna Nijo (indicated by arrow P1 in Figure 26), and about 215 bp, about 165 bp, and about 80 bp for H602 (indicated by arrow P2 in Figure 26). It should be noted here that the amplified fragments of about 245 bp and about 215 bp for Haruna Nijo may overlap one another if the resolution of electrophoresis is low. Thus, if the pattern of digested fragment were used as an index, it would be possible to determine the genotype of the tested barley, whether it is resistant or susceptible, with regard to the allele of the 3H chromosome gene locus involved in barley resistance to yellow mosaic disease.

### [Example 18: Detection of Genetic Marker k07966]

### (Method)

An amplification reaction was performed with primers of the base sequences of SEQ ID NO: 33 and 34, using the genomic DNA of a tested barley as a template. The reaction was performed with the following cycling parameters: one cycle consisting of 94°C for 2 minutes; 5 cycles consisting of 94°C for 30 seconds, 65°C for 30 seconds (with the temperature lowered by 1°C in each cycle), and 72°C for 2 minute; 35 cycles consisting of 94°C for 30 seconds, 60°C for 30 seconds, and 72°C for 2 minute; and one cycle consisting of 72°C for 7 minutes.

The amplified products were digested with restriction enzyme at 37°C for 15 hours, using 1.6 U HapII (TAKARA BIO INC.).

### (Results)

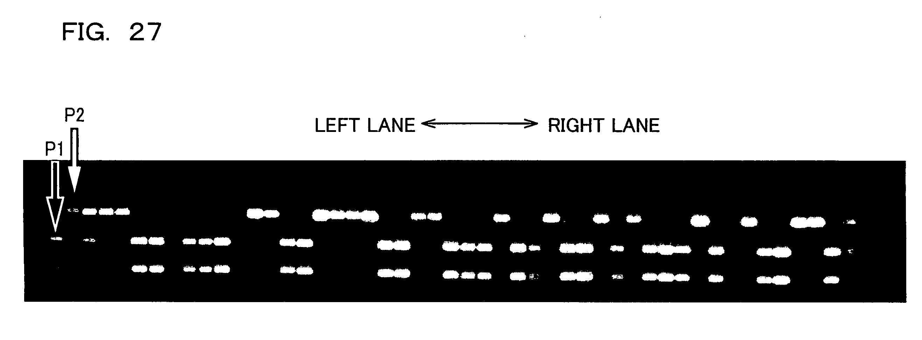

Figure 27 shows the result of electrophoresis performed on amplified fragments after the restriction enzyme treatment. In Figure 27, the leftmost lane is the result for the amplified fragment of Haruna Nijo, which is a barley variety resistant to yellow mosaic disease. The second lane from the left is the result from the amplified fragment of H602, which is a barley variety susceptible to yellow mosaic disease. The third lane from the left is the result for the F1 hybrids of Haruna Nijo and H602. The other lanes are the results for the amplified fragments of the DHHS population of double haploid line (DH line) grown from the cross between Haruna Nijo and H602.

Since the genetic marker is a CAPS marker, restriction enzyme (AluI) shows different digestion patterns. The results shown in Figure 27 revealed that the digested fragments obtained by the foregoing procedures were from both the resistant strain (Haruna Nijo) and the susceptible strain (H602). The fragment length was about 350 bp and about 208 bp for Haruna Nijo (indicated by arrow P1 in Figure 27), and about 558 bp for H602 (indicated by arrow P2 in Figure 27). Thus, if the pattern of digested fragment were used as an index, it would be possible to determine the genotype of the tested barley, whether it is resistant or susceptible, with regard to the allele of the 3H chromosome gene locus involved in barley resistance to yellow mosaic disease.

### [Example 19: Detection of Genetic Marker k04143]

### (Method)

An amplification reaction was performed with primers of the base sequences of SEQ ID NO: 5 and 6, using the genomic DNA of a tested barley as a template. The reaction was performed with the following cycling parameters: one cycle consisting of 94°C for 2 minutes; 5 cycles consisting of 94°C for 30 seconds, 65°C for 30 seconds (with the temperature lowered by 1°C in each cycle), and 72°C for 2 minute; 35 cycles consisting of 94°C for 30 seconds, 60°C for 30 seconds, and 72°C for 2 minute; and one cycle consisting of 72°C for 7 minutes.

The amplified products were digested with restriction enzyme at 37°C for 15 hours, using 1.6 U ApaL1 (TAKARA BIO INC.).

### (Results)

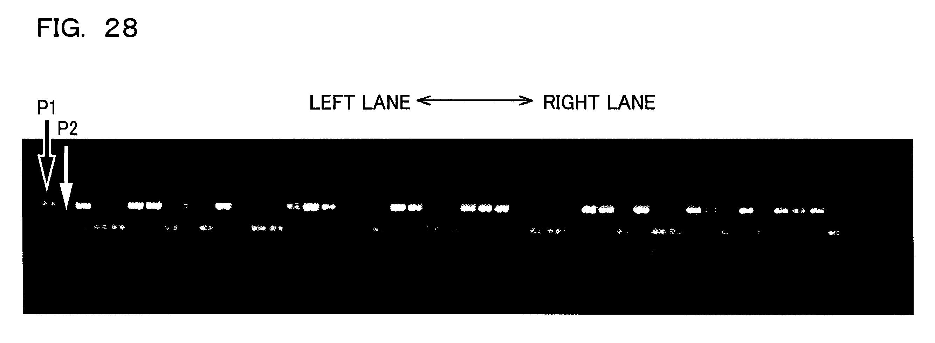

Figure 28 shows the result of electrophoresis performed on amplified fragments after the restriction enzyme treatment. In Figure 28, the leftmost lane is the result for the amplified fragment of Haruna Nijo, which is a barley variety resistant to yellow mosaic disease. The second lane from the left is the result from the amplified fragment of H602, which is a barley variety susceptible to yellow mosaic disease. The third lane from the left is the result for the F1 hybrids of Haruna Nijo and H602. The other lanes are the results for the amplified fragments of the DHHS population of double haploid line (DH line) grown from the cross between Haruna Nijo and H602.

Since the genetic marker is a CAPS marker, restriction enzyme (ApaLI) shows different digestion patterns. The results shown in Figure 28 revealed that the digested fragments obtained by the foregoing procedures were from both the resistant strain (Haruna Nijo) and the susceptible strain (H602). The fragment length was about 371 bp for Haruna Nijo (indicated by arrow P1 in Figure 28), and about 228 bp and about 143 bp for H602 (indicated by arrow P2 in Figure 28). Thus, if the pattern of digested fragment were used as an index, it would be possible to determine the genotype of the tested barley, whether it is resistant or susceptible, with regard to the allele of the 3H chromosome gene locus involved in barley resistance to yellow mosaic disease.

### INDUSTRIAL APPLICABILITY

Genetic markers according to the present invention are applicable to breeding of yellow mosaic disease-resistant barley, or isolation of genes involved in yellow mosaic disease, for example. The genetic markers are therefore expected to greatly improve the efficiency of breeding. Further, the yellow mosaic disease-resistant barley produced with the genetic markers of the present invention can suppress problems caused by yellow mosaic disease and therefore ensure good yield and good quality. The present invention is therefore generally applicable to a wide range of agricultural fields. The invention is also effective in food industry where barley is used as a raw material.

### SEQUENCE LISTING

JST A181-10US(PCT) DO Sequence Listing. txt

## Claims

1. A genetic marker that resides in 1H chromosome of barley and is linked to a gene locus involved in barley resistance to yellow mosaic disease,
wherein the genetic marker is amplified with a first primer set that comprises a primer having the base sequence of SEQ ID NO: 1 and a primer having the base sequence of SEQ ID NO: 2.

2. A genetic marker that resides in 1H chromosome of barley and is linked to a gene locus involved in barley resistance to yellow mosaic disease,
wherein the genetic marker is amplified with a second primer set that comprises a primer having the base sequence of SEQ ID NO: 3 and a primer having the base sequence of SEQ ID NO: 4.

3. A genetic marker that resides in 1H chromosome of barley and is linked to a gene locus involved in barley resistance to yellow mosaic disease,
wherein the genetic marker is amplified with a fifth primer set that comprises a primer having the base sequence of SEQ ID NO: 19 and a primer having the base sequence of SEQ ID NO: 20.

4. A genetic marker that resides in 1H chromosome of barley and is linked to a gene locus involved in barley resistance to yellow mosaic disease,
wherein the genetic marker is amplified with a sixth primer set that comprises a primer having the base sequence of SEQ ID NO: 21 and a primer having the base sequence of SEQ ID NO: 22.

5. A genetic marker that resides in 1H chromosome of barley and is linked to a gene locus involved in barley resistance to yellow mosaic disease,
wherein the genetic marker is amplified with a seventh primer set that comprises a primer having the base sequence of SEQ ID NO: 23 and a primer having the base sequence of SEQ ID NO: 24.

6. A genetic marker that resides in 2H chromosome of barley and is linked to barley resistance to yellow mosaic disease,
wherein the genetic marker is amplified by:
ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50;
pre-amplifying the ligated DNA fragment with an MseI universal primer having the base sequence of SEQ ID NO: 51, and an EcoRI universal primer having the base sequence of SEQ ID NO: 52; and
amplifying the pre-amplified fragment with an eighth primer set that comprises a primer having the base sequence of SEQ ID NO: 25 and a primer having the base sequence of SEQ ID NO: 26.

7. A genetic marker that resides in 2H chromosome of barley and is linked to barley resistance to yellow mosaic disease,
wherein the genetic marker is amplified a ninth primer set that comprises a primer having the base sequence of SEQ ID NO: 27 and a primer having the base sequence of SEQ ID NO: 28.

8. A genetic marker that resides in 3H chromosome of barley and is linked to barley resistance to yellow mosaic disease,
wherein the genetic marker is amplified a third primer set that comprises a primer having the base sequence of SEQ ID NO: 5 and a primer having the base sequence of SEQ ID NO: 6.

9. A genetic marker that resides in 3H chromosome of barley and is linked to barley resistance to yellow mosaic disease,
wherein the genetic marker is amplified a fourth primer set that comprises a primer having the base sequence of SEQ ID NO: 7 and a primer having the base sequence of SEQ ID NO: 8.

10. A genetic marker that resides in 3H chromosome of barley and is linked to barley resistance to yellow mosaic disease,
wherein the genetic marker is amplified by:
ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50;
pre-amplifying the ligated DNA fragment with an MseI universal primer having the base sequence of SEQ ID NO: 51, and an EcoRI universal primer having the base sequence of SEQ ID NO: 52; and
amplifying the pre-amplified fragment with a tenth primer set that comprises a primer having the base sequence of SEQ ID NO: 29 and a primer having the base sequence of SEQ ID NO: 30.

11. A genetic marker that resides in 3H chromosome of barley and is linked to barley resistance to yellow mosaic disease,
wherein the genetic marker-is amplified by:
ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50;
pre-amplifying the ligated DNA fragment with an MseI universal primer having the base sequence of SEQ ID NO: 51, and an EcoRI universal primer having the base sequence of SEQ ID NO: 52; and
amplifying the pre-amplified fragment with an eleventh primer set that comprises a primer having the base sequence of SEQ ID NO: 31 and a primer having the base sequence of SEQ ID NO: 32.

12. A genetic marker that resides in 3H chromosome of barley and is linked to barley resistance to yellow mosaic disease,
wherein the genetic marker is amplified a twelfth primer set that comprises a primer having the base sequence of SEQ ID NO: 33 and a primer having the base sequence of SEQ ID NO: 34.

13. A genetic marker that resides in 4H chromosome of barley and is linked to barley resistance to yellow mosaic disease,
wherein the genetic marker is amplified by:
ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50;
pre-amplifying the ligated DNA fragment with an MseI universal primer having the base sequence of SEQ ID NO: 51, and an EcoRI universal primer having the base sequence of SEQ ID NO: 52; and
amplifying the pre-amplified fragment with a thirteenth primer set that comprises a primer having the base sequence of SEQ ID NO: 35 and a primer having the base sequence of SEQ ID NO: 36.

14. A genetic marker that resides in 4H chromosome of barley and is linked to barley resistance to yellow mosaic disease,
wherein the genetic marker is amplified by:
ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50;
pre-amplifying the ligated DNA fragment with an MseI universal primer having the base sequence of SEQ ID NO: 51, and an EcoRI universal primer having the base sequence of SEQ ID NO: 52; and
amplifying the pre-amplified fragment with a fourteenth primer set that comprises a primer having the base sequence of SEQ ID NO: 37 and a primer having the base sequence of SEQ ID NO: 38.

15. A genetic marker that resides in 4H chromosome of barley and is linked to barley resistance to yellow mosaic disease,
wherein the genetic marker is amplified by:
ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50;
pre-amplifying the ligated DNA fragment with an MseI universal primer having the base sequence of SEQ ID NO: 51, and an EcoRI universal primer having the base sequence of SEQ ID NO: 52; and
amplifying the pre-amplified fragment with a fifteenth primer set that comprises a primer having the base sequence of SEQ ID NO: 39 and a primer having the base sequence of SEQ ID NO: 40.

16. A genetic marker that resides in 4H chromosome of barley and is linked to barley resistance to yellow mosaic disease,
wherein the genetic marker is amplified by:
ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50;
pre-amplifying the ligated DNA fragment with an MseI universal primer having the base sequence of SEQ ID NO: 51, and an EcoRI universal primer having the base sequence of SEQ ID NO: 52; and
amplifying the pre-amplified fragment with a sixteenth primer set that comprises a primer having the base sequence of SEQ ID NO: 41 and a primer having the base sequence of SEQ ID NO: 42.

17. A genetic marker that resides in 5H chromosome of barley and is linked to barley resistance to yellow mosaic disease,
wherein the genetic marker is amplified by:
ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50;
pre-amplifying the ligated DNA fragment with an MseI universal primer having the base sequence of SEQ ID NO: 51, and an EcoRI universal primer having the base sequence of SEQ ID NO: 52; and
amplifying the pre-amplified fragment with a seventeenth primer set that comprises a primer having the base sequence of SEQ ID NO: 43 and a primer having the base sequence of SEQ ID NO: 44.

18. A genetic marker that resides in 5H chromosome of barley and is linked to barley resistance to yellow mosaic disease,
wherein the genetic marker is amplified by:
ligating a DNA fragment, obtained by digesting genomic DNA of barley with restriction enzymes MseI and EcoRI, to an MseI adapter having the base sequences of SEQ ID NO: 47 and 48, and an EcoRI adapter having the base sequences of SEQ ID NO: 49 and 50;
pre-amplifying the ligated DNA fragment with an MseI universal primer having the base sequence of SEQ ID NO: 51, and an EcoRI universal primer having the base sequence of SEQ ID NO: 52; and
amplifying the pre-amplified fragment with an eighteenth primer set that comprises a primer having the base sequence of SEQ ID NO: 45 and a primer having the base sequence of SEQ ID NO: 46.

19. A method for isolating a DNA fragment that includes a gene locus involved in barley resistance to yellow mosaic disease, using a genetic marker of any one of claims 1 through 18.

20. A method for producing a yellow mosaic disease-resistant barley, which comprises introducing a DNA fragment, isolated by the method of claim 19 and including a gene locus involved in barley resistance to yellow mosaic disease, into genomic DNA of barley.

21. A yellow mosaic disease-resistant barley produced by the method of claim 20.

22. A method for screening for a yellow mosaic disease-resistant barley, using a genetic marker of any one of claims 1 through 18 as an index.
